# EUROPÄISCHE PATENTSCHRIFT

(11) **EP 1 420 644 B1**
(45) Veröffentlichungstag und Bekanntmachung des Hinweises auf die Patenterteilung: **31.05.2006**
(21) Anmeldenummer: 02796205.9
(22) Anmeldetag: 02.08.2002
(51) Int. Cl.: A01N 43/40, A01N 43/54, A01N 43/46, A01N 43/42, A01N 43/60, A01N 47/16, A01N 43/78, A01N 43/90, A01N 43/84, C07D 211/00, A61K 31/445

(54) **SUBSTITUIERTE PROPARGYLAMINE**
SUBSTITUTED PROPARGYLAMINES
AMINES PROPARGYLIQUES SUBSTITUEES

(30) Priorität: 23.08.2001 DE 10141339; 20.04.2002 DE 10217697
(43) Veröffentlichungstag der Anmeldung: 26.05.2004
(73) Patentinhaber: Bayer CropScience S.A., 69009 Lyon (FR)
(72) Erfinder: JAKOBI, Harald, 60598 Frankfurt (DE); CRAMP, Susan Mary, Essex CM7 8RH (GB); DICKHAUT, Joachim, 69121 Heidelberg (DE); LINDELL, Stephen, 65779 Kelkheim-Fischbach (DE); TIEBES, Jörg, 60431 Frankfurt (DE); CANALES, Maria, Asuncion, 41010 Sevilla (ES); JANS, Daniela, 61348 Bad Homburg (DE); HEMPEL, Waltraud, 65835 Liederbach (DE); ROYALTY, Reed, Nathan, 61462 Königstein (DE); McCOMB, Susan, Marie, Cary, North Carolina 27513 (US); THÖNESSEN, Maria-Theresia, 55262 Heidesheim (DE); WAIBEL, Jutta, Maria, 60529 Frankfurt (DE); SALDAGO, Vincent, Lee, 61440 Oberursel (DE)
(74) Vertreter: Lutze, Oliver
(86) Internationale Anmeldenummer: PCT/EP2002/008615
(87) Internationale Veröffentlichungsnummer: WO 2003/017764

(56) Entgegenhaltungen:
- EP-A- 0 758 652
- DE-A- 3 504 412
- FR-A- 2 576 021
- US-A- 5 380 883
- US-A- 5 622 954
- DATABASE CA [Online] CHEMICAL ABSTRACTS SERVICE, COLUMBUS, OHIO, US; P.GAYRAL ET AL.: "In vitro and in vivo antifilarial activity of ethynesulfonamides, epoxyethanesulfonamides and carboxamide analogs" retrieved from STN-INTERNATIONAL Database accession no. 124:105597 CA XP002220181 & ARZNEIMITTEL-FORSCHUNG, Bd. 45, Nr. 10, 1995, Seiten 1122-1127,
- DATABASE CA [Online] CHEMICAL ABSTRACTS SERVICE, COLUMBUS, OHIO, US; J.REISCH ET AL.: "Microbiological activity of simple acetylene compounds" retrieved from STN-INTERNATIONAL Database accession no. 67:88434 CA XP002220182 & ARZNEIM.-FORSCHUNG, Bd. 17, Nr. 7, 1967, Seiten 816-825,
- DATABASE CA [Online] CHEMICAL ABSTRACTS SERVICE, COLUMBUS, OHIO, US; G.N.PERSHIN ET AL.: "Bacteriological properties of some aromatic mono- and diacetylenic amines" retrieved from STN-INTERNATIONAL Database accession no. 74:99954 CA XP002220183 & IZV. AKAD. NAUK SSSR, SER. KHIM., Nr. 8, 1970 - 1904, Seite 1906
- DATABASE CA [Online] CHEMICAL ABSTRACTS SERVICE, COLUMBUS, OHIO, US; F.S.KINOYAN ET AL.: "Intensity of the acetylnic band in some tertiary amines and their quaternary ammonium salts" retrieved from STN-INTERNATIONAL Database accession no. 82:111174 CA XP002220184 & ARM. KHIM. ZH., Bd. 27, Nr. 11, 1974, Seiten 923-925,
- DATABASE CA [Online] CHEMICAL ABSTRACTS SERVICE, COLUMBUS, OHIO, US; E.A.CHUKHADZHYAN ET AL.: "Amines and ammonium compounds. XCIV. Base-catalyzed cyclization of dialkylpropargyl(allyl)(3-arylpropargyl)am monium salts" retrieved from STN-INTERNATIONAL Database accession no. 80:108293 CA XP002220185 & ZH. ORG. KHIM., Bd. 10, Nr. 1, 1974, Seiten 46-50,
- DATABASE CA [Online] CHEMICAL ABSTRACTS SERVICE, COLUMBUS, OHIO, US; I.L.KOTLYAREVSKII ET AL.: "Piperidol derivatives of some aromatic acetylenes" retrieved from STN-INTERNATIONAL Database accession no. 78:58212 CA XP002220186 & IZV. AKAD. NAUK SSSR, SER. KHIM. , Nr. 10, 1972, Seiten 2254-2257,

## Beschreibung

Die Erfindung betrifft die Verwendung substituierter Aryl- und Heteroarylpropargylamine als Schädlingsbekämpfungsmittel insbesondere gegen schädliche Arthropoden und Helminthen.

Ats Schädlingsbekämpfungsmittel werden in US-A-5 380 883 (E)-Methyt-α-(methoxymethylen)-7-[3-methyl-3-(1-piperidinyl)-1-butynyl]-napthalenacetat, in EP-A-0 758 652 acetylenische Derivate von substituierten Tetrahydro-5-nitro-pyrimidine und in US-A-5 622 954 5-[3-(4-Chlor-phenyl)-3-piperidin-1-yl-prop-1-ynyl]-6-methyl-pyrimidine-2,4-diamine beschrieben. In FR-A-2 576 021 und von Gayral et al. (Arzneimittel-Forschung, Bd. 45, Nr. 10, 1995, Seiten 1122-1127) werden helmithizide bzw. filarizide Wirkung von 3-Phenyl-4-yl-propynonen-Verbindungen mit 1-substitutierten, speziellen N-Heterocycylresten beschrieben. Verbindungen mit mikrobiologischer bzw. bakteriostatischer und fungistatischer Wirksamkeit werden von Reisch et al. (Arzneimittel-Forschung, Bd. 17, Nr. 7, 1967, Seiten 816-825), wie 1-(3-Phenyl-prop-2-ynyl)-piperidin, und Pershin et al. (Izv. Akad. Nauk SSSR, Ser. Khim., Nr. 8, 1970, Seiten 1904-1906), wie 1-[3-(3-Chlor-2,4,6-trimethoxy-phenyl)-prop-2-ynyl]-piperidin, 3,5-Bis-(piperidin-1-yl-methylen-ethinyliden)-1-Chlor 2,4,6-trimethylbenzol, 2,4-Bis-(piperidin-1-yl-methylen-ethinyliden)-1,5-dimethoxybenzol, 2,4-Bis-(piperidin-1-yl-methylen-ethinyliden)-1,5-benzyloxybenzol, beschrieben. Von Kinoyan et al. (Arm. Khim. Zh., Bd. 27, Nr. 11, 1974, Seiten 923-925), Chukhadzhyan et al. (Zh. Org. Khim., Bd. 10, Nr. 1, 1974, Seiten 46-50) und Kotlyarevskii et al. (Izv. Akad. Nauk SSSR, Ser. Khim., Nr. 10, 1972, Seiten 2254-2257) werden 1-(3-Tolyl-prop-2-ynyl)-piperidin-Verbindungen bzw. unterschiedlich 4-substituierte 1-[3-prop-2-ynyl]-2, 5-dimethyl-piperidin-4-ol-Verbindungen beschrieben, allerdings ohne jeglichen Hinweis auf deren Verwendung als Schädlingsbekämpfungsmittel.

In EP-A-0 041 324 wird 1-[3-(3,5-Bistrifluormethylphenyl)-2-propinyl]-4-tert-butylpiperidin als Rodentizid beschrieben. Ähnliche Verbindungen, in welchen der tertiär Butylrest substituiert ist, werden in DE-A-3 504 412 beschrieben. Weitere Verbindungen dieses Typs, in welchen der Butylrest durch andere Alkylreste ersetzt ist, werden in 1987 BCPC Mono. No 37 Stored Products Pest Control, S. 125, beschrieben. Daneben gibt es weitere Publikationen, worin Verbindungen genannt werden, bei denen Arylreste über Propargyl mit stickstoffhaltigen Heterocyclen verknüpft sind.

Überraschenderweise wurden nun gefunden, daß Verbindungen dieses Typs insektizide, akarizide und helminthizide Wirkung haben. Die Verbindungen sind teilweise neu.

Gegenstand der Erfindung ist ein Verfahren zur Bekämpfung von schädlichen Arthropoden, wie Insekten und Acarina, und Helminthen, wie Tierparasiten und pflanzenschädlichen Nematoden, wobei auf diese Schädlinge oder die von ihnen befallenen Pflanzen oder Tiere, Flächen oder Substrate eine wirksame Menge einer Verbindung gemäß Formel (I), 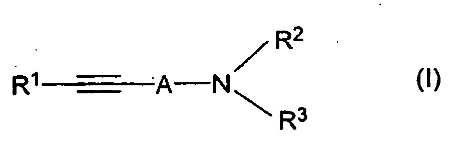 gegebenenfalls auch als N-Oxid und/oder Salz, worin
a) R¹ ein gegebenenfalls ein- oder mehrfach durch gleiche oder verschiedene Reste substituiertes Heteroaryl, Phenyl Biphenyl oder Phenanthryl bedeutet,
   und wenn b1) A eine Gruppe CR⁴R⁵ bedeutet, wobei
      R⁴ Wasserstoff, Alkyl bedeutet;
      R⁵ Wasserstoff, Halogen oder einen gegebenenfalls substituierten Kohlenwasserstoffrest, welcher geradkettige, verzweigte oder cyclische, gesättigte, teilgesättigte oder ungesättigte organische Reste umfasst, bedeutet;
   dann c1) R² und R³ gemeinsam mit dem Stickstoffatom, an das sie gebunden sind, ein gegebenenfalls ein- oder mehrfach durch gleiche oder verschiedene Reste substituiertes N-Heteroaryl, Aziridinyl, Pyrrolidinyl, Isoxazolidinyl, Isothioazolidinyl, Oxazolidinyl, Thiazolidinyl, Imidazolidinyl, 1,2,4-Oxadiazolidinyl, 1,2,4-Thiadiazolidinyl, 1,2,4-Triazolidin-3-yl, 1,3,4-Thiadiazolidin-2-yl, 1,3,4-Triazolidin-1-yl, 1,3,4-Triazolidin-2-yl, 2,3-Dihydropyrrolyl, 2,5-Dihydropyrrolyl, 2,3-Dihydroisoxazolyl, 4,5-Dihydroisoxazolyl, 2,5-Dihydroisothiazolyl, 2,3-Dihydropyrazolyl, 4,5-Dihydropyrazolyl, 2,5-Dihydropyrazolyl, 2,3-Dihydrooxazolyl, 4,5-Dihydrooxazolyl, 2,5-Dihydrooxazolyl, 2,3-Dihydrothiazolyl, 4,5-Dihydrothiazolyl, 2,5-Dihydrothiazolyl, 2,3-Dihydroimidazolyl, 4,5-Dihydroimidazolyl, 2,5-Dihydroimidazolyl, Morpholinyl, Piperidinyl, Piperazinyl, Tetrahydropyridazinyl, Tetrahydropyrazinyl, 1,3,5-Tetrahydrotriazinyl. 1,2,4-Tetrahydrotriazin-1-yl, 1,2,4-Tetrahydrotriazin-3-yl, 1,3-Dihydrooxazinyl, 3,4,5,6-Tetrahydropyridin-2-yl, 1,2,5,6-Tetrahydropyridin-1-yl, 1,2,3,4-Tetrahydropyridin-1-yl, 1,2-Dihydropyridin-1-yl, 1,4-Dihydropyridin-1-yl, 4H-1,3-Thiazinyl, 4H-3,1-Benzothiazin-2-yl, 2H-1,4-Benzothiazinyl, 1,3-Dihydrooxazin-2-yl, Hexahydroazepin-1-yl, Homopiperazin-1-yl, 1,2,3,4-Tetrahydrochinolin-1-yl, Decahydrochinolin-1-yl, 1,2,3,4-Tetrahydroisochinolin-1-yl, Decahydroisochinolin-1-yl, 1,3,3-Trimethyl-6-azabicyclo[3.2.1]octan-6-yl, 2,5-Diazabicyclo[2.2.1]heptan-2-yl, 2-Aza-5-oxabicyclo[2.2.1]heptan-2-yl, 2-Aza-5-thiabicyclo[2.2.1]heptan-2-yl, 2-Methyl-2,5-diazabicyclo[2.2.1]heptan-5-yl, 2-Benzyl-2,5-Diazabicyclo[2.2.1]heptan-5-yl oder 4-Azatricyclo[4.3.1.1(3,8)]undecan-5-on-1-yl bildet,
   oder wenn b2) A eine Gruppe C=O bedeutet,
   dann c2) R² und R³ gemeinsam mit dem Stickstoffatom, an das sie gebunden sind, ein gegebenenfalls ein- oder mehrfach durch gleiche oder verschiedene Reste substituiertes Piperidin bildet,
appliziert wird.

Weiterhin Gegenstand der Erfindung ist die Verwendung von Verbindungen der Formel (I) zur Bekämpfung von Arthropoden, wie Insekten und Acarina, und Helminthen, wie Tierparasiten und pflanzenschädlichen Nematoden.

Viele der Verbindungen gemäß Formel (I) sind neu, und die Erfindung beinhaltet alle neuen Verbindungen gemäß Formel (I), insbesondere diejenigen, in welchen
a) R¹ ein gegebenenfalls ein- oder mehrfach durch gleiche oder verschiedene Reste substituiertes Heteroaryl oder ein- oder mehrfach durch gleiche oder verschiedene Reste substituiertes Phenyl, Biphenyl oder Phenanthryl bedeutet,
b) A eine Gruppe CR⁴R⁵, oder C=O bedeutet, wobei
   R⁴ Wasserstoff, Alkyl bedeutet;
   R⁵ Wasserstoff oder einen gegebenenfalls substituierten Kohlenwasserstoffrest, welcher geradkettige, verzweigte oder cyclische, gesättigte, teilgesättigte oder ungesättigte organische Reste umfasst, bedeutet;
c) R² und R³ gemeinsam mit dem Stickstoffatom an das sie gebunden sind ein gegebenenfalls ein- oder mehrfach durch gleiche oder verschiedene Reste substituiertes Piperidin bildet,
mit der Maßgabe, daß, wenn R² und R³ gemeinsam mit dem Stickstoffatom, an das sie gebunden sind, einen unsubstituierten oder in der 4-Position durch gegebenenfalls substituiertes Alkyl substituierten Piperidinrest bilden, R¹ nicht 3,5-Bistrifluormethylphenyl ist.
und mit der Maßgabe, daß, die Verbindungen gemäß Formel (I) nicht
1-[3-(3-Chlor-2,4,6-trimethoxy-phenyl)-prop-2-inyl]-piperidin,
3,5-Bis-(piperidin-1-yl-prop-2-inyl)-1-chlor-2,4,6-trimethylbenzol,
2,4-Bis-(piperidin-1-yl-prop-2-inyl)-1,5-dimethooybenzvl,
2,4-Bis-(piperidin-1-yl-prop-2-inyl)-1,5-di(*n*-propoxy)benzol,
1-(3-*m*-Tolyl-prop-2-inyl)-piperidin,
1-(3-*p*-Tolyl-prop-2-inyl)-piperidin,
1-(3-*o*-Tolyl-prop-2-inyl)-piperidin,
1-[3-(4-Hydroxy-phenyl)-prop-2-inyl]-2, 5-dimethyl-piperidin-4-ol,
1-Phenyl-3-(1-piperidyl)-prop-1yn,
1-[3-(4-*n*-Propoxyphenyl)-prop-2-inyl]-2,5-dimethyl-piperidin-4-ol.
1-[3-Phenyl-prop-2-inyl]-2,5-dimethyl-piperidin-4-ol
sind.

In der obigen Formel ist ein Kohlenwasserstoffrest ein geradkettiger, verzweigter oder cyclischer, gesättigter, teilgesättigter oder ungesättigter organischer Rest mit vorzugsweise 1 bis 20 Kohlenstoffatomen, z.B. Alkyl, Alkenyl, Alkinyl, Cycloalkyl, Cycloalkenyl, Cycloalkinyl, Aryl oder Benzyl. Ebenso sind zusammengesetzte Begriffe, wie Cycloalkylalkenyl, Cycloalkinylalkyl und Arylalkinyl, von dieser Definition umfaßt. Enthält ein Kohlenwasserstoffrest noch zusätzlich Heteroatome, so können sich diese grundsätzlich, d.h. sofern es der chemische Aufbau erlaubt, an beliebiger Position des Kohlenwasserstoffrests befinden.

In Formel (I) und allen nachfolgenden Formeln können kettenförmige kohlenstoffhaltige Reste, wie Alkyl, Alkoxy, Halogenalkyl, Halogenalkoxy, Alkylamino und Alkylthio, sowie die entsprechenden ungesättigten und/oder substituierten Reste im Kohlenstoffgerüst, wie Alkenyl und Alkinyl, jeweils geradkettig oder verzweigt sein. Wenn nicht speziell angegeben, sind bei diesen Resten die niederen Kohlenstoffgerüste, vorzugsweise mit 1 bis 6 C-Atomen bzw. bei ungesättigten Gruppen mit 2 bis 4 C-Atomen, bevorzugt.

Alkylreste, auch in den zusammengesetzten Gruppen wie Alkoxy, Halogenalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl, 1,4-Dimethylpentyl, und Benzyl; Alkenyl- und Alkinylreste, auch in den zusammengesetzten Gruppen, haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl. Die Mehrfachbindung kann sich in beliebiger Position des ungesättigten Rests befinden.

Cycloalkyl bedeutet ein carbocyclisches, gesättigtes Ringsystem mit vorzugsweise drei bis acht C-Atomen, z.B. Cyclopropyl, Cyclobutyl, Cyclopentyl oder Cyclohexyl. Analog bedeutet Cycloalkenyl eine monocyclische Alkenylgruppe mit drei bis acht Kohlenstoffringgliedern, z.B. Cyclopropenyl, Cyclobutenyl, Cyclopentenyl und Cyclohexenyl, wobei sich die Doppelbindung an beliebiger Position befinden kann.

Im Falle zusammengesetzter Reste, wie Cycloalkylalkenyl, kann sich der erstgenannte Rest an beliebiger Position des zweitgenannten befinden.

Im Falle einer zweifach substituierten Aminogruppe, wie Dialkylamino, können diese beiden Substituenten gleich oder verschieden sein.

Halogen bedeutet Fluor, Chlor, Brom oder lod. Halogenalkyl, -alkenyl und -alkinyl, etc. bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₂CF₃, CHClCH₂F, CCl₃, CCl₂F, CClF₂, CHCl₂, CH₂CH₂Cl; Halogenalkoxy ist z.B. OCF₃, OCHF₂, OCH₂F, OCF₂CF₃, OCH₂CF₃ und OCH₂CH₂Cl; entsprechendes gilt für andere durch Halogen substituierte Reste.

Unter dem Begriff "Heterocyclyl" ist ein gesättigtes oder partiell ungesättiges mono- oder polycyclisches Ringsystem, mit vorzugsweise 3 bis 14 Ringgliedern, zu verstehen, das ein oder mehrere, vorzugsweise ein bis drei, Heteroatome, vorzugsweise aus einer Gruppe Sauerstoff, Stickstoff ("N-Heterocyclyl") und Schwefel, enthält. Die Verknüpfung kann, sofern chemisch möglich, an beliebiger Position des Heterocyclus erfolgen. Beispiele dafür sind Oxiranyl, Aziridinyl, Tetrahydrofuranyl, Tetrahydrothienyl, Pyrrolidinyl, Isoxazolidinyl, Isothioazolidinyl, Pyrazolidinyl, Oxazolidinyl, Thiazolidinyl, Imidazolidinyl, 1,2,4-Oxadiazolidinyl, 1,2,4-Thiadiazolidinyl, 1,2,4-Triazolidin-3-yl, 1,3,4-Thiadiazolidin-2-yl, 1,3,4-Triazolidin-1-yl, 1,3,4-Triazolidin-2-yl, 2,3-Dihydrofuryl, 2,5-Dihydrofuryl, 2,3-Dihydrothienyl, 2,5-Dihydrothienyl, 2,3-Dihydropyrrolyl, 2,5-Dihydropyrrolyl, 2,3-Dihydroisoxazolyl, 4,5-Dihydroisoxazolyl, 2,5-Dihydroisothiazolyl, 2,3-Dihydropyrazolyl, 4,5-Dihydropyrazolyl, 2,5-Dihydropyrazolyl, 2,3-Dihydrooxazolyl, 4,5-Dihydrooxazolyl, 2,5-Dihydrooxazolyl, 2,3-Dihydrothiazolyl, 4,5-Dihydrothiazolyl, 2,5-Dihydrothiazolyl, 2,3-Dihydroimidazolyl, 4,5-Dihydroimidazolyl, 2,5-Dihydroimidazolyl, Morpholinyl, Piperidinyl, Piperazinyl, Tetrahydropyridazinyl, Tetrahydropyrazinyl, 1,3,5-Tetrahydrotriazinyl, 1,2,4-Tetrahydrotriazin-1-yl, 1,2,4-Tetrahydrotriazin-3-yl, 1,3-Dihydrooxazinyl, 1,3-Dithian-2-yl, Tetrahydropyranyl, 1,3-Dioxolan-2-yl, 3,4,5,6-Tetrahydropyridin-2-yl, 1,2,5,6-Tetrahydropyridin-1-yl, 1,2,3,4-Tetrahydropyridin-1-yl, 1,2-Dihydropyridin-1-yl, 1,4-Dihydropyridin-1-yl, 4H-1,3-Thiazinyl, 4H-3,1-Benzothiazin-2-yl,-1,3-Dithian-2-yl, 1,1-Dioxo-2,3,4,5-tetrahydrothien-2-yl, 2H-1,4-Benzothiazinyl, 1,3-Dihydrooxazin-2-yl, Hexahydroazepin-1-yl, Homopiperazin-1-yl, 1,2,3,4-Tetrahydrochinolin-1-yl, Decahydrochinolin-1-yl, 1,2,3,4-Tetrahydroisochinolin-1-yl, Decahydroisochinolin-1-yl, 1,3,3-Trimethyl-6-azabicyclo[3.2.1]octan-6-yl, 2,5-Diazabicyclo[2.2.1]heptan-2-yl, 2-Aza-5-oxabicyclo[2.2.1]heptan-2-yl, 2-Aza-5-thiabicyclo[2.2.1]heptan-2-yl, 2-Methyl-2,5-diazabicyclo[2.2.1]heptan-5-yl, 2-Benzyl-2,5-Diazabicyclo[2.2.1]heptan-5-yl, 4-Azatricyclo[4.3.1.1(3,8)]undecan-5-on-4-yl.

Aryl steht für einen aromatischen mono- oder polycyclischen Kohlenwasserstoffrest mit vorzugsweise 6 bis 14, besonders bevorzugt 6 bis 12, C-Atomen, z.B. Phenyl, Biphenyl und Phenanthryl.

Heteroaryl steht für ein aromatisches mono-, bi- oder tricyclisches Ringsystem, mit vorzugsweise 5 bis 14 Ringgliedern, das neben Kohlenstoffringgliedern ein bis vier Stickstoffatome oder ein bis drei Stickstoffatome ("N-Heteroaryl") und ein Sauerstoff- oder ein Schwefelatom oder ein Sauerstoff- oder ein Schwefelatom enthält. Beispiele für 5-gliedriges Heteroaryl sind 1-Pyrrolyl, 2-Pyrrolyl, 3-Pyrrolyl, 1-Pyrazolyl, 3-Pyrazolyl, 4-Pyrazolyl, 5-Pyrazolyl, 1-Imidazolyl, 2-Imidazolyl, 4-Imidazolyl, 1,2,4-Triazol-3-yl, 1,3,4-Triazol-2-yl, 1,2,3-Triazol-1-yl, 2-Furyl, 3-Furyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl. Beispiele für 6-gliedriges Heteroaryl sind 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 6-Pyrimidinyl, 2-Pyrazinyl, 1,3,5-Triazin-2-yl, 1,2,4-Triazin-3-yl und 1,2,4,5-Tetrazin-3-yl. Beispiele für anneliertes 5-gliedriges Heteroaryl sind Benzothiazol-2-yl und Benzoxazol-2-yl. Beispiele für benzokondensierte 6-gliedriges Heteroaryl sind Chinolinyl, Isochinolinyl, Chinazolinyl und Chinoxalinyl.

Die Verbindungen der allgemeinen Formel (I) können je nach Art und Verknüpfung der Substituenten als Stereoisomere vorliegen. Sind beispielsweise eine oder mehrere Alkenylgruppen vorhanden, so können Diastereomere auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Ist eine Gruppe mehrfach substituiert, so ist darunter zu verstehen, daß bei der Kombination der verschiedenen Substituenten die allgemeinen Grundsätze des Aufbaus chemischer Verbindungen zu beachten sind, d.h. daß nicht Verbindungen gebildet werden, von denen der Fachmann weiß, daß sie chemisch instabil oder nicht möglich sind.

Bevorzugt haben die Symbole und Indizes der allgemeinen Formel (I) folgende Bedeutung:
R¹ bedeutet ein Aryl oder Heteroaryl, gegebenenfalls ein- oder mehrfach, gleich oder verschieden substituiert durch: Alkyl, Alkenyl, Alkinyl, Cycloalkyl, Cycloalkenyl, Cycloalkylalkyl, Cycloalkylalkenyl, Cycloalkylalkinyl, Aryl, Arylalkyl, Arylalkenyl, Arylalkinyl, Heteroaryl, Heteroarylalkyl, Heteroarylalkenyl, Heteroarylalkinyl, Heterocyclyl, Heterocyclylalkyl, Heterocyclylalkenyl, Heterocyclylalkinyl, Hydroxy, Alkoxy, Alkenyloxy, Alkinyloxy, Cycloalkoxy, Cycloalkylalkoxy, Cycloalkylalkenyloxy, Cycloalkylalkinyloxy, Cycloalkenyloxy, Aryloxy, Arylalkoxy, Arylalkenyloxy, Arylalkinyloxy, Heteroaryloxy, Heteroarylalkoxy, Heteroarylalkenyloxy, Heteroarylalkinyloxy, Heterocyclyloxy, Heterocyclylalkoxy, Heterocyclylalkenyloxy, Heterocyclylalkinyloxy, Thio, Alkylthio, Alkenylthio, Alkinylthio, Cycloalkylthio, Cycloalkylalkylthio, Cycloalkylalkenylthio, Cycloalkylalkinylthio, Cycloalkenylthio, Arylthio, Arylalkylthio, Arylalkenylthio, Arylalkinylthio, Heteroarylthio, Heteroarylalkylthio, Heteroarylalkenylthio, Heteroarylalkinylthio, Heterocyclylthio, Heterocyclylalkylthio, Heterocyclylalkenylthio, Heterocyclylalkinylthio, Amino, gegebenenfalls substituiertes Mono- oder Dialkylamino, gegebenenfalls substituiertes Mono- oder Diarylamino, gegebenenfalls substituiertes Mono- oder Di-Heteroarylamino, gegebenenfalls substituiertes N-Alkyl-N-arylamino, gegebenenfalls substituiertes N-Alkyl-N-Heteroarylamino, Alkenylamino, Alkinylamino, Cycloalkylamino, Cycloalkenylamino, Heterocyclylalkylamino, Heterocyclylalkenylamino, Alkylsulfonyl, Alkenylsulfonyl, Alkinylsulfonyl, Cycloalkylsulfonyl, Cycloalkylalkylsulfonyl, Cycloalkylalkenylsulfonyl, Cycloalkylalkinylsulfonyl, Arylsulfonyl, Arylalkylsulfonyl, Arylalkenylsulfonyl, Arylalkinylsulfonyl, Heteroarylsulfonyl, Heteroarylalkylsulfonyl, Heteroarylalkenylsulfonyl, Heteroarylalkinylsulfonyl, Heterocyclylsulfonyl, Heterocyclylalkylsulfonyl, Heterocyclylalkenylsulfonyl, Heterocyclylalkinylsulfonyl, Alkylsulfinyl, Alkenylsulfinyl, Alkinylsulfinyl, Cycloalkylsulfinyl, Cycloalkylalkylsulfinyl, Cycloalkylalkenylsulfinyl, Cycloalkylalkinylsulfinyl, Arylsulfinyl, Arylalkylsulfinyl, Arylalkenylsulfinyl, Arylalkinylsulfinyl, Heteroarylsulfinyl, Heteroarylalkylsulfinyl, Heteroarylalkenylsulfinyl, Heteroarylalkinylsulfinyl, Heterocyclylsulfinyl, Arylalkylsulfinyl, Heterocyclylalkenylsulfinyl, Heterocyclylalkinylsulfinyl, Aminosulfonyl, gegebenenfalls substituiertes Mono- oder Dialkylaminosulfonyl, gegebenenfalls substituiertes Mono- oder Diarylaminosulfonyl, gegebenenfalls substituiertes Mono- oder Di-heteroarylaminosulfonyl, gegebenenfalls substituiertes N-Alkyl-N-arylaminosulfonyl, gegebenenfalls substituiertes N-Alkyl-N-heteroarylaminosulfonyl, Alkylsulfonyloxy, Alkenylsulfonyloxy, Alkinylsulfonyloxy, Cycloalkylsulfonyloxy, Cycloalkylalkylsulfonyloxy, Cycloalkylalkenylsulfonyloxy, Cycloalkylalkinylsulfonyloxy, Arylsulfonyloxy, Arylalkylsulfonyloxy, Arylalkenylsulfonyloxy, Arylalkinylsulfonyloxy, Heteroarylsulfonyloxy, Heteroarylalkylsulfonyloxy, Heteroarylalkenylsulfonyloxy, Heteroarylalkinylsulfonyloxy, Heterocyclylsulfonyloxy, Heterocyclylalkylsulfonyloxy, Heterocyclylalkenylsulfonyloxy, Heterocyclylalkinylsulfonyloxy, Alkylsulfonylamino, Alkenylsulfonylamino, Alkinylsulfonylamino, Cycloalkylsulfonylamino, Cycloalkylalkylsulfoamino, Cycloalkylalkenylsulfonylamino, Cycloalkylalkinylsulfonylamino, Arylsulfonylamino, Arylalkylsulfonylamino, Arylalkenylsulfonoamino, Arylalkinylsulfonylamino, Heteroarylsulfonylamino, Heteroarylalkylsulfonylamino, Heteroarylalkenylsulfonoamino, Heteroarylalkinylsulfonylamino, Alkylsulfonyl-N-alkylamino, Alkenylsulfonyl-N-alkylamino, N-Alkyl-alkinylsulfonyl-N-alkylamino, Cycloalkylsulfonyl-N-alkylamino, Cycloalkylalkylsulfonyl-N-alkylamino, Cycloalkylalkenylsulfonyl-N-alkylamino, Cycloalkylalkinylsulfonyl-N-alkylamino, Arylsulfonyl-N-alkylamino, Heteroarylsulfonyl-N-alkylamino, Arylalkylsulfonyl-N-alkylamino, Heteroarylalkylsulfonyl-N-alkylamino, Arylalkenylsulfonyl-N-alkylamino, Heteroarylalkenylsulfonyl-N-alkylamino, Arylalkinylsulfonyl-N-alkylamino, Heteroarylalkinylsulfonyl-N-alkylamino, Heterocyclylsulfonyl-N-alkylamino, Heterocyclylalkylsulfonyl-N-alkylamino, Heterocyclylalkenylsulfonyl-N-alkylamino, Heterocyclylalkinylsulfonyl-N-alkylamino, Formyl, Alkylcarbonyl, Alkenylcarbonyl, Alkinylcarbonyl, Cycloalkylcarbonyl, Cycloalkylalkylcarbonyl, Cycloalkylalkenylcarbonyl, Cycloalkylalkinylcarbonyl, Arylcarbonyl, Arylalkylcarbonyl, Arylalkenylcarbonyl, Arylalkinylcarbonyl, Heteroarylcarbonyl, Heteroarylalkylcarbonyl, Heteroarylalkenylcarbonyl, Heteroarylalkinylcarbonyl, Heterocyclylcarbonyl, Heterocyclylalkylcarbonyl, Heterocyclylalkenyl, Heterocyclylalkinylcarbonyl, Formyloxy, Alkylcarbonyloxy, Alkenylcarbonyloxy, Alkinylcarbonyloxy, Cycloalkylcarbonyloxy, Cycloalkylalkylcarbonyloxy, Cycloalkylalkenylcarbonyloxy, Cycloalkylalkinylcarbonyloxy, Arylcarbonyloxy, Arylalkylcarbonyloxy, Arylalkenylcarbonyloxy, Arylalkinylcarbonyloxy, Heteroarylcarbonyloxy, Heteroarylalkylcarbonyloxy, Heteroarylalkenylcarbonyloxy, Heteroarylalkinylcarbonyloxy, Heterocyclylcarbonyloxy, Heterocyclylalkylcarbonyloxy, Heterocyclylalkenyloxy, Heterocyclylalkinylcarbonyloxy, Carboxyl, Alkoxycarbonyl, Alkenyloxycarbonyl, Alkinyloxycarbonyl, Cycloalkoxycarbonyl, Cycloalkylalkoxycarbonyl, Cycloalkylalkenyloxycarbonyl, Cycloalkylalkinyloxycarbonyl, Aryloxycarbonyl, Arylalkoxycarbonyl, Arylalkenyloxycarbonyl, Arylalkinyloxycarbonyl, Heteroaryloxycarbonyl, Heteroarylalkoxycarbonyl, Heteroarylalkenyloxycarbonyl, Heteroarylalkinyloxycarbonyl, Heterocyclyloxycarbonyl, Heterocyclylalkoxycarbonyl, Heterocyclylalkenyloxycarbonyl, Heterocyclylalkinyloxycarbonyl, Aminocarbonyl, gegebenenfalls substituiertes Mono- oder Dialkylaminocarbonyl, gegebenenfalls substituiertes Mono- oder Diarylaminocarbonyl, gegebenenfalls substituiertes Mono- oder Di-heteroarylaminocarbonyl, gegebenenfalls substituiertes N-Alkyl-N-arylaminocarbonyl, gegebenenfalls substituiertes N-Alkyl-N-heteroarylaminocarbonyl, gegebenenfalls substituiertes Alkylcarbonylamino, gegebenenfalls substituiertes Alkylcarbonyl-N-alkylamino, gegebenenfalls substituiertes Arylcarbonylamino, gegebenenfalls substituiertes Arylcarbonyl-N-arylamino, gegebenenfalls substituiertes heteroarylcarbonylamino, gegebenenfalls substituiertes Heteroarylcarbonyl-N-heteroarylamino, gegebenenfalls substituiertes Alkylcarbonyl-N-arylamino, gegebenenfalls substituiertes Arylcarbonyl-N-alkylamino, gegebenenfalls substituiertes Alkylcarbonyl-N-heteroarylamino, gegebenenfalls substituiertes Heteroarylcarbonyl-N-alkylamino, Alkoxycarbonylamino, Alkenyloxycarbonylamino, Alkinyloxycarbonylamino, Cycloalkoxycarbonylamino, Cycloalkylalkoxycarbonylamino, Cycloalkylalkenyloxycarbonylamino, Cycloalkylalkinyloxycarbonylamino, Aryloxycarbonylamino, Arylalkoxycarbonylamino, Arylalkenyloxycarbonylamino, Arylalkinyloxycarbonylamino, Heteroaryloxycarbonylamino, Heteroarylalkoxycarbonylamino, Heteroarylalkenyloxycarbonylamino, Heteroarylalkinyloxycarbonylamino, Heterocyclyloxycarbonylamino, Heterocyclylalkoxycarbonylamino, Heterocyclylalkenyloxycarbonylamino, Heterocyclylalkinyloxycarbonylamino, Alkoxycarbonyl-N-alkylamino, Alkenyloxycarbonyl-N-alkylamino, Alkinyloxycarbonyl-N-alkylamino, Cycloalkoxycarbonyl-N-alkylamino, Cycloalkylalkoxycarbonyl-N-alkylamino, Cycloalkylalkenyloxycarbonyl-N-alkylamino, Cycloalkylalkinyloxycarbonyl-N-alkylamino, Aryloxycarbonyl-N-alkylamino, Arylalkoxycarbonyl-N-alkylamino, Arylalkenyloxycarbonyl-N-alkylamino, Arylalkinyloxycarbonyl-N-alkylamino, Heteroarylalkoxycarbonyl-N-alkylamino, Heteroarylalkenyloxycarbonyl-N-alkylamino, Heteroarylalkinyloxycarbonyl-N-alkylamino, Heterocyclylalkoxycarbonyl-N-alkylamino, Heterocyclylalkenyloxycarbonyl-N-alkylamino, Heterocyclylalkinyloxycarbonyl-N-alkylamino, Formyl, Halogen, Halogenalkyl, Halogenalkenyl, Halogenalkinyl, Halogenalkoxy, Halogenalkenyloxy, Halogenalkinyloxy, Halogenalkylthio, Halogenalkenylthio, Halogenalkinylthio, Halogenalkylamino, Halogenalkenylamino, Halogenalkinylamino, Halogenalkylsulfonyl, Halogenalkenylsulfonyl, Halogenalkinylsulfonyl, Halogenalkylsulfinyl, Halogenalkenylsulfinyl, Halogenalkinylsulfinyl, Halogenalkylcarbonyl, Halogenalkenylcarbonyl, Halogenalkinylcarbonyl, Halogenalkylcarbonyloxy, Halogenalkenylcarbonyloxy, Halogenalkinylcarbonyloxy, Halogenalkoxycarbonyl, Halogenalkenyloxycarbonyl, Halogenalkinyloxycarbonyl, Halogenalkylaminocarbonyl, Halogenalkenylaminocarbonyl, Halogenalkinylaminocarbonyl, Halogenalkoxycarbonylamino, Halogenalkenyloxycarbonylamino, Halogenalkinyloxycarbonylamino, Alkoxyalkoxy, Arylalkoxyalkoxy, Cyano, Nitro, oder einen Rest aus der Gruppe Alkyl-NH-N=CH-, Aryl-(CH₂)ₙ-NH-N=CH-, Alkoxy-N=CH-, Aryl-(CH₂)ₙ-O-N=CH-, Alkyl-NH-NH-CO- und Arylalkyl-NH-NH-CO-.

R¹ bedeutet besonders bevorzugt ein gegebenenfalls ein- oder mehrfach, gleich oder verschieden durch Halogen, Nitro, Cyano, Alkyl, Cycloalkyl, Alkenyl, Alkinyl, Alkoxy, Alkylcarbonyl, Alkoxycarbonyl, Alkylcarbonyloxy, Alkylthio, Alkylsulfinyl, Alkylsulfonyl, Alkylsulfonyloxy, Aryl, Aryloxy, Heteroaryl, Heterocyclyl, Heterocyclylalkyl, Benzyl - wobei die siebzehn letztgenannten Gruppen gegebenenfalls durch einen oder mehrere, gleiche oder verschiedene Reste aus der Gruppe Halogen, Nitro, Cyano, Alkyl, Halogenalkyl, Cycloalkyl, Alkenyl, Alkinyl, Alkoxy, Halogenalkoxy und Alkylthio substituiert sein können - substituiertes Heteroaryl oder Aryl.

Als Substituenten, mit denen der Aryl- oder Heteroarylrest der Gruppe R¹ gegebenenfalls substituiert ist, sind ganz besonders bevorzugt Halogen, Nitro, Cyano, (C₁-C₄)-Alkyl, (C₁-C₄)-Halogenalkyl, (C₂-C₄)-Halogenalkenyl, (C₂-C₄)-Halogenalkinyl, (C₁-C₄)-Halogenalkoxy, (C₂-C₄)-Halogenalkenyloxy, (C₂-C₄)-Halogenalkinyloxy, gegebenenfalls substituiertes Aryl, Heteroaryl, (C₁-C₄)-Alkoxycarbonyl, (C₁-C₄)-Alkoxy, (C₁-C₄)-Alkylthio, (C₁-C₄)-Alkylcarbonyloxy, (C₁-C₄)-Alkylsulfonyl, Aryloxy, (C₁-C₄)-Alkylcarbonyl, Heterocyclyl, Heterocyclylalkyl, wobei Aryl, Heteroaryl und/oder Heterocyclylsysteme gegebenenfalls durch einen oder mehrere Reste aus der Gruppe Halogen, (C₁-C₄)-Alkyl, (C₁-C₄)-Halogenalkyl oder (C₁-C₄)-Alkoxy substituiert sein können, oder zwei Substituenten bilden zusammen eine Gruppe -O-CH₂-O- oder -O-(CH₂)₂-O-.

Insbesondere bevorzugt sind Halogen, (C₁-C₄)-Alkyl, (C₁-C₄)-Halogenalkyl, Nitro, Cyano, (C₁-C₄)-Alkoxycarbonyl, (C₁-C₄)-Alkoxy, (C₁-C₄)-Alkylthio, Phenyl, (C₁-C₄)-Alkylcarbonyloxy, (C₁-C₄)-Alkylsulfonyl, Phenoxy, (C₁-C₄)-Alkylcarbonyl, (C₁-C₄)-Alkyl-piperidin-1-yl, wobei Phenyl und Piperidylreste gegebenenfalls durch einen oder mehrere Reste aus der Gruppe Halogen, (C₁-C₄)-Alkyl, (C₁-C₄)-Alkoxy oder (C₁-C₄)-Halogenalkyl substituiert sind, oder daß zwei Substituenten zusammen eine Gruppe -O-CH₂-O- oder -O-(CH₂)₂-O- bilden.
Aus der letztgenannten Gruppe von Substituenten sind bevorzugt: F, Cl, CF₃, Methyl, Ethyl, i-Propyl, n-Propyl, Nitro, Cyano, -COOCH₃, -OCH₃, -OC₂H₅, -SCH₃, Phenyl, o-Phenyl, -O-C(O)-i-Propyl, -SO₂CH₃ -C(O)CH₃, -O-CH₂-O- und -CH₃-(4-Methyl-piperidin-1-yl).

In der Gruppe R¹ ist der Aryl- oder Heteroaryl-Rest bevorzugt aus der Gruppe 2-Thienyl, 3-Thienyl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 6-Pyrimidinyl, 2-Pyrazinyl, 1,3,5-Triazin-2-yl, 1,2,4-Triazin-3-yl, Chinolinyl, Isochinolinyl, Chinazolinyl, Chinoxalinyl, Naphthyl oder Phenyl, insbesondere aus der Gruppe Phenyl, 2-Pyridyl, 3-Pyridyl, 4-Pyridyl, 2-Pyrimidinyl, 4-Pyrimidinyl, 5-Pyrimidinyl, 2-Thienyl, 3-Thienyl und Chinolin-4-yl.

A bedeutet eine Gruppe CR⁴R⁵, oder C=O, wobei
R⁴ bevorzugt Wasserstoff, Fluor oder Methyl, besonders bevorzugt Wasserstoff oder Fluor und ganz besonders bevorzugt Wasserstoff, bedeutet;
R⁵ bevorzugt Wasserstoff, Fluor, (C₁-C₄)-Alkyl, (C₂-C₄)-Alkenyl, (C₂-C₄)-Alkinyl, (C₃-C₆)-Cycloalkyl, (C₃-C₈)-Cycloalkenyl, (C₃-C₆)-Cycloalkyl-(C₁₋C₄)-alkyl, Aryl, Aryl-(C₁-C₄)-alkyl, Heteroaryl, Heteroaryl-(C₁-C₄)-alkyl, besonders bevorzugt Wasserstoff, Fluor oder Methyl und ganz besonders bevorzugt Wasserstoff bedeutet.

R² und R³ bilden gemeinsam mit dem Stickstoffatom, an das sie gebunden sind, eine N-Heterocyclyl- oder N-Heteroarylgruppe, gegebenenfalls ein- oder mehrfach, gleich oder verschieden substituiert durch Alkyl, Alkenyl, Alkinyl, Cycloalkyl, Cycloalkenyl, Cycloalkylalkyl, Cycloalkylalkenyl, Cycloalkylalkinyl, Aryl, Arylalkyl, Arylalkenyl, Arylalkinyl, Heteroaryl, Heteroarylalkyl, Heteroarylalkenyl, Heteroarylalkinyl, Heterocyclyl, Heterocyclylalkyl, Heterocyclylalkenyl, Heterocyclylalkinyl, Hydroxy, Alkoxy, Alkenyloxy, Alkinyloxy, Cycloalkoxy, Cycloalkylalkoxy, Cycloalkylalkenyloxy, Cycloalkylalkinyloxy, Cycloalkenyloxy, Aryloxy, Arylalkoxy, Arylalkenyloxy, Arylalkinyloxy, Heteroaryloxy, Heteroarylalkoxy, Heteroarylalkenyloxy, Heteroarylalkinyloxy, Heterocyclyloxy, Heterocyclylalkoxy, Heterocyclylalkenyloxy, Heterocyclylalkinyloxy, Thio, Alkylthio, Alkenylthio, Alkinylthio, Cycloalkylthio, Cycloalkylalkylthio, Cycloalkylalkenylthio, Cycloalkylalkinylthio, Cycloalkenylthio, Arylthio, Arylalkylthio, Arylalkenylthio, Arylalkinylthio, Heteroarylthio, Heteroarylalkylthio, Heteroarylalkenylthio, Heteroarylalkinylthio, Heterocyclylthio, Heterocyclylalkylthio, Heterocyclylalkenylthio, Heterocyclylalkinylthio, Amino, gegebenenfalls substituiertes Mono- oder Dialkylamino, gegebenenfalls substituiertes Mono- oder Diarylamino, gegebenenfalls substituiertes Mono- oder Di-heteroarylamino, gegebenenfalls substituiertes N-Alkyl-N-arylamino, gegebenenfalls substituiertes N-Alkyl-N-heteroarylamino, Alkenylamino, Alkinylamino, Cycloalkylamino, Cycloalkenylamino, Heterocyclylalkylamino, Heterocyclylalkenylamino, Alkylsulfonyl, Alkenylsulfonyl, Alkinylsulfonyl, Cycloalkylsulfonyl, Cycloalkylalkylsulfonyl, Cycloalkylalkenylsulfonyl, Cycloalkylalkinylsulfonyl, Arylsulfonyl, Arylalkylsulfonyl, Arylalkenylsulfonyl, Arylalkinylsulfonyl, Heteroarylsulfonyl, Heteroarylalkylsulfonyl, Heteroarylalkenylsulfonyl, Heteroarylalkinylsulfonyl, Heterocyclylsulfonyl, Heterocyclylalkylsulfonyl, Heterocyclylalkenylsulfonyl, Heterocyclylalkinylsulfonyl, Alkylsulfinyl, Alkenylsulfinyl, Alkinylsulfinyl, Cycloalkylsulfinyl, Cycloalkylalkylsulfinyl, Cycloalkylalkenylsulfinyl, Cycloalkylalkinylsulfinyl, Arylsulfinyl, Arylalkylsulfinyl, Arylalkenylsulfinyl, Arylalkinylsulfinyl, Heteroarylsulfinyl, Heteroarylalkylsulfinyl, Heteroarylalkenylsulfinyl, Heteroarylalkinylsulfinyl, Heterocyclylsulfinyl, Arylalkylsulfinyl, Heterocyclylalkenylsulfinyl, Heterocyclylalkinylsulfinyl, Aminosulfonyl, gegebenenfalls substituiertes Mono- oder Dialkylaminosulfonyl, gegebenenfalls substituiertes Mono- oder Diarylaminosulfonyl, gegebenenfalls substituiertes Mono- oder Di-Heteroarylaminosulfonyl, gegebenenfalls substituiertes N-Alkyl-N-arylaminosulfonyl, gegebenenfalls substituiertes N-Alkyl-N-heteroarylaminosulfonyl, Alkylsulfonyloxy, Alkenylsulfonyloxy, Alkinylsulfonyloxy, Cycloalkylsulfonyloxy, Cycloalkylalkylsulfonyloxy, Cycloalkylalkenylsulfonyloxy, Cycloalkylalkinylsulfonyloxy, Arylsulfonyloxy, Arylalkylsulfonyloxy, Arylalkenylsulfonyloxy, Arylalkinylsulfonyloxy, Heteroarylsulfonyloxy, Heteroarylalkylsulfonyloxy, Heteroarylalkenylsulfonyloxy, Heteroarylalkinylsulfonyloxy, Heterocyclylsulfonyloxy, Heterocyclylalkylsulfonyloxy, Heterocyclylalkenylsulfonyloxy, Heterocyclylalkinylsulfonyloxy, Alkylsulfonylamino, Alkenylsulfonylamino, Alkinylsulfonylamino, Cycloalkylsulfonylamino, Cycloalkylalkylsulfoamino, Cycloalkylalkenylsulfonylamino, Cycloalkylalkinylsulfonylamino, Arylsulfonylamino, Arylalkylsulfonylamino, Arylalkenylsulfonoamino, Arylalkinylsulfonylamino, Heteroarylsulfonylamino, Heteroarylalkylsulfonylamino, Heteroarylalkenylsulfonoamino, Heteroarylalkinylsulfonylamino, Alkylsulfonyl-N-alkylamino, Alkenylsulfonyl-N-alkylamino, N-Alkyl-alkinylsulfonyl-N-alkylamino, Cycloalkylsulfonyl-N-alkylamino, Cycloalkylalkylsulfonyl-N-alkylamino, Cycloalkylalkenylsulfonyl-N-alkylamino, Cycloalkylalkinylsulfonyl-N-alkylamino, Arylsulfonyl-N-alkylamino, Heteroarylsulfonyl-N-alkylamino, Arylalkylsulfonyl-N-alkylamino, Heteroarylalkylsulfonyl-N-alkylamino, Arylalkenylsulfonyl-N-alkylamino, Heteroarylalkenylsulfonyl-N-alkylamino, Arylalkinylsulfonyl-N-alkylamino, Heteroarylalkinylsulfonyl-N-alkylamino, Heterocyclylsulfonyl-N-alkylamino, Heterocyclylalkylsulfonyl-N-alkylamino, Heterocyclylalkenylsulfonyl-N-alkylamino, Heterocyclylalkinylsulfonyl-N-alkylamino, Alkylcarbonyl, Alkenylcarbonyl, Alkinylcarbonyl, Cycloalkylcarbonyl, Cycloalkylalkylcarbonyl, Cycloalkylalkenylcarbonyl, Cycloalkylalkinylcarbonyl, Arylcarbonyl, Arylalkylcarbonyl, Arylalkenylcarbonyl, Arylalkinylcarbonyl, Heteroarylcarbonyl, Heteroarylalkylcarbonyl, Heteroarylalkenyl, Heteroarylalkinylcarbonyl, Heterocyclylcarbonyl, Heterocyclylalkylcarbonyl, Heterocyclylalkenyl, Heterocyclylalkinylcarbonyl, Formyloxy, Alkylcarbonyloxy, Alkenylcarbonyloxy, Alkinylcarbonyloxy, Cycloalkylcarbonyloxy, Cycloalkylalkylcarbonyloxy, Cycloalkylalkenylcarbonyloxy, Cycloalkylalkinylcarbonyloxy, Arylcarbonyloxy, Arylalkylcarbonyloxy, Arylalkenylcarbonyloxy, Arylalkinylcarbonyloxy, Heteroarylcarbonyloxy, Heteroarylalkylcarbonyloxy, Heteroarylalkenylcarbonyloxy, Heteroarylalkinylcarbonyloxy, Heterocyclylcarbonyloxy, Heterocyclylalkylcarbonyloxy, Heterocyclylalkenyloxy, Heterocyclylalkinylcarbonyloxy, Carboxyl, Alkoxycarbonyl, Alkenyloxycarbonyl, Alkinyloxycarbonyl, Cycloalkoxycarbonyl, Cycloalkylalkoxycarbonyl, Cycloalkylalkenyloxycarbonyl, Cycloalkylalkinyloxycarbonyl, Aryloxycarbonyl, Arylalkoxycarbonyl, Arylalkenyloxycarbonyl, Arylalkinyloxycarbonyl, Heteroaryloxycarbonyl, Heteroarylalkoxycarbonyl, Heteroarylalkenyloxycarbonyl, Heteroarylalkinyloxycarbonyl, Heterocyclyloxycarbonyl, Heterocyclylalkoxycarbonyl, Heterocydylalkenyloxycarbonyl, Heterocyclylalkinyloxycarbonyl, Aminocarbonyl, gegebenenfalls substituiertes Mono- oder Dialkylaminocarbonyl, gegebenenfalls substituiertes Mono- oder Diarylaminocarbonyl, gegebenenfalls substituiertes Mono- oder Di-heteroarylaminocarbonyl, gegebenenfalls substituiertes N-Alkyl-N-arylaminocarbonyl, gegebenenfalls substituiertes N-Alkyl-N-heteroarylaminocarbonyl, gegebenenfalls substituiertes Alkylcarbonylamino, gegebenenfalls substituiertes Alkylcarbonyl-N-alkylamino, gegebenenfalls substituiertes Arylcarbonylamino, gegebenenfalls substituiertes Arylcarbonyl-N-arylamino, gegebenenfalls substituiertes Heteroarylcarbonylamino, gegebenenfalls substituiertes Heteroarylcarbonyl-N-heteroarylamino, gegebenenfalls substituiertes Alkylcarbonyl-N-arylamino, gegebenenfalls substituiertes Arylcarbonyl-N-alkylamino, gegebenenfalls substituiertes Alkylcarbonyl-N-heteroarylamino, gegebenenfalls substituiertes Heteroarylcarbonyl-N-alkylamino, Alkoxycarbonylamino, Alkenyloxycarbonylamino, Alkinyloxycarbonylamino, Cycloalkoxycarbonylamino, Cycloalkytalkoxycarbonylamino, Cycloalkylalkenyloxycarbonylamino, Cycloalkylalkinyloxycarbonylamino, Aryloxycarbonylamino, Arylalkoxycarbonylamino, Arylalkenyloxycarbonylamino, Arylalkinyoxycarbonylamino, Heteroaryloxycarbonylamino, Heteroarylalkoxycarbonylamino, Heteroarylalkenyloxycarbonylamino, Heteroarylalkinyloxycarbonylamino, Heterocyclyloxycarbonylamino, Heterocyclylalkoxycarbonylamino, Heterocyclylalkenyloxycarbonylamino, Heterocyclylalkinyloxycarbonylamino, Alkoxycarbonyl-N-alkylamino, Alkenyloxycarbonyl-N-alkylamino, Alkinyloxycarbonyl-N-alkylamino, Cycloalkoxycarbonyl-N-alkylamino, Cycloalkylalkoxycarbonyl-N-alkylamino, Cycloalkylalkenyloxycarbonyl-N-alkylamino, Cycloalkylalkinyloxycarbonyl-N-alkylamino, Aryloxycarbonyl-N-alkylamino, Arylalkoxycarbonyl-N-alkylamino, Arylalkenyloxycarbonyl-N-alkylamino, Arylalkinyloxycarbonyl-N-alkylamino, Heteroarylalkoxycarbonyl-N-alkylamino, Heteroarylalkenyloxycarbonyl-N-alkylamino, Heteroarylalkinyloxycarbonyl-N-alkylamino, Heterocyclylalkoxycarbonyl-N-alkylamino, Heterocyclylalkenyloxycarbonyl-N-alkylamino, Heterocyclylalkinyloxycarbonyl-N-alkylamino, Formyl, Halogen, Halogenalkyl, Halogenalkenyl, Halogenalkinyl, Halogenalkoxy, Halogenalkenyloxy, Halogenalkinyloxy, Halogenalkylthio, Halogenalkenylthio, Halogenalkinylthio, Halogenalkylamino, Halogenalkenylamino, Halogenalkinylamino, Halogenalkylsulfonyl, Halogenalkenylsulfonyl, Halogenalkinylsulfonyl, Halogenalkylsulfinyl, Halogenalkenylsulfinyl, Halogenalkinylsulfinyl, Halogenalkylcarbonyl, Halogenalkenylcarbonyl, Halogenalkinylcarbonyl, Halogenalkylcarbonyloxy, Halogenalkenylcarbonyloxy, Halogenalkinylcarbonyloxy, Halogenalkoxycarbonyl, Halogenalkenyloxycarbonyl, Halogenalkinyloxycarbonyl, Halogenalkylaminocarbonyl, Halogenalkenylaminocarbonyl, Halogenalkinylaminocarbonyl, Halogenalkoxycarbonylamino, Halogenalkenyloxycarbonylamino, Halogenalkinyloxycarbonylamino, Alkoxyalkoxy, Arylalkoxyalkoxy, Cyano, Nitro, oder einen Rest aus der Gruppe Alkyl-NH-N=CH-, Aryl-(CH₂)ₙNH-N=CH-, Alkoxy-N=CH-, Aryl-(CH₂)ₙO-N=CH-, Alkyl-NH-NH-CO- und Arylalkyl-NH-NH-CO-.

R² und R³ bilden besonders bevorzugt gemeinsam mit dem Stickstoffatom, an das sie gebunden sind, ein gegebenenfalls ein- oder mehrfach, gleich oder verschieden durch (C₁-C₄)-Alkyl, (C₂-C₄)-Alkenyl, (C₂-C₄)-Alkinyl, (C₃-C₆)-Cycloalkyl, (C₃-C₆)-Heterocyclyl, (C₃-C₈)-Cycloalkenyl, (C₃-C₆)-Cycloalkyl-(C₁-C₄)-alkyl, Aryl-(C₁-C₄)-alkyl, Heteroaryl-(C₁-C₄)-alkyl, (C₁-C₄)-Alkylcarbonyl, (C₁-C₄)-Alkylcarbonyloxy, (C₁-C₄)-Alkylsulfonyl, (C₁-C₄)-Alkylsulfonyloxy, (C₁-C₄)-Alkoxycarbonyl, SCN, (C₁-C₄)-Dialkylamino, Formyl, - wobei die genannten Gruppen gegebenenfalls durch ein oder mehrere, gleiche oder verschiedene Reste aus der Gruppe Halogen, Nitro, Cyano, Alkyl, Halogenalkyl, Cycloalkyl, Alkenyl, Alkinyl, Phenyl, Alkoxy, Halogenalkoxy und Alkylthio substituiert sind - substituiertes N-Heterocyclyl oder N-Heteroaryl. Zwei Substituenten zusammen bilden gegebenenfalls eine Gruppe -O-CH₂-O- oder -O-(CH₂)₂-O- oder, am selben C-Atom, =O.

R² und R³ bilden ganz besonders bevorzugt gemeinsam mit dem Stickstoffatom, an das sie gebunden sind, ein gegebenenfalls ein- oder mehrfach, gleich oder verschieden durch (C₁-C₄)-Alkyl, (C₂-C₄)-Alkenyl, (C₂-C₄)-Alkinyl, (C₃-C₆)-Cycloalkyl, (C₃-C₆)-Heterocyclyl, (C₃-C₈)-Cycloalkenyl, (C₃-C₆)-Cycloalkyl-(C₁₋C₄)-alkyl, Aryl-(C₁-C₄)-alkyl, Heteroaryl-(C₁-C₄)-alkyl, (C₁-C₄)-Alkylcarbonyl, (C₁₋C₄)-Alkylcarbonyloxy, (C₁-C₄)-Alkoxycarbonyl, (C₁-C₄)-Alkylsulfonyl, (C₁-C₄)-Alkylsulfonyloxy, - wobei die genannten Gruppen gegebenenfalls durch ein oder mehrere, gleiche oder verschiedene Reste aus der Gruppe Halogen, Nitro, Cyano, Alkyl, Halogenalkyl, Cycloalkyl, Alkenyl, Alkinyl, Alkoxy, Halogenalkoxy und Alkylthio substituiert sind - SCN, (C₁-C₄)-Dialkylamino, Formyl, =O, -O-CH₂-O-, -O-(CH₂)₂-O-substituiertes N-Heterocyclyl,oder N-Heteroaryl.

R² und R³ bilden bevorzugt gemeinsam mit dem Stickstoffatom, an das sie gebunden sind, ein gegebenenfalls ein- oder mehrfach, gleich oder verschieden durch die oben genannten Substituenten substituiertes Piperidin, Piperazin, Morpholin, Azepam, Decahydrochinolin, 1,2,5,6-Tetrahydropyridin oder 6-Azabicyclo[3.2.1]octan, besonders bevorzugt ein Piperidin.

Je nach Art der oben definierten Substituenten weisen die Verbindungen der allgemeinen Formel (I) saure oder basische Eigenschaften auf und können Salze bilden. Bevorzugt sind dabei die auf dem Gebiet der Schädlingsbekämpfung gängigen und tolerablen Salze. Tragen die Verbindungen der allgemeinen Formel (I) beispielsweise Gruppen wie Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit (C₁-C₄)-Alkylresten sowie Mono-, Di- und Trialkanolamine von (C₁-C₄)-Alkanolen. Tragen die Verbindungen der allgemeinen Formel (I) beispielsweise Gruppen wie Amino, Alkylamino oder andere, basische Eigenschaften induzierende Gruppen, so können diese Verbindungen mit Säuren zu Salzen umgesetzt werden. Geeignete Säuren sind beispielsweise Mineralsäuren, wie Salz-, Schwefel- und Phosphorsäure, organische Säuren, wie Essigsäure oder Oxalsäure, und saure Salze, wie NaHSO₄ und KHSO₄. Die so erhältlichen Salze weisen ebenfalls insektizide, akarizide und/oder helminthizide/nematizide Eigenschaften auf.

Die Verbindungen der allgemeinen Formel (I) können ein oder mehrere asymmetrische Kohlenstoffatome oder Stereoisomere an Doppelbindungen aufweisen. Es können daher Enantiomere oder Diastereomere auftreten. Die Erfindung umfaßt sowohl die reinen Isomeren als auch deren Gemische. Die Gemische von Diastereomeren können nach gebräuchlichen Methoden, z.B. durch selektive Kristallisation aus geeigneten Lösungsmitteln oder durch Chromatographie in die Isomeren aufgetrennt werden. Racemate können nach üblichen Methoden in die Enantiomeren aufgetrennt werden.

Die Herstellung der erfindungsgemäßen Verbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

In allen nachfolgend genannten Formeln haben die Substituenten und Symbole, sofern nicht anders definiert, dieselbe Bedeutung wie unter Formel (I) beschrieben.

Die Verbindungen der Formel (I) können je nach Bedeutung der Substituenten beispielsweise nach einem oder mehreren der in den folgenden Schemata angegebenen Verfahren hergestellt werden.

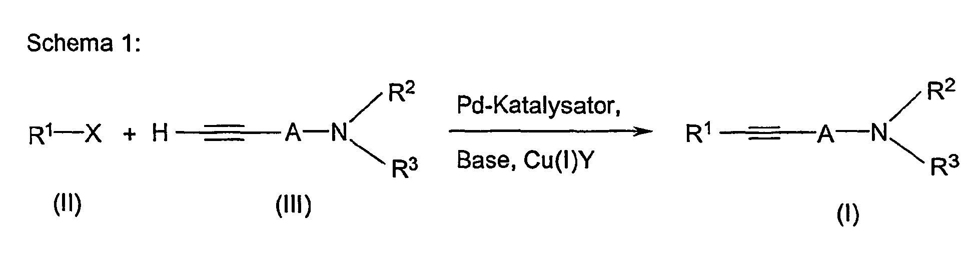

Durch die in Schema 1 angegebene palladiumkatalysierte Kreuzkupplung (Sonogashira-Kupplung) einer Verbindung der Formel (II), in der X für -OSO₂CF₃ oder Halogen, bevorzugt lod, steht, mit einer Verbindung der Formel (III) erhält man die erfindungsgemäßen Verbindungen der Formel (I). Diese Reaktion wird bevorzugt in Gegenwart einer Aminbase, z.B. Diethylamin, Triethylamin, Piperidin, Pyrrolidin oder DBU, die neben anderen Lösungsmitteln, wie Benzol, Toluol, DMF, THF oder Diethylether auch selbst als Lösungsmittel dienen kann, oder einer anderen geeigneten Base , z.B. eines Alkalimetallalkoxids, wie Kaliumtertiärbutylat in einem inerten organischen Lösungsmittel, wie DMSO, Acetonitril; Lösungsmittel und eines Palladiumkatalysators sowie eines Kupfer(I)salzes (Y ist vorzugsweise I, Cl, Br, Cyano oder SCN), bevorzugt Kupfer(I)iodid (Y= I), durchgeführt. Als Palladiumkatalysator-Systeme eignen sich zum Beispiel Pd(PPh₃)₄, PdCl₂(PPh₃)₂ und Pd(OAc)₂(PPh₃)₂ oder Pd(OAc)₂ und ein Triarylphosphin, bevorzugt Triphenylphosphin oder Tri-(o-tolyl)phosphin. Diese Methoden sind beispielsweise in *Tetrahedron Lett.* 4467 (1975), *Comprehensive Organic Synthesis* (B. M. Trost, I. Flemming, Eds), Pergamon Press, Oxford, Vol 3, 521-549 (1991), *Org. Prep. Proct. Int.* 129 (1995), *J. Med. Chem.* 40, 3542 (1997), *J. Org. Chem.* 63, 1109 (1998) beschrieben.

Verbindungen der Formel (II) sind entweder kommerziell erhältlich oder können gemäß allgemein bekannten Methoden, z.B. Houben-Weyl, Methoden der Organischen Chemie, Georg-Thieme-Verlag, Stuttgart oder *Chem. Pharm. Bull.* 27, 270 (1979), hergestellt werden.

Verbindungen der Formel (III) können beispielsweise gemäß Schema 2 aus Verbindungen der Formel (IV), in der X' für eine Abgangsgruppe z.B. ein Chlor-, Brom- oder lodatom sowie ein Alkylsulfonyloxy- oder Arylsulfonyloxyradikal, beispielsweise ein Tosyloxyradikal, steht, hergestellt werden.

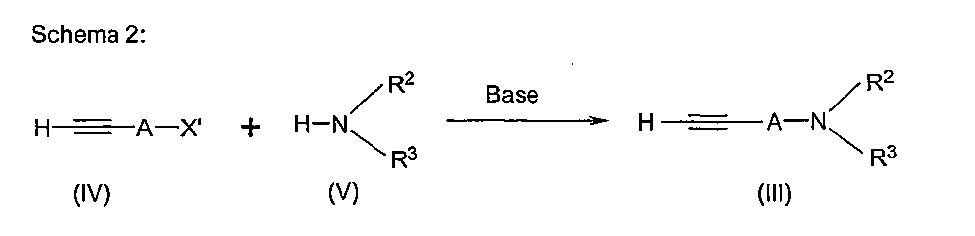

Man erhält die Verbindungen der Formel (III) in Gegenwart einer anorganischen oder organischen Base, wie Kaliumcarbonat oder überschüßigem Amin der Formel (V), in einem inerten organischen Lösungsmittel, wie Methanol, Aceton oder DMF. Solche Methoden sind beispielsweise aus *Org. Magn. Reson.* 14, 161 (1980), *Tetrahedron* 41, 5685 (1985), *J*. *Med. Chem.* 34, 746 (1991), *J*. *Org. Chem.* 56, 3707 (1991), *J*. *Org. Chem.* 57, 3000 (1992), *J*. *Med. Chem.* 37, 2735 (1994) bekannt.

Verbindungen der Formel (I), in denen A für CR⁴R⁵ steht, wobei R⁵ hier Wasserstoff bedeutet, insbesondere in denen A für CH₂ steht, sind beispielsweise auch gemäß Schema 3 erhältlich.

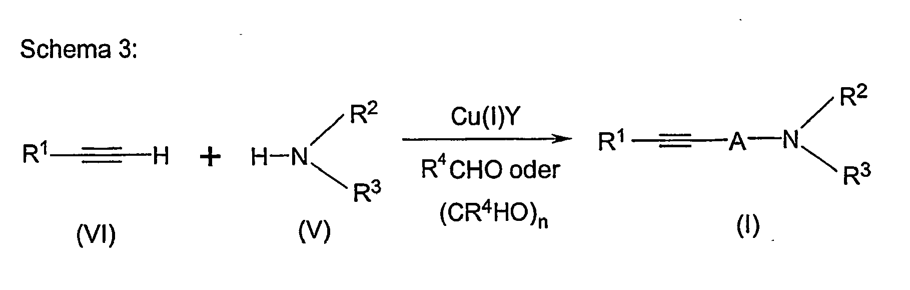

Man erhält die Verbindungen der Formel (I) gemäß Schema 3 durch Umsetzung von Verbindungen der Formel (V) mit Verbindungen der Formel (VI) und einem Aldehyd, wie beispielsweise Benzaldehyd, Acetaldehyd oder Formaldehyd oder einer Aldehydquelle, wie Paraformaldehyd oder Formalin in Gegenwart eines Kupfer(I)salzes, bevorzugt Kupfer(I)chlorid (Y= Cl), in einem inerten organischen Lösungsmittel wie zum Beispiel Dioxan. Solche Methoden sind beispielsweise aus *Chem. Ber.* 66,418 (1933), *J*. *prakt. Chem.* 331, 187 (1989), *Tetrahedron Lett.* 39, 967 (1998) bekannt.

Verbindungen der Formel (VI) sind entweder kommerziell erhältlich oder durch Sonogashira-Kupplung und anschließende Abspaltung der Schutzgruppe aus R¹ X und einem geschützten Acetylenäquivalent, wie beispielsweise Trimethylsilylacetylen oder 2-Methyl-3-butin-2-ol, gemäß bekannten Methoden zugänglich. Solche Methoden sind beispielsweise in *Comprehensive Organic Synthesis* (B. M. Trost, I. Flemming, Eds), Pergamon Press, Oxford, Vol 3, 521-549 (1991), *Synthesis.* (1980), 627, *J. Org. Chem.* 50, 1763 (1985), *Tetrahedron Lett.* 34, 2071 (1993), *Angew. Chem. Int. Ed. Engl.* 18, 406 (1993), *Synthesis* (1996), 589 beschrieben.

Insbesondere Verbindungen der Formel (I), in der A für CR⁴R⁵ steht, sind beispielsweise auch gemäß Schema 4 erhältlich.

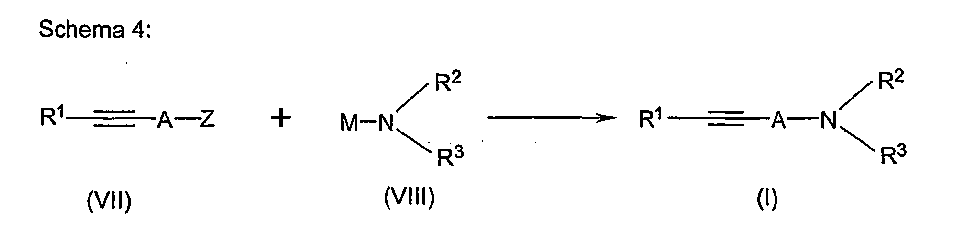

Man erhält die Verbindungen der Formel (I) gemäß Schema 4 durch Umsetzung von Verbindungen der Formel (VII), in denen Z ein Chlor-, Brom- oder lodatom oder ein Alkylsulfonyloxy- oder Arylsulfonyloxyradikal, beispielsweise ein Tosyloxyradikal, bedeutet, mit Verbindungen der Formel (VIII), in denen M ein Alkalimetallatom, vorzugsweise ein Lithiumatom, bedeutet. Falls Z in der Formel (VII) Chlor, Brom oder lod bedeutet, kann M in der Formel (VIII) darüber hinaus auch ein Wasserstoffatom bedeuten. Wenn M in der Formel (VIII) ein Wasserstoffatom bedeutet, findet die Reaktion in Gegenwart einer anorganischen Base, wie Kaliumcarbonat, und in einem inerten organischen Lösungsmittel, wie Aceton oder DMF, statt. Im Falle, daß M in der Formel (VIII) eine andere Bedeutung als Wasserstoff hat, sind inerte organische Lösungsmittel, wie Diethylether oder THF, bevorzugt.
Verbindungen der Formel (VII) können nach oder analog bekannten Methoden hergestellt werden. Diese Methoden sind beispielsweise aus *Chem. Phys. Lipids* 13, 159 (1974), *Synthesis* (1975), 255, *J*. *Am. Chem. Soc*. 60, 2662 (1938), *Bull. Chem. Soc. Jpn.* 46, 954 (1973), *J. Med. Chem.* 21, 253 (1978), *Bull. Soc. Chim. Fr.* (1969), 4514, *J*. *Org. Chem.* 49,4344 (1984), *J. Med. Chem.* 41,1084 (1998), *J. Org. Chem.* 63, 7472 (1998) bekannt.

Kollektionen aus Verbindungen der Formel (I), die nach oben genannten Schemata synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S. H. DeWitt in "Annual Reports in Combinatorial Chemistry and Molecular Diversity: Automated Synthesis", Band 1, Verlag Escom, 1997, Seite 69 bis 77, beschrieben ist.

Zur parallelisierten Reaktionsdurchführung und Aufarbeitung können eine Reihe von im Handel erhältlichen Geräten verwendet werden, wie sie beispielsweise von den Firmen Stem Corporation, Woodrolfe Road, Tollesbury, Essex, CM9 8SE, England, oder H+P Labortechnik GmbH, Bruckmannring 28, 85764 Oberschleißheim, Deutschland, oder der Firma Radleys, Shirehill, Saffron Walden, Essex, England, angeboten werden. Für die parallelisierte Aufreinigung von Verbindungen der allgemeinen Formel (I) beziehungsweise von bei der Herstellung anfallenden Zwischenprodukten stehen unter anderem Chromatographieapparaturen zur Verfügung, beispielsweise der Firma ISCO Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständige integrierten Automationssystemen umgangen werden, bei denen die jeweiligen Automationsmodule beispielsweise durch Roboter bedient werden. Derartige Automationssysteme können zum Beispiel von der Firma Zymark Corporation, Zymark Center, Hopkinton, MA 01748, USA, bezogen werden.

Neben den hier beschriebenen Verfahren kann die Herstellung von Verbindungen der allgemeinen Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z.B. Barry A. Bunin in "The Combinatorial Index", Verlag Academic Press, 1998.

Die Verwendung von Festphasen unterstützten Synthesemethoden erlaubt eine Reihe von literaturbekannten Protokollen, die wiederum manuell oder automatisierten ausgeführt werden können. Zum Beispiel kann die "Teebeutelmethode" (Houghten, US 4,631,211; Houghten et al., Proc. Natl. Acad. Sci, 1985, 82, 5131-5135) mit Produkten der Firma IRORI, 11149 North Torrey Pines Road, La Jolla, CA 92037, USA, teilweise automatisiert werden. Die Automatisierung von Festphasen unterstützten Parallelsynthesen gelingt beispielsweise durch Apparaturen der Firmen Argonaut Technologies Inc., 887 Industrial Road, San Carlos, CA 94070, USA, oder MultiSynTech GmbH, Wullener Feld 4, 58454 Witten, Deutschland.

Die Herstellung gemäß der hier beschriebenen Verfahren liefert Verbindungen der Formel (I) in Form von Substanzkollektionen, die Bibliotheken genannt werden. Gegenstand der vorliegenden Erfindung sind auch Bibliotheken, die mindestens zwei Verbindungen der Formel (I) enthalten.

Die Verbindungen der Formel (I) eignen sich bei guter Pflanzenverträglichkeit und günstiger Warmblütertoxizität zur Bekämpfung von tierischen Schädlingen, insbesondere Arthropoden, wie Insekten und Acarina, und Heleninthen, wie Tierparasiten und pflanzenschädlichen Nematoden, ganz besonders bevorzugt zur Bekämpfung von Insekten und Spinnentieren, die in der Landwirtschaft, im Gartenbau, in Forsten, im Vorrats- und Materialschutz, in der Tierhaltung und - zucht, im häuslichen Bereich sowie auf dem Hygienesektor vorkommen. Sie sind gegen normal sensible und resistente Arten sowie alle oder einzelne Entwicklungsstadien wirksam. Zu den oben erwähnten Schädlingen gehören:

Aus der Ordnung der Isopoda zum Beispiel Armadillidium spp., Oniscus spp., Porcellio spp.
Aus der Ordnung der Diplopoda zum Beispiel Blaniulus spp..
Aus der Ordnung der Chilopoda zum Beispiel Geophilus spp., Scutigera spp..
Aus der Ordnung der Symphyla zum Beispiel Scutigerella spp..
Aus der Ordnung der Thysanura zum Beispiel Lepisma spp..
Aus der Ordnung der Collembola zum Beispiel Onychiurus spp..
Aus der Ordnung der Orthoptera zum Beispiel Blattella spp., Blattella germanica, Blatta orientalis, Periplaneta spp., Periplaneta americana, Periplaneta australasiae, Leucophaea spp., Acheta spp., Acheta domesticus, Gryllotalpa spp., Gryllus spp., Gryllus bimaculatus, Locusta spp., Locusta migratoria migratorioides, Melanoplus spp., Schistocerca spp..
Aus der Ordnung der Dermaptera zum Beispiel Forficula spp., Forficula auricularia.
Aus der Ordnung des Isoptera zum Beispiel Reticulitermes spp., Reticulitermes speratus, Coptotermes spp., Coptotermes formosanus.
Aus der Ordnung der Anoplura zum Beispiel Pediculus spp., Pediculus humanus humanus, Pediculus humanus capitis, Haematopinus spp., Linognathus spp..
Aus der Ordnung der Mallophaga zum Beispiel Trichodectes spp., Damalinea spp..
Aus der Ordnung der Thysanoptera zum Beispiel Frankliniella spp., Frankliniella fusca, Frankliniella occidentalis, Frankliniella tritici, Kakothrips spp., Hercinothrips spp., Scirtothrips spp., Scirtothrips citri, Scirtothrips aurantii, Taeniothrips spp., Thrips spp., Thrips oryzae, Thrips palmi, Thrips tabaci.
Aus der Ordnung der Heteroptera zum Beispiel Eurygaster spp., Stephanitis spp., Lygus spp., Aelia spp., Eurydema spp., Dysdercus spp., Piesma spp. Piesma quadrata, Rhodnius prolixus, Triatoma spp., Cimex lectularius.
Aus der Ordnung der Homoptera zum Beispiel Aleurodes spp., Aleurodes brassicae, Aleurodes proletella, Bemisia spp., Bemisia tabaci, Trialeurodes spp., Trialeurodes vaporariorum, Brevicoryne spp., Brevicoryne brassicae, Cryptomyzus spp., Aphis spp., Aphis fabae, Aphis gossypii, Aphis pomi, Eriosoma spp., Hyalopterus spp., Phylloxera spp., Pemphigus spp., Macrosiphum spp., Macrosiphum avenae, Myzus spp., Myzus persicae, Phorodon spp., Phorodon humuli, Rhopalosiphum spp., Rhopalosiphum padi, Empoasca spp., Euscelis spp., Eulecanium spp., Saissetia spp., Aonidiella spp., Aonidiella aurantii, Aspidiotus spp., Nephotettix spp., Nephotettix cincticeps, Laodelphax spp., Laodelphax striatellus, Nilaparvata spp., Nilaparvata lugens, Sogatella spp., Pseudococcus spp., Psylla spp., Psylla mali, Aphrophora spp., Aeneolamia spp.
Aus der Ordnung der Lepidoptera zum Beispiel Pectinophora spp., Pectinophora gossypiella, Bupalus spp., Cheimatobia spp., Cnephasia spp., Hydraecia spp., Lithocolletis spp., Hyponomeuta spp., Plutella spp., Plutella xylostella, Malacosoma spp., Euproctis spp., Lymantria spp., Bucculatrix spp., Phytometra spp., Scrobipalpa spp., Phthorimaea spp., Gnorimoschema spp., Autographa spp., Evergestis spp., Lacanobia spp., Cydia spp., Cydia pomonella, Pseudociaphila spp., Phyllocnistis spp., Agrotis spp., Agrotis segetum, Agrotis ipsilon, Euxoa spp., Feltia spp., Earias spp., Heliothis spp., Heliothis virescens, Heliothis armigera, Heliothis zea, Helicoverpa spp., Helicoverpa armigera, Helicoverpa zea, Bombyx spp., Bombyx mori, Laphygma spp., Mamestra spp., Mamestra brassicae, Panolis spp., Prodenia spp., Prodenia litura, Spodoptera spp., Spodoptera littoralis, Spodoptera litura, Spodoptera exigua, Trichoplusia spp., Trichoplusia ni, Carpocapsa spp., Carpocapsa pomonella, Pieris spp., Pieris brassicae, Chilo spp., Chilo suppressalis, Ostrinia spp., Ostrinia nubilalis, Pyrausta spp., Pyrausta nubilalis, Ephestia spp., Ephestia kuehniella, Galleria spp., Galleria mellonella, Cacoecia spp., Capua spp., Choristoneura spp., Clysia spp., Hofmannophila spp., Homona spp., Tineola spp., Tinea spp., Tinea pellionella, Tortrix spp. Tortrix vitisana, Lobesia spp., Lobesia botrana.
Aus der Ordnung der Coleoptera zum Beispiel Anobium spp., Rhizopertha spp., Rhizopertha dominica, Bruchidius spp., Bruchidius obtectus, Acanthoscelides spp., Acanthoscelides obtectus, Hylotrupes spp., Aclypea spp., Agelastica spp., Leptinotarsa spp., Leptinotarsa decemlineata, Psylliodes spp., Chaetocnema spp., Cassida spp., Bothynoderes spp., Clivina spp., Ceutorhynchus spp., Ceutorhynchus assimilis, Phyllotreta spp., Apion spp., Sitona spp., Bruchus spp., Phaedon spp., Phaedon cochleariae, Diabrotica spp., Diabrotica undecimpunctata, Diabrotica virgifera, Psylloides spp., Epilachna spp., Epilachna varivestis, Atomaria spp., Atomaria linearis, Oryzaephilus spp., Anthonomus spp., Anthonomus grandis, Sitophilus spp., Sitophilus granarius, Sitophilus oryzae, Otiorhynchus spp., Otiorrhynchus sulcatus, Cosmopolites spp., Ceuthorrynchus spp., Hypera spp., Dermestes spp., Trogoderma spp., Anthrenus spp., Attagenus spp., Lyctus spp., Meligethes spp., Meligethes aeneus, Ptinus spp., Niptus spp., Gibbium spp., Tribolium spp., Tenebrio spp., Tenebrio molitor, Agriotes spp., Agriotes lineatus, Conoderus spp., Melolontha spp., Melolontha melolontha, Amphimallon spp., Costelytra spp., Costelytra zealandica.
Aus der Ordnung der Hymenoptera zum Beispiel Diprion spp., Diprion pini, Hoplocampa spp., Lasius spp., Monomorium spp., Vespa spp..
Aus der Ordnung der Diptera zum Beispiel Drosophila spp., Drosophila melanogaster, Chrysomyxa spp., Hypoderma spp., Tannia spp., Bibio spp., Bibio hortulanus, Oscinella spp., Oscinella frit, Phorbia spp., Pegomyia spp., Anastrepha spp., Ceratitis spp., Dacus spp., Rhagoletis spp., Bactrocera spp., Toxotrypana spp., Tipula spp., Tipula paludosa, Tipula oleracea, Dermatobia spp., Dermatobia hominis, Cordylobia spp., Cordylobia anthropophaga, Gasterophilus spp., Hypoderma spp., Cuterebra spp., Cochliomyia spp., Wohlfahrüa spp., Stomoxys spp., Calliphora spp., Calliphora erythrocephala, Gastrophilus spp., Hyppobosca spp., Lucilia spp., Lucilia sericata, Musca spp., Musca domestica, Fannia spp., Fannia canicularis, Oestrus spp., Tabanus spp., Aedes spp., Aedes aegypti, Culex spp., Culex quinquefasciatus, Anopheles spp., Anopheles arabiensis.
Aus der Ordnung der Siphonaptera zum Beispiel Xenopsylla spp., Xenopsylla cheopsis, Ctenocephalides spp., Ctenocephalides felis, Ctenocephalides canis, Ceratophyllus spp., Pulex spp., Pulex irritans.
Aus der Ordnung der Acarina zum Beispiel Acarus spp., Acarus siro, Bryobia spp., Bryobia praetiosa, Panonychus spp., Panonychus ulmi, Panonychus citri, Tetranychus spp., Tetranychus urticae, Eotetranychus spp., Oligonychus spp., Eutetranychus spp., Eriophyes spp., Eriophyes ribis, Phyllocoptruta spp., Phyllocoptruta oleivora, Tarsonemus spp., Argas spp., Argas reflexus, Argas persicus, Ornithodoros spp., Ornithodoros moubata, Dermacentor spp., Dermacentor marginatus, Hyalomma spp., Dermanyssus spp., Dermanyssus gallinae, Boophilus spp., Boophilus microplus, Haemaphysalis spp., Psoroptes spp., Chorioptes spp., Sarcoptes spp., Rhipicephalus spp., Rhipicephalus sanguineus, Ixodes spp., Ixodes ricinus, Amblyomma spp..
Aus der Klasse der Helminthen zum Beispiel Schistosomen spp., Fasciola spp., Dicrocoelium spp., Opisthorchis spp., Clonorchis spp., Paragonimus spp., Taenia saginata, Taenia solium, Echinococcus granulosus, Echinococcus multilocularis, Hymenolepis nana, Diphyllobothrium latum, Onchocerca volvulus, Wuchereria bancrofti, Brugia malayi, Brugia timori, Loa Loa, Dracunculus medinensis, Enterobius vermicularis, Trichinella spiralis, Trichinella nativa, Trichinella britovi, Trichinella nelsoni, Trichinella pseudopsiralis, Ascaris spp., Ascaris lumbricoides, Trichuris trichuria, Ancylostoma duodenale, Ancylostoma ceylanicum, Ancylostoma braziliensis, Strongyloides stercoralis, Strongyloides fuellebomi, Haemonchus spp., Ostertagia spp., Trichostrongulus spp., Cooperia spp., Bunostomum spp., Nematodirus spp. Chabertia spp., Strongyloides spp., Oesophagostomum spp., Hyostrongulus spp., Ancylostoma spp., Dictyocaulus filaria, Heterakis spp;
und aus der Untergruppe der pflanzenparasitären Nematoden zum Beispiel Meloidogyne spp., Meloidogyne incognita, Meloidogyne hapla, Meloidogyne javanica, Heterodera spp., Heterodera trifolii, Heterodera avenae, Heterodera schachtii, Heterodera glycines, Globodera spp., Globodera rostochiensis, Globodera pallida, Radopholus spp., Radopholus similis, Pratylenchus spp., Pratylenchus neglectus, Pratylenchus penetrans, Pratylenchus curvitatus; Tylenchulus spp., Tylenchulus semipenetrans, Tylenchorhynchus spp., Tylenchorhynchus dubius, Tylenchorhynchus claytoni, Rotylenchus spp., Rotylenchus robustus, Heliocotylenchus spp., Haliocotylenchus multicinctus, Belonoaimus spp., Belonoaimus longicaudatus, Longidorus spp., Longidorus elongatus, Trichodorus spp., Trichodorus primitivus, Xiphinema spp., Xiphinema index, Ditylenchus spp., Ditylenchus dipsaci, Ditylenchus destructor, Aphelenchoides spp., Aphelenchoides ritzemabosi, Anguina spp., Anguina tritici.

Die Verbindungen der Formel (I) eignen sich auch zur Bekämpfung von tierischen Schädlingen, insbesondere Arthropoden, wie Insekten und Acarina, in Räumen, speziell zur Bekämpfung von Fliegen, wie beispielsweise aus den Familien Muscidae (beispielsweise gemeine Hausfliegen, Stubenfliegen), Calliphoridae (beispielsweise Goldfliegen, Totenfliegen (Cynomyia mortuorum), blaue Fleischfliegen) und Sarcophagidae (beispielsweise Fleischfliegen), Moskitos, wie beispielsweise Aedes aegypti, Anopheles arabiensis und Culex quinquefasciatus, und Schaben, wie beispielsweise Blattella germanica und Periplaneta americana.

Die Erfindung betrifft auch Mittel, beispielsweise Schädlingsbekämpfungsmittel, vorzugsweise insektizide, akarizide, ixodizide oder heiminthizide/nematizide, besonders bevorzugt insektizide, akarizide und heiminthizide/nematizide Mittel, die eine oder mehrere Verbindungen der Formel (I) neben geeigneten Formulierungshilfsmitteln enthalten.

Die erfindungsgemäßen Mittel enthalten die Wirkstoffe der Formel (I) im allgemeinen zu 1 bis 95 Gew.-%.

Zur Herstellung der erfindungsgemäßen Mittel gibt man den Wirkstoff und die weiteren Zusätze zusammen und bringt sie in eine geeignete Anwendungsform.

Die Mittel können auf verschiedene Art formuliert werden, je nachdem wie es durch die biologischen und/oder chemisch-physikalischen Parameter vorgegeben ist. Als Formulierungsmöglichkeiten kommen daher beispielsweise in Frage:
Spritzpulver (WP), emulgierbare Konzentrate (EC), wäßrige Lösungen (SL), Emulsionen, versprühbare Lösungen, Dispersionen auf Öl- oder Wasserbasis (SC), Suspoemulsionen (SE), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder. Daneben können die Mittel als Tauchbäder oder Sprühnebel-Anwendungen, in Form von Schäumen, Pasten, Gelen, Salben, Lotionen, Shampoos, Haarfestigern, wirkstoffhaltigen Matten (z.B. flach oder in Kissenform), imprägnierten Artikeln, Aerosolen, druckhaltigen und drucklosen Sprays, Zusätzen zu Farblacken und Nahrungsmitteln, sowie, zur Verwendung als Räucher- und Verdampfungsmittel, als brennbare Feststoffe (z.B. in Konus- oder Spiralen-Form) oder als brennbare Öle (z.B. verteilt über einen erhitzten Docht) und in weiteren, dem Fachmann geläufigen Formulierungen eingesetzt werden.

Diese einzelnen Formulierungstypen sind im Prinzip bekannt und beispielsweise beschrieben in: Winnacker-Küchler, "Chemische Technologie", Band 7, C. Hanser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel, d.h. Träger- und/oder oberflächenaktive Stoffe, wie Inertmaterialien, Tenside, Lösungsmittel und weitere Zusatzstoffe sind ebenfalls bekannt und beispielsweise beschrieben in: Watkins, "Handbook of Insecticide Dust Diluents and Garriers", 2nd Ed., Darland Books, Caldwell N.J.; H. v. Olphen, "Introduction to Clay Colloid Chemistry", 2nd Ed., J. Wiley & Sons, N.Y.; Marsden, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1967; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze, wie Cadodecylbenzol-sulfonat, oder nichtionische Emulgatoren, wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxyethylensorbitester.

Stäubemittel erhält man z.B. durch Vermahlen des Wirkstoffes mit fein verteilten festen Stoffen, z.B. Talkum, natürlichen Tonen, wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise gewünschtenfalls in Mischung mit Düngemitteln - granuliert werden.

Aerosole, beispielsweise vertrieben in Dosen, werden durch Auflösen des Wirkstoffes in Wasser und/oder organischen Lösungsmitteln, wie z.B. Aceton, desodorierendem Petroleum, gesättigten C₈-C₁₃ Kohlenwasserstoffen, pflanzlichen Ölen, unter Zusatz von weiteren geeigneten Stoffen, wie z.B. Emulgatoren, Piperonylbutoxide, Sorbitan Monooleate, Polyoxyethylenglycerol Monooleate, Duftstoffen, und geeigneten Treibgasen, wie z.B. Kohlendioxid, Butan, hergestellt. Anwendungsfertige Sprays, beispielsweise für die Verwendung in Räumen, erhält man z.B. durch Mischung des Wirkstoffs mit geruchlosem Kerosin und Antioxidantien, wobei noch weitere Zusatzstoffe, wie z.B. Emulgatoren, Synergisten (z.B. Piperonylbutoxid) oder Duftstoffe, beigemischt werden können. Köder können beispielsweise durch Vermischung des Wirkstoffs mit Lockstoffen und/oder Nahrungsmitteln, wie z.B. Zucker, sowie Trägermaterialien, wie z.B. Parafinwachs, hergestellt werden.

Eine weitere vorteilhafte Ausführungsform für den Einsatz in Räumen ist die Anwendung als Räucher- und Verdampfungsmittel, die in verschiedenen Verfahren verwendet werden kann. In einem dieser Verfahren werden brennbaren Feststoffen, wie z.B. Sägemehl (z.B. von Kiefem), Stärke und Kokosschalenpulver sowie Blatt- und Stengel-Pulver weiterer Pflanzen (z.B. Pyrethrum, Zedar), unter Zusatz von Farbstoffen und gegebenenfalls Fungiziden, durch geeignete Bindemittel in speziellen Formen, wie z.B. einem Mäander, einer Spirale oder einem Konus, verfestigt und anschließend der Wirkstoff aufgebracht. Durch den langsamen und kontrollierten Abbrand findet dann die Verteilung des Wirkstoffs im Raum statt. Ein anderes Verfahren verwendet Matten oder Kissen aus nicht brennbaren Fasern als Träger, in die der Wirkstoff sowie gegebenenfalls noch weitere Stoffen eingearbeitet wurden. Diese Träger werden auf eine Heizplatte gelegt, die unter kontollierten Bedingungen erhitzt wird und dadurch den Wirkstoff freisetzt. Ein weiteres Verfahren verwendet ein Öl, welchem der Wirkstoff zugesetzt wird und in welches ein Lampendocht, bestehend beispielsweise aus Baumwolle und/oder Zellulose in gepresster Form, eintaucht, der beim Abbrand den Wirkstoff aus dem Öl in den Raum freisetzt. Eine Variante dieses Verfahrens benutzt einen Docht aus nicht brennbaren Fasern, der durch ein elektrisches Gerät erhitzt wird und so die Raumverteilung des im Öl enthaltenen Wirkstoffs bewirkt. Bei den oben genannten Verfahren wird der Wirkstoff entweder direkt oder in bereits formulierter Form aufgebracht. Häufig werden hierbei auch z.B. Farb- und Duftstoffe zugesetzt, sowie geeignete Fungizide zum Schutz der Naturstoffträger vor natürlicher Zersetzung.

In Spritzpulvern beträgt die Wirkstoffkonzentration üblicherweise etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden. Köder enthalten im allgemeinen 0,01 bis 60 Gew.-% an Wirkstoff, vorzugsweise 0,1 bis 5 Gew.-%; Aerosole enthalten im allgemeinen 0,01 bis 50 Gew.-%, vorzugsweise 0,1 bis 5 Gew.-%; anwendungsfertige Sprays enthalten im allgemeinen 0,01 bis 50 Gew.-%., vorzugsweise 0,05 bis 10 Gew.-%. Wirkstoffgehalte bei den Räucher- und Verdampfungsmitteln liegen bei brennbaren Feststoffen im allgemeinen im Bereich von 0,01 bis 60 Gew.-%, bei wirkstoffhaltigen Matten und Kissen im Bereich von 0,01 bis 60 Gew.-% und bei wirkstoffhaltigen Ölen im Bereich von 0,01 bis 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,0005 und 10,0 kg/ha oder mehr Aktivsubstanz, vorzugsweise liegt sie jedoch zwischen 0,001 und 5 kg/ha Wirkstoff.

Die erfindungsgemäßen Wirkstoffe können in ihren handelsüblichen Formulierungen sowie in den aus diesen Formulierungen bereiteten Anwendungsformen in Mischungen mit anderen Wirkstoffen, z.B. Schädlingsbekämpfungsmitteln, wie Insektiziden, Akariziden und Nematiziden, Fungiziden, Lockstoffen, Sterilantien, Molluskiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen und durch Mikroorganismen hergestellte Stoffe.

Bevorzugte Mischungspartner sind:
1. aus der Gruppe der Phosphorverbindungen
   Acephate, Azamethiphos, Azinphos-ethyl, Azinphos-methyl, Bromophos, Bromophos-ethyl, Cadusafos (F-67825), Chlorethoxyphos, Chlorfenvinphos, Chlormephos, Chlorpyrifos, Chlorpyrifos-methyl, Demeton, Demeton-S-methyl, Demeton-S-methyl sulfon, Dialifos, Diazinon, Dichlorvos, Dicrotophos, Dimethoate, Disulfoton, EPN, Ethion, Ethoprophos, Etrimfos, Famphur, Fenamiphos, Fenitriothion, Fensulfothion, Fenthion, Fonofos, Formothion, Fosthiazate (ASC-66824) Heptenophos, Isazophos, Isothloate, Isoxathion, Malathion, Methacrifos, Methamidophos, Methidathion, Salithion, Mevinphos, Monocrotophos, Naled, Omethoate, Oxydemeton-methyl, Parathion, Parathion-methyl, Phenthoate, Phorate, Phosalone, Phosfolan, Phosphocarb (BAS-301), Phosmet, Phosphamidon, Phoxim, Pirimiphos, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tebupirimfos,Tetrachlorvinphos, Thiometon, Triazophos, Trichlorphon, Vamidothion;
2. aus der Gruppe der Carbamate
   Alanycarb (OK-135), Aldicarb, 2-sec.-Butylphenylmethylcarbamate (BPMC), Carbaryl, Carbofuran, Carbosulfan, Cloethocarb, Benfuracarb, Ethiofencarb, Furathiocarb, HCN-801, Isoprocarb, Methomyl, 5-Methyl-m-cumenylbutyryl(methyl)carbamate, Oxamyl, Pirimicarb, Propoxur, Thiodicarb, Thiofanox, 1-Methylthio(ethylideneamino)-N-methyl-N-(morpholinothio)carbamate (UC 51717), Triazamate;
3. aus der Gruppe der Carbonsäureester
   Acrinathrin, Allethrin, Alphametrin, 5-Benzyl-3-furylmethyl-(E)-(1R)-cis-2,2-dimethyl-3-(2-oxothiolan-3-ylidenemethyl)cyclopropanecarboxylate, Beta-Cyfluthrin, Beta-Cypermethrin, Bioallethrin, Bioallethrin((S)-cyclopentylisomer), Bioresmethrin, Bifenthrin, (RS)-1-Cyano-1-(6-phenoxy-2-pyridyl)methyl-(1RS)-trans-3-(4-tert-butylphenyl)-2,2-dimethylcyclopropanecarboxylate (NCI 85193), Cycloprothrin, Cyfluthrin, Cyhalothrin, Cythithrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-Isomer), Imiprothrin (S-41311), Lambda-Cyhalothrin, Permethrin, Phenothrin ((R)-Isomer), Prallethrin, Pyrethrine (natürliche Produkte), Resmethrin, Tefluthrin, Tetramethrin, Theta-Cypermethrin (TD-2344), Tralomethrin, Transfluthrin, Zeta-Cypermethrin (F-56701);
4. aus der Gruppe der Amidine
   Amitraz, Chlordimeform;
5. aus der Gruppe der Zinnverbindungen
   Cyhexatin, Fenbutatinoxide;
6. Sonstige
   Abamectin, ABG-9008, Acetamiprid, Anagrapha falcitera, AKD-1022, AKD-3059, ANS-118, Bacillus thuringiensis, Beauveria bassianea, Bensultap, Bifenazate (D-2341), Binapacryl, BJL-932, Bromopropylate, BTG-504, BTG-505, Buprofezin, Camphechlor, Cartap, Chlorobenzilate, Chlorfenapyr, Chlorfluazuron, 2-(4-Chlorphenyl)-4,5-diphenylthiophen (UBI-T 930), Chlorfentezine, Chromafenozide (ANS-118), CG-216, CG-217, CG-234, A-184699, Cyclopropancarbonsäure-(2-naphthylmethyl)ester (Ro12-0470), Cyromazin, Diacloden (Thiamethoxam), Diafenthiuron, N-(3,5-Dichlor-4-(1,1,2,3,3,3-hexafluor-1-propyloxy) phenyl)carbamoyl)-2-chlorbenzcarboximidsäureethylester, DDT, Dicofol, Diflubenzuron, N-(2,3-Dihydro-3-methyl-1,3-thiazol-2-ylidene)-2,4-xylidine, Dinobuton, Dinocap, Diofenolan, DPX-062, Emamectin-Benzoate (MK-244), Endosulfan, Ethiprole (Sulfethiprole), Ethofenprox, Etoxazole (YI-5301), Fenazaquin, Fenoxycarb, Fipronil, Fluazuron, Flumite (Flufenzine, SZI-121), 2-Fluoro-5-(4-(4-ethoxyphenyl)-4-methyl-1-pentyl)diphenylether (MTI 800), Granulose- und Kernpolyederviren, Fenpyroximate, Fenthiocarb, Flubenzimine, Flucycloxuron, Flufenoxuron, Flufenprox (ICI-A5683), Fluproxyfen, Gamma-HCH, Halofenozide (RH-0345), Halofenprox (MTI-732), Hexaflumuron (DE_473), Hexythiazox, HOl-9004, Hydramethylnon (AC 217300), Lufenuron, Imidacloprid, Indoxacarb (DPX-MP062), Kanemite (AKD-2023), M-020, MTI-446, Ivermectin, M-020, Methoxyfenozide (Intrepid, RH-2485), Milbemectin, NC-196, Neemgard, Nitenpyram (TI-304), 2-Nitromethyl-4,5-dihydro-6H-thiazin (DS 52618), 2-Nitromethyl-3,4-dihydrothiazol (SD 35651), 2-Nitromethylene-1,2-thiazinan-3-ylcarbamaldehyde (WL 108477), Pyriproxyfen (S-71639), NC-196, NC-1111, NNI-9768, Novaluron (MCW-275), OK-9701, OK-9601, OK-9602, Propargite, Pymethrozine, Pyridaben, Pyrimidifen (SU-8801), RH-0345, RH-2485, RYI-210, S-1283, S-1833, SB7242, SI-8601, Silafluofen, Silomadine (CG-177), Spinosad, SU-9118, Tebufenozide, Tebufenpyrad (MK-239), Teflubenzuron, Tetradifon, Tetrasul, Thiacloprid, Thiocyclam, TI-435, Tolfenpyrad (OMI-88), Triazamate (RH-7988), Triflumuron, Verbutin, Vertalec (Mykotal), YI-5301.

Die oben genannten Kombinationspartner stellen bekannte Wirkstoffe dar, die zum großen Teil in C D S Tomlin (Editor), The Pesticide Manual, 12. Auflage, The British Crop Protection Council, Farnham, UK, 2000, beschrieben sind.

Der Wirkstoffgehalt der aus den handelsüblichen Formulierungen bereiteten Anwendungsformen kann von 0,00000001 bis zu 95 Gew.-% Wirkstoff, vorzugsweise zwischen 0,00001 und 1 Gew.-% liegen. Die Anwendung geschieht in einer den Anwendungsformen angepaßten üblichen Weise.

Die Verbindungen der Formel (I) können in ihren handelsüblichen Formulierungen auch in Kombination mit Fungiziden angewendet werden. Bei diesen Fungiziden handelt es sich im allgemeinen um Wirkstoffe, die in C D S Tomlin (Editor), The Pesticide Manual, 12. Auflage, The British Crop Protection Council, Farnham, UK, 2000, beschrieben sind. Die Anwendung geschieht in einer den Anwendungsformen angepaßten üblichen Weise, beispielsweise dadurch, daß man zur Bekämpfung pathogener Pilze auf diese, oder die von ihnen befallenen Pflanzen, Flächen oder Substrate oder auf Saatgut eine fungizid wirksame Menge eines erfindungsgemäßen Verbindung oder eines erfindungsgemäßen Mittels appliziert.

Die Verbindungen der Formel (I) können auch zur Bekämpfung von Schadorganismen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pflanzenschutzmitteln, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung in wirtschaftlich bedeutenden transgenen Kulturen von Nutz- und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Bei der Anwendung in transgenen Kulturen, insbesondere mit Insektenresistenzen treten neben den in anderen Kulturen zu beobachtenden Wirkungen gegenüber Schadorganismen oftmals Wirkungen auf, die für die Applikation in der jeweiligen transgenen Kultur spezifisch sind, beispielsweise ein verändertes oder speziell erweitertes Spektrum an Schädlingen, die bekämpft werden können, oder veränderte Aufwandmengen, die für die Applikation eingesetzt werden können.

Die erfindungsgemäßen Verbindungen der Formel (I) bzw. diese enthaltenden Mittel finden Verwendung beispielsweise in der Landwirtschaft, im Gartenbau, in Forsten, und im Vorratsschutz. Bevorzugt ist die Anwendung in wirtschaftlich bedeutenden Kulturen von Nutz- und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Die Anwendung der erfindungsgemäßen Verbindungen beinhaltet neben direkter Applikation auf die Schadorganismen jede andere Applikation, bei der Verbindungen der Formel (I) auf die Schadorganismen wirken. Solche indirekten Applikationen können beispielsweise in der Anwendung von Verbindungen liegen, die, beispielsweise im Boden, der Pflanze oder dem Schadorganismus, zu Verbindungen der Formel (I) zerfallen oder abgebaut werden.

Die erfindungsgemäße Verwendung von Verbindungen der Formel (I) bzw. diese enthaltende Mittel, beispielsweise als Insektizid, Akarizid oder Heiminthizid/Nematizid, schließt auch den Fall ein, bei dem die Verbindung der Formel (I) oder deren Salz erst nach dem Ausbringen, beispielsweise im Schadorganismus, einer Pflanze oder im Boden, aus einer Vorläufersubstanz gebildet wird.

Neben den bisher genannten und üblichen Applikationsverfahren zeigen die erfindungsgemäßen Wirkstoffe der Formel (I) eine hervorragende systemische Wirkung. Die Wirkstoffe können daher auch über Pflanzenteile, unterirdische wie oberirdische (z.B. Wurzel, Stolonen, Stengel, Stamm, Blatt), in die Pflanzen eingebracht werden, wenn die Wirkstoffe in flüssiger oder fester Form auf, in und/oder in die direkte Umgebung der Pflanze appliziert werden (z.B. Granulate in der Erdapplikation, Applikation in gefluteten Reisfeldern, Stamminjektion bei Bäumen, Stengelbandagen bei perenierenden Pflanzen).

Daneben sind die erfindungsgemäßen Wirkstoffe in besonderer Weise zur Behandlung von vegetativem und generativem pflanzlichem Vermehrungsmaterial einsetzbar, wie z.B. von Saatgut von beispielsweise Getreide, Gemüse, Baumwolle, Reis, Zuckerrübe und anderen Kultur- und Zierpflanzen, von Zwiebeln, Stecklingen und Knollen weiterer vegetativ vermehrter Kultur- und Zierpflanzen. Die Behandlung hierfür kann vor der Saat bzw. dem Pflanzvorgang erfolgen (z.B. durch spezielle Techniken des 'Seed Coatings', durch Beizung in flüssiger oder fester Form oder als 'Seedbox Treatment'), während des Saatvorgangs bzw. des Pflanzens oder nach dem Saat- bzw. Pflanzvorgang durch spezielle Applikationstechniken (z.B. Saatreihenbehandlung). Die angewandte Wirkstoffmenge kann entsprechend der Anwendung in einem größerem Bereich schwanken. Im allgemeinen liegen die Aufwandmengen zwischen 1 g und 10 kg Wirkstoff pro Hektar Bodenfläche. Die Behandlungsverfahren für pflanzliches Vermehrungsmaterial und das so behandelte pflanzliche Vermehrungsmaterial sind weitere Gegenstände der Erfindung.

Die erfindungsgemäßen Wirkstoffe eignen sich auch auf dem veterinärmedizinischen Gebiet, vorzugsweise zur Bekämpfung von Endo- und Ektoparasiten, und auf dem Gebiet der Tierhaltung. Die Anwendung der erfindungsgemäßen Wirkstoffe kann in bekannter Weise geschehen, wie durch orale Anwendung in Form von beispielsweise Tabletten, Kapseln, Tränken oder Granulaten, durch dermale Anwendung in Form beispielsweise des Tauchens (Dippen), Sprühens (Sprayen), Aufgießen (pour-on and spot-on) und des Einpuderns, sowie durch parenterale Anwendung in Form beispielsweise der Injektion.

Die erfindungsgemäßen Verbindungen der Formel (I) können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die Verbindungen, gegebenenfalls in geeigneten Formulierungen und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die Verbindungen können bei Rindern z.B. in Dosierungen von 0,01 bis 1 mg/kg Körpergewicht eingesetzt werden.

Die erfindungsgemäßen Verbindungen eignen sich daneben auch für den Einsatz in technischen Bereichen, beispielsweise als Holzschutzmittel, als Konservierungsmittel in Dichtmassen, in Anstrichfarben, in Kühlschmiermittel für die Metallbearbeitung oder als Konservierungsmittel in Bohr- und Schneidölen.

Weitere bevorzugte Anwendungsbereiche sind der Vorrats- und Materialschutz, der Hygienesektor und der häusliche Bereich, wobei als bevorzugte Ausführungsform der Erfindung die erfindungsgemäße Zusammensetzung in den entsprechenden Räumlichkeiten eingesetzt und gegebenenfalls mit weiteren Maßnahmen, wie z.B. Klebtafeln oder Fallen, kombiniert wird. Geeignete Dosierungen und Formulierungen sind auch hier insbesondere von der Art und der Höhe des Befallsdruck abhängig und lassen sich nach den üblichen Methoden leicht ermitteln und festlegen.

Die Erfindung wird durch die folgenden Beispiele illustriert. Die Herstellungsbeispiele können auch bereits bekannte Verbindungen enthalten, wobei diese dazu dienen den Herstellungsprozess der erfindungsgemässen Verbindungen zu beschreiben. Bereits bekannte Verbindungen, aufgelistet neben erfindungsgemässen Verbindungen in den folgenden Tabellen, finden auch in dem erfindungsgemässen Verfahren Verwendung.

### A. Chemische Beispiele

### Beispiel 1

### 1-(3-o-Tolyl-2-propinyl)piperidin

Eine Mischung aus 2-lodtoluol (6,54 g), N-(2-Propinyl)piperidin (3,69 g), Dichlorbis(triphenylphosphino)palladium(II) (0,05 g) und Kupfer(I)iodid (0,1 g) in trockenem Diethylamin (50 ml) wurde 5 Stunden unter Rückfluß erhitzt. Das Lösungsmittel wurde im Vakuum entfernt, der Rückstand in Diethylether aufgenommen und mit Wasser gewaschen. Die organische Phase wurde über Magnesiumsulfat getrocknet und unter Vakuum vollständig eingeengt. Destillation des Rückstands im Vakuum ergaben 3,98 g eines Öls mit Siedepunkt 96-98°C/0,08 mm Hg.

### Herstellung des Edukts 1-(2-Propinyl)piperidin:

Zu einer Lösung von Piperidin (92,14 g) in trockenem Methanol (100 ml) tropfte man unter Rühren eine Lösung von 2-Propinylchlorid (40,31 g) in trockenem Methanol (50 ml). Die Mischung wurde 3 Stunden bei 25°C gerührt und anschließend filtriert.

Das Filtrat wurde im Vakuum eingeengt und der Rückstand destilliert (Siedepunkt: 163-165°C/Atmosphärendruck).

### Beispiel 2

### 1-(4-Methoxyphenyl-2-propinyl)piperidin Hydrochlorid

Ersatz von 2-lodtoluol gegen 4-lodanisol lieferte nach dem Verfahren von Beipiel 1 ein Rohprodukt, das mit überschüssiger Salzsäure in Methanol versetzt wurde. Entfernen des Lösungsmittels gab nach Kristallisation aus Isopropanol farblose Nadeln mit Schmelzpunkt 223-225°C.

### Beispiel 3

### 1-[3-(4-Methoxyphenyl)-2-propinyl]piperidin

Das Produkt aus Beispiel 2 wurde in Wasser gelöst, die Lösung mit 2N Natronlauge alkalisch gestellt und mit Diethylether extrahiert. Nach Trocknen der Etherextrakte mit Magnesiumsulfat wurde das Lösungsmittel entfernt und der Rückstand im Vakuum destilliert. Man erhielt ein Öl mit Siedepunkt 126-128°C/0,1 mm Hg.

### Beispiel 4

### 1-[3-(3-Fluorphenyl)-2-propinyl]-4-methylpiperidin

Eine Mischung aus 3-Fluoriodbenzol (8,88 g), 4-Methyl-1-(2-propinyl)piperidin (6,80 g), Palladiumacetat (0,045 g), Tri-*o*-toloylphosphin (0,24 g) und Kupfer(I)iodid (0,1 g) in trockenem Diethylamin (50 ml) wurde 5 Stunden unter Rückfluß erhitzt. Das Lösungsmittel wurde im Vakuum entfernt, der Rückstand in Diethylether aufgenommen und mit Wasser gewaschen. Die organische Phase wurde über Magnesiumsulfat getrocknet und das Lösungsmittel im Vakuum entfernt. Destillation des Rückstands im Vakuum ergab ein Öl (5,20 g) mit Siedepunkt 105°C/0,07 mm Hg. Herstellung des Edukts 4-Methyl-1-(2-propinyl)piperidin:
Zu einer Suspension von 4-Methylpiperidin (10,0 g) und Kaliumcarbonat (13,96 g) in trockenem Aceton (80 ml) tropfte man bei 0-5°C unter Rühren Propargylbromid (12,01 g). Die Mischung wurde 6 Stunden bei 50°C gerührt und anschließend filtriert. Das Filtrat wurde im Vakuum eingeengt und der Rückstand in Dichlormethan aufgenommen. Die organische Phase wurde mit Wasser gewaschen (dreimal je 15 ml), mit Natriumsulfat getrocknet und im Vakuum eingeengt. Destillation des Rückstands im Vakuum ergaben 12,73 g eines Öls mit Siedepunkt 60°C/16 mm Hg.

### Beispiel 5

### 1-(3-Phenyl-2-propinyl)piperidin

Zu einer Suspension von Paraformaldehyd (3,6 g) in Dioxan (10 ml) gab man unter Rühren nacheinander eine Lösung von Phenylacetylen (10,2 g) in Dioxan (10 ml), eine Lösung von Piperidin (13,3 g) in Dioxan (10 ml) und Kupfer(I)chlorid (0,1 g). Man erhitzte das Gemisch 6 Stunden unter Rückfluß, ließ auf 25°C abkühlen und säuerte mit 20%iger Salzsäure an. Die Lösung wurde mit Diethylether gewaschen, die wäßrige Phase mit 50%iger Natronlauge alkalisch gestellt und mit Diethylether (dreimal je 100 ml) extrahiert. Die organische Phase wurde mit Magnesiumsulfat getrocknet und vollständig eingeengt. Destillation des Rückstands im Vakuum ergab ein Öl (13,0 g) mit Siedepunkt 95°C/0,045 mm Hg.

### Beispiel 6

### 1-[3-(3,5-Bistrifluormethylphenyl)-2-propinyl]piperidin

Eine Mischung von 1-(3,5-Bistrifluormethylphenyl)-3-bromprop-1-in (2,53 g), Piperidin (5 ml) und wasserfreiem Kaliumcarbonat (5 g) in trockenem Aceton (50 ml) wurde für 18 h unter Rühren auf Rückfluß erhitzt. Entfernen des Lösungsmittels im Vakuum und Destillation des Rückstands im Vakuum ergaben 2,26 g eines Öls mit Siedepunkt 95°C/0,025 mm Hg.

### Beispiel 7

### 1-[3-(3,5-Bistrifluormethylphenyl)-2-propinyl]piperidin Hydrochlorid

Durch eine Lösung von 1-(3,5-Bistrifluormethylphenyl)-3-piperidinoprop-1-in (2,26 g) in trockenem Diethylether (200 ml) leitete man einen Überschuß von trockenem HCl-Gas. Nach Filtration, Waschen (Diethylether) und Trocknen des entstandenen weißen Niederschlags erhielt man 2,16 g feines, weißes Pulver, mit Schmelzpunkt 214,5°C.

Die Verbindungen der nachfolgenden Tabellen wurden analog zu den Beispielen 1 bis 7 erhalten.

In den Tabellen werden folgende Abkürzungen benutzt
Me = Methyl
Et = Ethyl
Pr = n-Propyl
Prⁱ = iso-Propyl
Bu = n-Butyl
Bu^{t} = tertiär-Butyl
Ph = Phenyl
Sdp = Siedepunkt
Schmp = Schmelzpunkt

| | |
|---|---|
| L¹ | 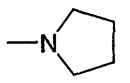 |
| L² | 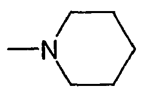 |
| L³ | 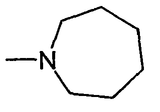 |
| L⁴ | 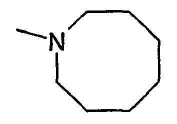 |
| L⁵ | 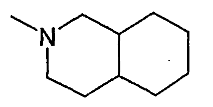 |
| L⁶ | 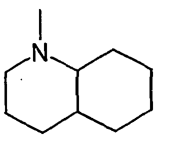 |
| L⁷ | 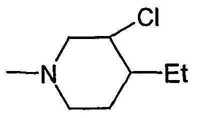 |
| L⁸ | 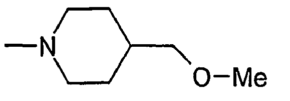 |
| L⁹ | 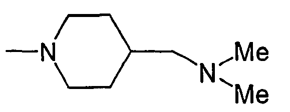 |
| L¹⁰ | 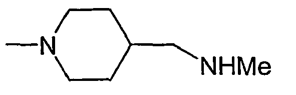 |
| L¹¹ | 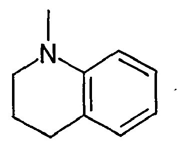 |
| L¹² | 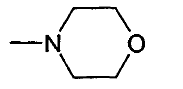 |
| L¹³ | 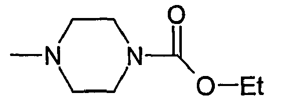 |
| L¹⁴ | 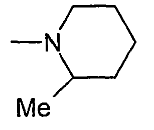 |
| L¹⁵ | 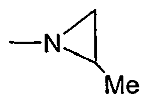 |
| L¹⁶ | 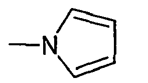 |
| L¹⁷ | 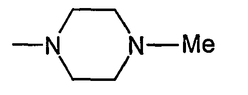 |
| L¹⁸ | 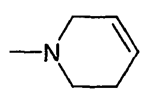 |
| L¹⁹ | 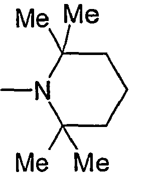 |
| L²⁰ | 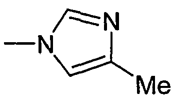 |
| L²¹ | 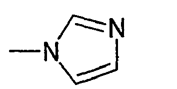 |
| L²² | 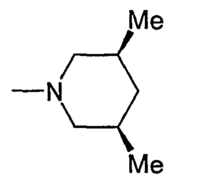 |
| L²³ | 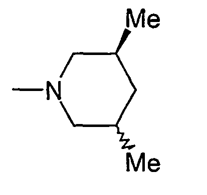 |
| L²⁴ | 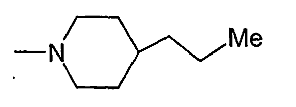 |
| L²⁵ | 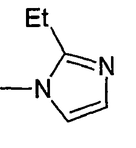 |
| L²⁶ | 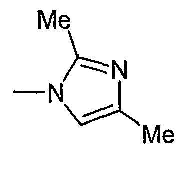 |
| L²⁷ | 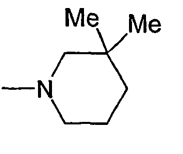 |
| L²⁸ | 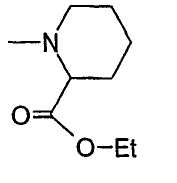 |
| L²⁹ | 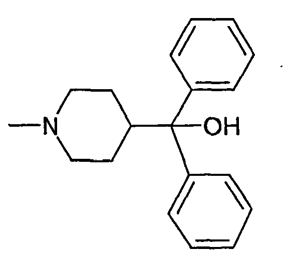 |
| L³⁰ | 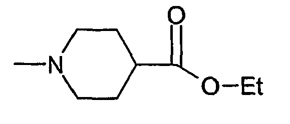 |
| L³¹ | 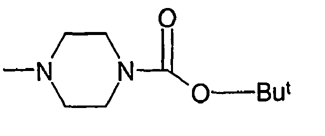 |
| L³² | 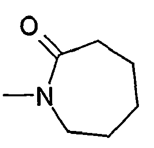 |
| L³³ | 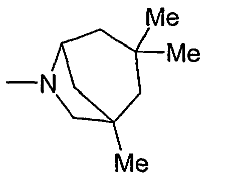 |
| L³⁴ | 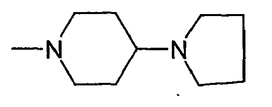 |
| L³⁵ | 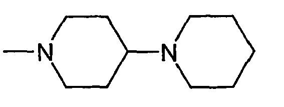 |
| L³⁶ | 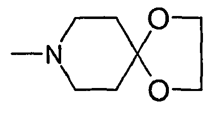 |
| L³⁷ | 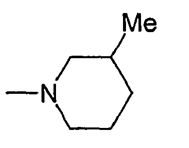 |
| L³⁸ | 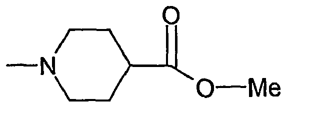 |
| L³⁹ | 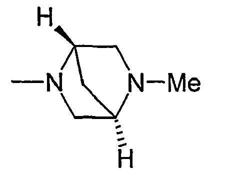 |
| L⁴⁰ | 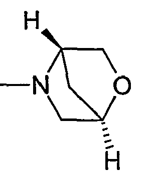 |
| L⁴¹ | 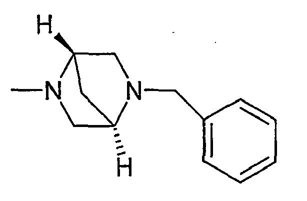 |
| L⁴² | 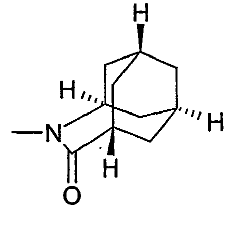 |
| L⁴³ | 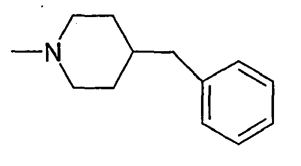 |
| L⁴⁴ | 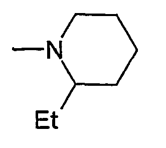 |
| L⁴⁵ | 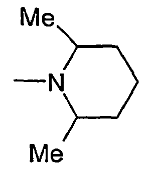 |
| L⁴⁶ | 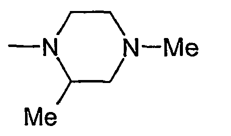 |
| L⁴⁷ | 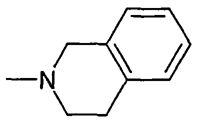 |
| L⁴⁸ | 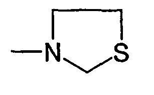 |
| L⁴⁹ | 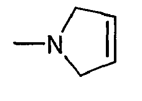 |
| L⁵⁰ | 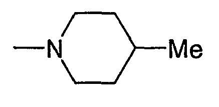 |
| L⁵¹ | 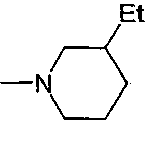 |
| L⁵² | 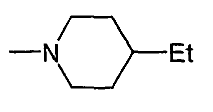 |
| L⁵³ | 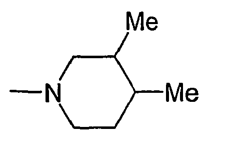 |
| L⁵⁴ | 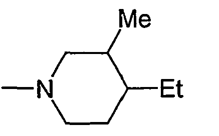 |
| L⁵⁵ | 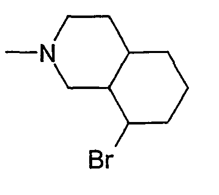 |
| L⁵⁶ | 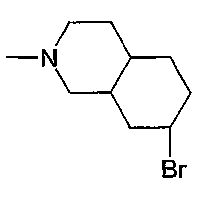 |
| L⁵⁷ | 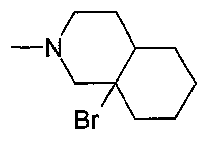 |
| L⁵⁸ | 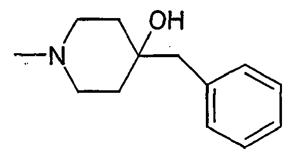 |
| L⁵⁹ | 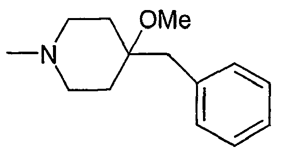 |
| L⁶⁰ | 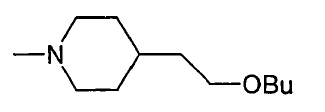 |
| L⁶¹ | 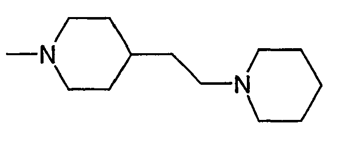 |
| L⁶² | 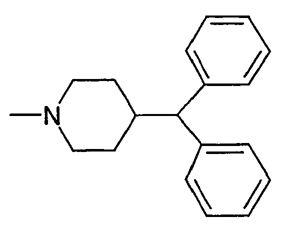 |
| L⁶³ | 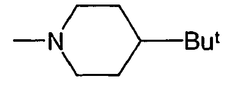 |
| L⁶⁴ | 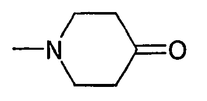 |
| L⁶⁵ | 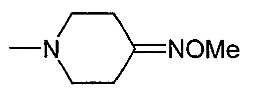 |
| L⁶⁶ | 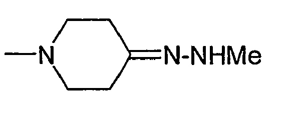 |
| L⁶⁷ | 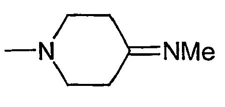 |
| L⁶⁸ | 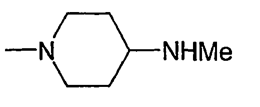 |
| L⁶⁹ | 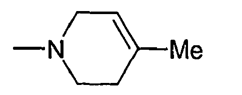 |
| L⁷⁰ | 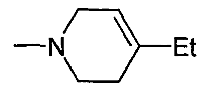 |
| L⁷¹ | 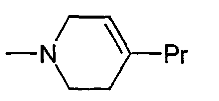 |
| L⁷² | 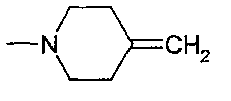 |
| L⁷³ | 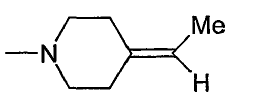 |
| L⁷⁴ | 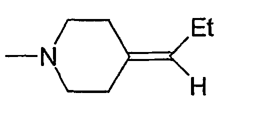 |
| L⁷⁵ | 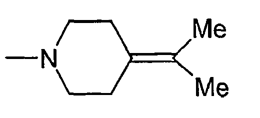 |
| L⁷⁶ | 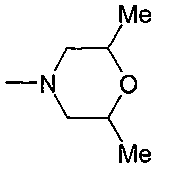 |
| L⁷⁷ | 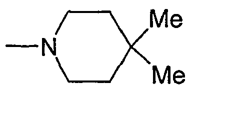 |
| L⁷⁸ | 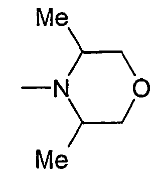 |
| L⁷⁹ | 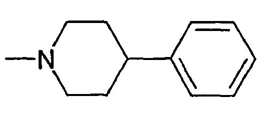 |
| L⁸⁰ | 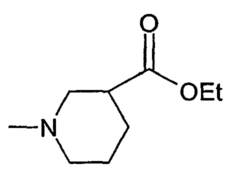 |
| L⁸¹ | 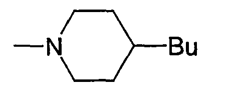 |
| L⁸² | 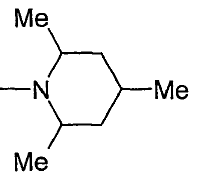 |
| L⁸³ | 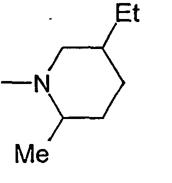 |
| L⁸⁴ | 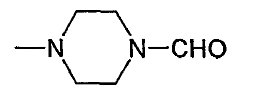 |
| L⁸⁵ | 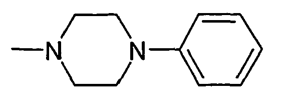 |
| L⁸⁶ | 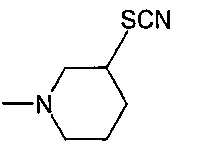 |
| L⁸⁷ | 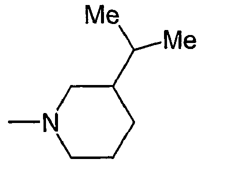 |
| L⁸⁸ | 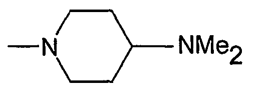 |
| L⁸⁹ | 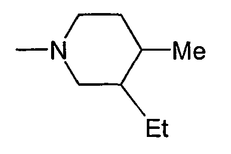 |
| L⁹⁰ | 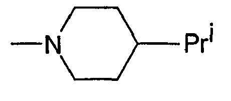 |
| L⁹¹ | 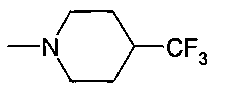 |
| L⁹² | 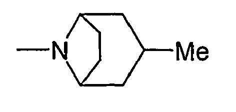 |
| L⁹³ | 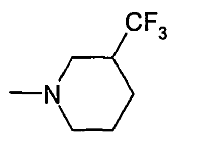 |

| | | | | | |
|---|---|---|---|---|---|
| 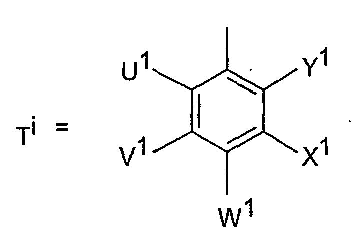 | | | | | |

| Tⁱ | U¹ | V¹ | W¹ | X¹ | Y¹ |
|---|---|---|---|---|---|
| T¹ | H | H | H | H | H |
| T² | Cl | H | H | H | H |
| T³ | H | Cl | H | H | H |
| T⁴ | H | H | Cl | H | H |
| T⁵ | Cl | Cl | H | H | H |
| T⁶ | Cl | H | Cl | H | H |
| T⁷ | Cl | H | H | Cl | H |
| T⁸ | Cl | H | H | H | Cl |
| T⁹ | H | Cl | Cl | H | H |
| T¹⁰ | H | Cl | H | Cl | H |
| T¹¹ | H | Cl | H | H | Cl |
| T¹² | Cl | Cl | Cl | H | H |
| T¹³ | Cl | Cl | H | Cl | H |
| T¹⁴ | Cl | Cl | H | H | Cl |
| T¹⁵ | Cl | H | Cl | Cl | H |
| T¹⁶ | Cl | H | Cl | H | Cl |
| T¹⁷ | Cl | Cl | Cl | Cl | H |
| T¹⁸ | Cl | Cl | Cl | H | Cl |
| T¹⁹ | Cl | Cl | H | Cl | Cl |
| T²⁰ | Cl | Cl | Cl | Cl | Cl |
| T²¹ | F | H | H | H | H |
| T²² | H | F | H | H | H |
| T²³ | H | H | F | H | H |
| T²⁴ | F | F | H | H | H |
| T²⁵ | F | H | F | H | H |
| T²⁶ | F | H | H | F | H |
| T²⁷ | F | H | H | H | F |
| T²⁸ | H | F | F | H | H |
| T²⁹ | H | F | H | F | H |
| T³⁰ | H | F | H | H | F |
| T³¹ | CF₃ | H | H | H | H |
| T³² | H | CF₃ | H | H | H |
| T³³ | H | H | CF₃ | H | H |
| T³⁴ | CF₃ | CF₃ | H | H | H |
| T³⁵ | CF₃ | H | CF₃ | H | H |
| T³⁶ | CF₃ | H | H | CF₃ | H |
| T³⁷ | CF₃ | H | H | H | CF₃ |
| T³⁸ | H | CF₃ | CF₃ | H | H |
| T³⁹ | H | CF₃ | H | CF₃ | H |
| T⁴⁰ | H | CF₃ | H | H | CF₃ |
| T⁴¹ | H | CF₃ | CF₃ | CF₃ | H |
| T⁴² | Cl | CF₃ | H | H | H |
| T⁴³ | Cl | H | CF₃ | H | H |
| T⁴⁴ | Cl | H | H | CF₃ | H |
| T⁴⁵ | Cl | H | H | H | CF₃ |
| T⁴⁶ | H | Cl | CF₃ | H | H |
| T⁴⁷ | H | Cl | H | CF₃ | H |
| T⁴⁸ | H | Cl | H | H | CF₃ |
| T⁴⁹ | H | H | Cl | CF₃ | H |
| T⁵⁰ | H | H | Cl | H | CF₃ |
| T⁵¹ | H | H | H | Cl | CF₃ |
| T⁵² | Me | Cl | H | H | H |
| T⁵³ | H | Cl | Me | H | H |
| T⁵⁴ | H | Cl | H | Me | H |
| T⁵⁵ | H | Me | Cl | H | H |
| T⁵⁶ | Me | H | Cl | H | H |
| T⁵⁷ | H | F | Cl | H | H |
| T⁵⁸ | H | F | H | Cl | H |
| T⁵⁹ | H | Cl | F | H | H |
| T⁶⁰ | H | NO₂ | H | H | H |
| T⁶¹ | H | H | NO₂ | H | H |
| T⁶² | H | CN | H | H | H |
| T⁶³ | H | H | CN | H | H |
| T⁶⁴ | H | NO₂ | Cl | H | H |
| T⁶⁵ | H | NO₂ | H | Cl | H |
| T⁶⁶ | H | Cl | NO₂ | H | H |
| T⁶⁷ | H | CN | Cl | H | H |
| T⁶⁸ | H | CN | H | Cl | H |
| T⁶⁹ | H | Cl | CN | H | H |
| T⁷⁰ | H | NO₂ | CF₃ | H | H |
| T⁷¹ | H | NO₂ | H | CF₃ | H |
| T⁷² | H | CF₃ | NO₂ | H | H |
| T⁷³ | H | CN | CF₃ | H | H |
| T⁷⁴ | H | CN | H | CF₃ | H |
| T⁷⁵ | H | CF₃ | CN | H | H |
| T⁷⁶ | H | Me | H | H | H |
| T⁷⁷ | H | H | Me | H | H |
| T⁷⁸ | COOMe | H | H | H | H |
| T⁷⁹ | H | COOMe | H | H | H |
| T⁸⁰ | H | H | COOMe | H | H |
| T⁸¹ | H | Cl | Cl | OMe | H |
| T⁸² | H | Cl | OMe | Cl | H |
| T⁸³ | H | CF₃ | OMe | CF₃ | H |
| T⁸⁴ | H | CF₃ | OMe | Cl | H |
| T⁸⁵ | OMe | H | H | H | H |
| T⁸⁶ | H | OMe | H | H | H |
| T⁸⁷ | H | H | OMe | H | H |
| T⁸⁸ | Me | H | H | H | H |
| T⁸⁹ | H | SMe | H | H | H |
| T⁹⁰ | H | H | SMe | H | H |
| T⁹¹ | H | Ph | H | H | H |
| T⁹² | H | Ph | Cl | H | H |
| T⁹³ | H | Ph | CF₃ | H | H |
| T⁹⁴ | H | H | Ph | H | H |
| T⁹⁵ | H | Cl | Ph | H | H |
| T⁹⁶ | H | CF₃ | Ph | H | H |
| T⁹⁷ | H | Cl | Ph | Cl | H |
| T⁹⁸ | H | CF₃ | Ph | CF₃ | H |
| T⁹⁹ | H | H | HC=CH-CF₃ | H | H |
| T¹⁰⁰ | H | Cl | HC=CH-CF₃ | H | H |
| T¹⁰¹ | H | HC=CH-CF₃ | H | H | H |
| T¹⁰² | H | HC=CH-CF₃ | Cl | H | H |
| T¹⁰³ | H | H | OEt | H | H |
| T¹⁰⁴ | H | H | Et | H | H |
| T¹⁰⁵ | H | Me | F | H | H |
| T¹⁰⁶ | Me | H | F | H | H |
| T¹⁰⁷ | Cl | H | H | Me | H |
| T¹⁰⁸ | Me | H | H | F | H |
| T¹⁰⁹ | H | OC(O)Pr | H | H | H |
| T¹¹⁰ | Me | H | H | Cl | H |
| T¹¹¹ | Me | H | Me | H | H |
| T¹¹² | Cl | H | H | H | Me |
| T¹¹³ | Me | Me | H | H | H |
| T¹¹⁴ | Me | H | H | H | Me |
| T¹¹⁵ | Et | H | H | H | H |
| T¹¹⁶ | H | Me | Me | H | H |
| T¹¹⁷ | H | SO₂Me | H | H | H |
| T¹¹⁸ | Prⁱ | H | H | H | H |
| T¹¹⁹ | F | H | F | H | F |
| T¹²⁰ | EtO | H | H | H | H |
| T¹²¹ | F | H | H | F | H |
| T¹²² | F | H | F | H | H |
| T¹²³ | H | Cl | Cl | Me | H |
| T¹²⁴ | H | H | Prⁱ | H | H |
| T¹²⁵ | F | F | F | F | F |
| T¹²⁶ | Me | H | H | Me | H |
| T¹²⁷ | H | H | OPh | H | H |
| T¹²⁸ | H | H | C(O)Me | H | H |
| T¹²⁹ | H | OCH₂O | | H | H |
| T¹³⁰ | H | Me | H | Me | H |
| T¹³¹ | H | CF₃ | H | OMe | H |
| T¹³² | H | F | Me | H | H |
| T¹³³ | H | H | 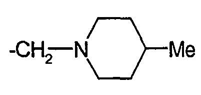 | H | H |
| T¹³⁴ | H | Cl | F | Cl | H |
| T¹³⁵ | H | CF₃ | COOMe | H | H |
| T¹³⁶ | _{H} | OMe | OMe | H | H |
| T¹³⁷ | H | Cl | Cl | Cl | H |
| T¹³⁸ | H | NO₂ | H | NO₂ | H |
| T¹³⁹ | H | CF₃ | H | NO₂ | H |
| T140 | Cl | H | CF₃ | H | Cl |
| T¹⁴¹ | H | C(O)Me | H | H | H |
| T¹⁴² | H | H | (2-Cl, 5-CF₃)Ph | H | H |
| T¹⁴³ | H | H | (4-CF₃)Ph | H | H |
| T¹⁴⁴ | H | H | (3-Cl)Ph | H | H |
| T¹⁴⁵ | H | H | (2-Cl)Ph | H | H |
| T¹⁴⁶ | H | H | (3-Me, 5-Me)Ph | H | H |
| T¹⁴⁷ | H | H | (4-OMe)Ph | H | H |
| T¹⁴⁸ | H | H | (2-Cl, 4-Cl)Ph | H | H |
| T¹⁴⁹ | H | H | (3-F, 5-NO₂)Ph | H | H |
| T¹⁵⁰ | H | H | (3-CO₂Et)Ph | H | H |
| T¹⁵¹ | H | H | (2-Me, 5-NO₂)Ph | H | H |
| T¹⁵² | H | H | (2-Cl, 3-Cl)Ph | H | H |
| T¹⁵³ | H | H | (3-CF₃)Ph | H | H |
| T¹⁵⁴ | H | H | (2-Me)Ph | H | H |
| T¹⁵⁵ | H | H | 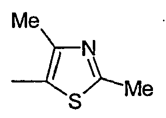 | H | H |
| T¹⁵⁶ | H | H | 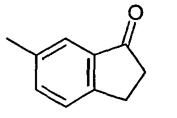 | H | H |
| T¹⁵⁷ | H | H | 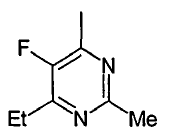 | H | H |
| T¹⁵⁸ | H | H | 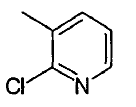 | H | H |
| T¹⁵⁹ | H | H | 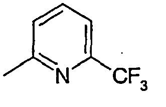 | H | H |
| T¹⁶⁰ | H | H | 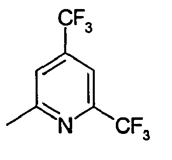 | H | H |
| T¹⁶¹ | H | H | (4-CN)Ph | H | H |
| T¹⁶² | H | H | 2-Thienyl | H | H |
| T¹⁶³ | H | H | (2-F, 6-F)Ph | H | H |
| T¹⁶⁴ | H | H | 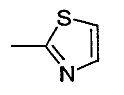 | H | H |
| T¹⁶⁵ | H | H | 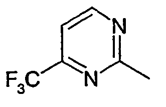 | H | H |
| T¹⁶⁶ | H | H | (4-OCH₂CF₃)Ph | H | H |
| T¹⁶⁷ | H | H | (4-Me)Ph | H | H |
| T¹⁶⁸ | H | F | Br | H | H |
| T¹⁶⁹ | H | CF₃ | Br | H | H |
| T¹⁷⁰ | H | (4-CF₃)Ph | H | H | H |
| T¹⁷¹ | H | (3-CF₃, 5-CF₃)Ph | H | H | H |
| T¹⁷² | H | (3-Cl)Ph | H | H | H |
| T¹⁷³ | H | (4-NO₂)Ph | H | H | H |
| T¹⁷⁴ | H | 1-Naphthyl | H | H | H |
| T¹⁷⁵ | H | (4-Cl)Ph | H | H | H |
| T¹⁷⁶ | H | 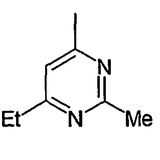 | H | H | H |
| T¹⁷⁷ | H | 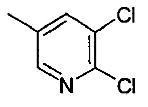 | H | H | H |
| T¹⁷⁸ | H | 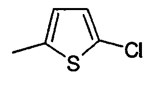 | H | H | H |
| T¹⁷⁹ | H | (4-CN)Ph | H | H | H |
| T¹⁸⁰ | H | 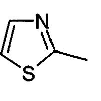 | H | H | H |
| T¹⁸¹ | H | F | (4-F)Ph | H | H |
| T¹⁸² | H | F | Et | H | H |
| T¹⁸³ | H | CF₃ | Et | H | H |
| T¹⁸⁴ | H | CF₃ | H | Me | H |
| T¹⁸⁵ | H | Cl | OCF₂CHFCl | Cl | H |
| T¹⁸⁶ | H | CF₃ | Me | H | H |
| T¹⁸⁷ | H | CF₃ | F | H | H |
| T¹⁸⁸ | F | Cl | F | H | H |
| T¹⁸⁹ | H | F | (4-OMe)Ph | H | H |
| T¹⁹⁰ | H | F | (4-Me)Ph | H | H |
| T¹⁹¹ | H | F | (3-Cl, 4-F)Ph | H | H |
| T¹⁹² | H | F | (4-CF₃)Ph | H | H |
| T¹⁹³ | H | F | (3,4-OCH₂CH₂O)Ph | H | H |
| T¹⁹⁴ | H | F | [4-C(O)Me]Ph | H | H |
| T¹⁹⁵ | H | F | (4-OCF₃)Ph | H | H |
| T¹⁹⁶ | H | F | (4-tBu)Ph | H | H |
| T¹⁹⁷ | H | F | (4-Cl)Ph | H | H |
| T¹⁹⁸ | H | F | (3-Me)Ph | H | H |
| T¹⁹⁹ | H | F | (3-Cl)Ph | H | H |
| T⁶⁰⁰ | H | CF₃ | (4-SMe)Ph | H | H |
| T⁶⁰¹ | H | CF₃ | 3-Thienyl | H | H |
| T⁶⁰² | H | CF₃ | (4- CF₃)Ph | H | H |
| T⁶⁰³ | H | CF₃ | (4-OCF₃)Ph | H | H |
| T⁶⁰⁴ | H | CF₃ | [4-C(O)Me]Ph | H | H |
| T⁶⁰⁵ | H | CF₃ | (4-Cl)Ph | H | H |
| T⁶⁰⁶ | H | CF₃ | 1-Naphthyl | H | H |
| T⁶⁰⁷ | H | CF₃ | (2-F)Ph | H | H |
| T⁶⁰⁸ | H | CF₃ | (2-Cl)Ph | H | H |
| T⁶⁰⁹ | H | F | 3-Thienyl | H | H |

| | | | | |
|---|---|---|---|---|
| 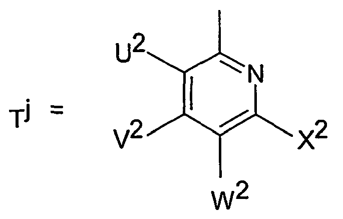 | | | | |

| Tⱼ | U² | V² | W² | X² |
|---|---|---|---|---|
| T²⁰⁰ | Cl | H | CF₃ | H |
| T²⁰¹ | H | Cl | CF₃ | H |
| T²⁰² | H | H | CF₃ | Cl |
| T²⁰³ | H | CF₃ | H | Cl |
| T²⁰⁴ | CF₃ | H | H | Cl |
| T²⁰⁵ | Cl | CF₃ | H | H |
| T²⁰⁶ | Cl | H | H | CF₃ |
| T²⁰⁷ | H | Cl | H | CF₃ |
| T²⁰⁸ | H | H | Cl | CF₃ |
| T²⁰⁹ | CF₃ | Cl | H | H |
| T²¹⁰ | H | CF₃ | Cl | H |
| T²¹¹ | CF₃ | H | Cl | H |
| T²¹² | Br | H | H | H |
| T²¹³ | H | Br | H | H |
| T²¹⁴ | H | H | Br | H |
| T²¹⁵ | H | H | H | Br |
| T²¹⁶ | CF₃ | H | H | H |
| T²¹⁷ | H | CF₃ | H | H |
| T²¹⁸ | H | H | CF₃ | H |
| T²¹⁹ | H | H | H | CF₃ |
| T²²⁰ | H | H | H | H |
| T²²¹ | CN | H | H | H |
| T²²² | H | CN | H | H |
| T²²³ | H | H | CN | H |
| T²²⁴ | H | H | H | CN |
| T²²⁵ | NO₂ | H | H | H |
| T²²⁶ | H | NO₂ | H | H |
| T²²⁷ | H | H | NO₂ | H |
| T²²⁸ | H | H | H | NO₂ |
| T²²⁹ | CF₃ | CF₃ | H | H |
| T²³⁰ | CF₃ | H | CF₃ | H |
| T²³¹ | CF₃ | H | H | CF₃ |
| T²³² | H | CF₃ | CF₃ | H |
| T²³³ | H | CF₃ | H | CF₃ |
| T²³⁴ | H | H | CF₃ | CF₃ |
| T²³⁵ | Cl | H | OCF₂CF₂H | H |
| T²³⁶ | H | CF₃ | H | Me |
| T²³⁷ | H | Me | H | Me |
| T²³⁸ | H | CF₃ | CN | Cl |

| | | | |
|---|---|---|---|
| 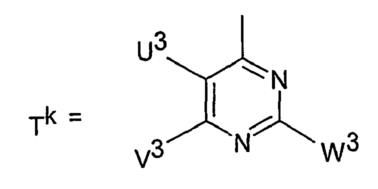 | | | |

| T^{k} | U³ | V³ | W³ |
|---|---|---|---|
| T³⁰⁰ | H | Me | Me |
| T³⁰¹ | H | Me | SMe |
| T³⁰² | H | Me | Cl |
| T³⁰³ | Cl | Me | Cl |
| T³⁰⁴ | H | Me | CF₃ |
| T³⁰⁵ | Cl | Me | CF₃ |
| T³⁰⁶ | Br | Me | CF₃ |
| T³⁰⁷ | Br | Me | Cl |
| T³⁰⁸ | F | Me | Cl |
| T³⁰⁹ | F | Me | CF₃ |
| T³¹⁰ | H | Et | Me |
| T³¹¹ | F | Et | Me |
| T³¹² | Cl | Et | Me |
| T³¹³ | Cl | Et | H |
| T³¹⁴ | Cl | Et | CF₃ |
| T³¹⁵ | F | Et | CF₃ |
| T³¹⁶ | H | Et | CF₃ |
| T³¹⁷ | H | CF₃ | H |
| T³¹⁸ | F | CF₃ | H |
| T³¹⁹ | Cl | CF₃ | H |
| T³²⁰ | Br | CF₃ | H |
| T³²¹ | H | CF₃ | Me |
| T³²² | F | CF₃ | Me |
| T³²³ | Cl | CF₃ | Me |
| T³²⁴ | Br | CF₃ | Me |
| T³²⁵ | H | CF₃ | SMe |
| T³²⁶ | F | CF₃ | SMe |
| T³²⁷ | Cl | CF₃ | SMe |
| T³²⁸ | Br | CF₃ | SMe |
| T³²⁹ | H | CF₃ | Cl |
| T³³⁰ | F | CF₃ | Cl |
| T³³¹ | Cl | CF₃ | Cl |
| T³³² | Br | CF₃ | Cl |
| T³³³ | H | CF₃ | CF₃ |
| T³³⁴ | F | CF₃ | CF₃ |
| T³³⁵ | Cl | CF₃ | CF₃ |
| T³³⁶ | Br | CF₃ | CF₃ |
| T³³⁷ | H | CF₃ | CCl₃ |
| T³³⁸ | F | CF₃ | CCl₃ |
| T³³⁹ | Cl | CF₃ | CCl₃ |
| T³⁴⁰ | Br | CF₃ | CCl₃ |
| T³⁴¹ | H | Me | CCl₃ |
| T³⁴² | F | Me | CCl₃ |
| T³⁴³ | Cl | Me | CCl₃ |
| T³⁴⁴ | Br | Me | CCl₃ |
| T³⁴⁵ | Br | H | CCl₃ |
| T³⁴⁶ | Cl | H | CF₂CF₂CF₃ |
| T³⁴⁷ | CF₃ | H | CF₂CF₂CF₃ |
| T³⁴⁸ | COOEt | H | CF₃ |
| T³⁴⁹ | Me | Me | CF₃ |
| T³⁵⁰ | H | H | CF₃ |

| | | | |
|---|---|---|---|
| 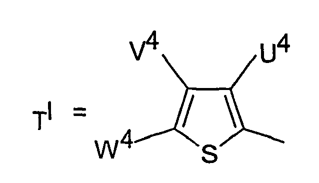 | | | |

| T^{l} | U⁴ | V⁴ | W⁴ |
|---|---|---|---|
| T⁴⁰⁰ | H | H | H |
| T⁴⁰¹ | Cl | H | H |
| T⁴⁰² | H | Cl | H |
| T⁴⁰³ | H | H | Cl |
| T⁴⁰⁴ | CF₃ | H | H |
| T⁴⁰⁵ | H | CF₃ | H |
| T⁴⁰⁶ | H | H | CF₃ |
| T⁴⁰⁷ | H | H | COMe |
| T⁴⁰⁸ | H | CF₃ | Cl |
| T⁴⁰⁹ | H | Cl | CF₃ |
| T⁴¹⁰ | H | Cl | Cl |
| T⁴¹¹ | H | NO₂ | Br |
| T⁴¹² | H | H | CHO |
| T⁴¹³ | H | H | 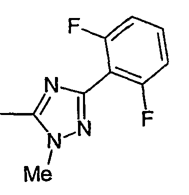 |
| T⁴¹⁴ | H | H | 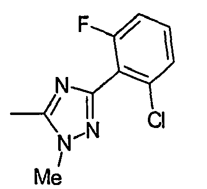 |
| T⁴¹⁵ | H | H | NO₂ |
| T⁴¹⁶ | _{H} | Me | Br |
| T⁴¹⁷ | H | H | Br |
| T⁴¹⁸ | H | H | (3-CF₃)Ph |
| T⁴¹⁹ | H | H | (4-F)Ph |
| T⁴²⁰ | _{H} | Me | (4-F)Ph |
| T⁴²¹ | H | Me | (4-Me)Ph |
| T⁴²² | H | Me | (4-Ph)Ph |
| T⁴²³ | _{H} | Me | 3-Thienyl |
| T⁴²⁴ | _{H} | Me | (4-Cl)Ph |
| T⁴²⁵ | H | H | Ph |
| T⁴²⁶ | H | H | (4-Me)Ph |
| T⁴²⁷ | H | H | (4-CF₃)Ph |
| T⁴²⁸ | H | H | (2-Cl)Ph |
| T⁴²⁹ | H | H | (4-OCF₃)Ph |
| T⁴³⁰ | H | H | (4-Cl)Ph |
| T⁴³¹ | H | H | (3-Cl)Ph |
| T⁴³² | H | H | (3-Me)Ph |
| T⁴³³ | H | H | (3-CF₃, 5-CF₃)Ph |
| T⁴³⁴ | H | H | (2-Cl, 4-Cl)Ph |
| T⁴³⁵ | H | H | (3-Cl, 5-Cl)Ph |
| T⁴³⁶ | H | H | (2-Cl, 4-Cl)Ph |
| T⁴³⁷ | H | H | -HC=NOCH₂Ph |
| T⁴³⁸ | H | H | Me |
| T⁴³⁹ | H | H | -HC=NOCH₂[(2-Cl, 4-Cl)Ph] |

| | | | |
|---|---|---|---|
| 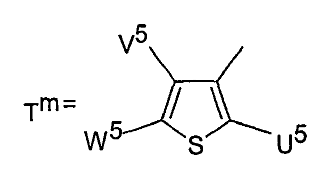 | | | |

| T^{m} | U⁵ | V⁵ | W⁵ |
|---|---|---|---|
| T⁴⁵⁰ | H | H | H |
| T⁴⁵¹ | H | CF₃ | H |
| T⁴⁵² | H | H | CF₃ |
| T⁴⁵³ | H | Cl | H |
| T⁴⁵⁴ | H | H | Cl |
| T⁴⁵⁵ | Cl | H | H |

| | |
|---|---|
| T⁵⁰⁰ | 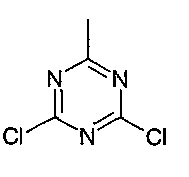 |
| T⁵⁰¹ | 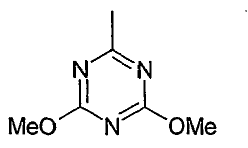 |
| T⁵⁰² | 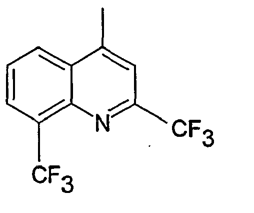 |
| T⁵⁰³ | 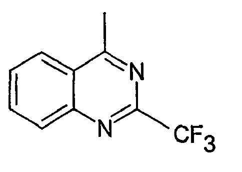 |
| T⁵⁰⁴ | 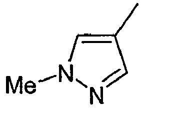 |
| T⁵⁰⁵ | 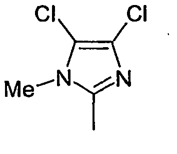 |
| T⁵⁰⁶ | 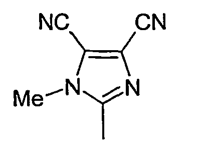 |
| T⁵⁰⁷ | 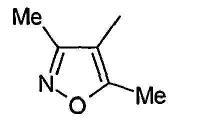 |
| T⁵⁰⁸ | 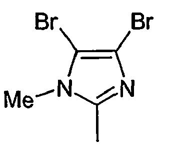 |
| T⁵⁰⁹ | 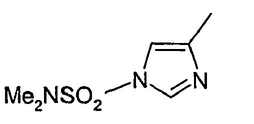 |
| T⁵¹⁰ | 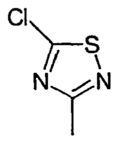 |
| T⁵¹¹ | 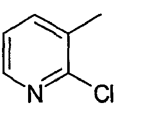 |
| T⁵¹² | 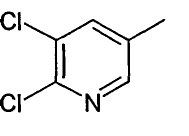 |
| T⁵¹³ | 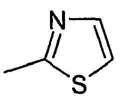 |
| T⁵¹⁴ | 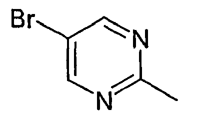 |
| T⁵¹⁵ | 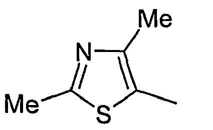 |
| T⁵¹⁶ | 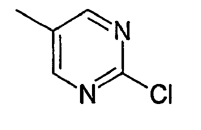 |
| T⁵¹⁷ | 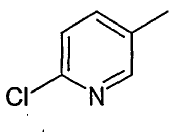 |
| T⁵¹⁸ | 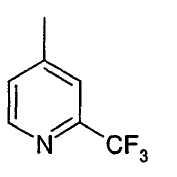 |
| T⁵¹⁹ | 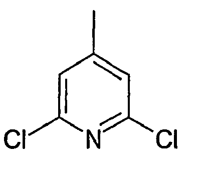 |
| T⁵²⁰ | 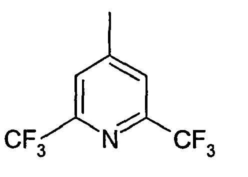 |
| T⁵²¹ | 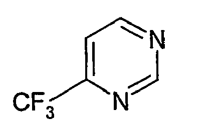 |
| T⁵²² | 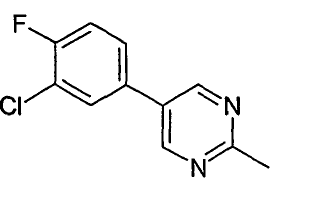 |
| T⁵²³ | 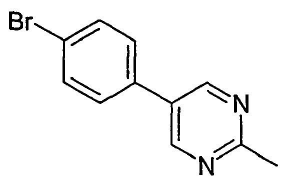 |
| T⁵²⁴ | 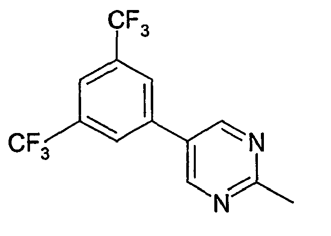 |
| T⁵²⁵ | 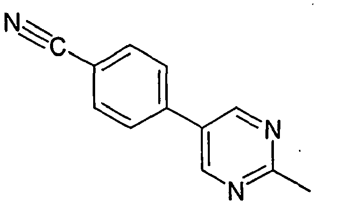 |
| T⁵²⁶ | 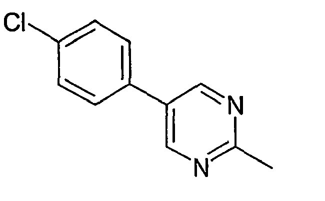 |
| T⁵²⁷ | 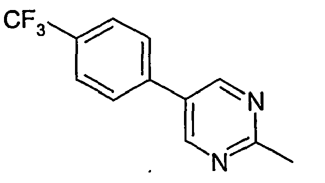 |
| T⁵²⁸ | 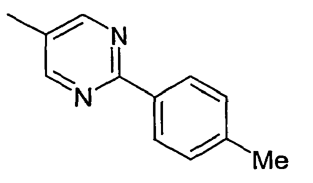 |
| T⁵²⁹ | 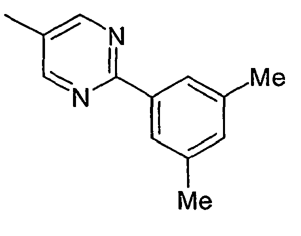 |
| T⁵³⁰ | 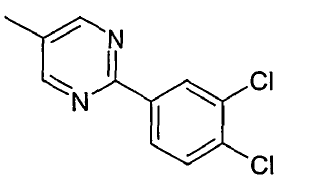 |
| T⁵³¹ | 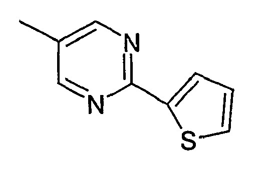 |

**Tabelle 1**

| | | | | |
|---|---|---|---|---|
| 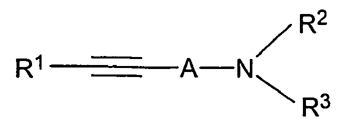 | | | | |

| Verb. Nr. | _{R}1 | A | 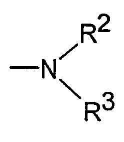 | Physiko-chemische Daten |
|---|---|---|---|---|
| 8 | T⁷⁶ | CH₂ | L² | Sdp 92°C/0,1 mm Hg |
| 9 | T⁷⁸ | CH₂ | L² | Sdp 120-124°C/0,015 mm Hg |
| 10 | T⁶⁰ | CH₂ | L² | Sdp 155°C/0,03 mm Hg |
| 11 | T²⁶ | CH₂ | L⁵⁰ | Sdp 110°C/0,04 mm Hg |
| 12 | T²⁷ | CH₂ | L⁵⁰ | Sdp 98°C/0,05 mm Hg |
| 13 | T⁸ | CH₂ | L⁵⁰ | Schmp 37-38°C |
| 14 | T⁴⁹ | CH₂ | L⁵⁰ | Sdp 123-125°C/0,06 mm Hg |
| 15 | T¹² | CH₂ | L⁵⁰ | Schmp 67-68°C |
| 16 | T¹⁵ | CH₂ | L⁵⁰ | Schmp 79-79,5 °C |
| 17 | T⁸² | CH₂ | L⁵⁰ | Schmp 52-53°C |
| 18 | T⁴⁹ | CH₂ | L⁵¹ | Sdp 135°C/0,05 mm Hg |
| 19 | T¹² | CH₂ | L⁵¹ | Sdp 142-144°C/0,2 mm Hg |
| 20 | T⁷⁷ | CH₂ | L⁵⁰ | Sdp 118°C/0,05 mm Hg |
| 21 | T⁸⁵ | CH₂ | L⁵⁰ | Sdp 145°C/0,1 mm Hg |
| 22 | T⁶³ | CH₂ | L⁵⁰ | Schmp 64-65°C |
| 23 | T⁶¹ | CH₂ | L⁵⁰ | Schmp 90-91 °C |
| 24 | T³² | CH₂ | L⁵⁰ | Sdp 92-93°C/0,06 mm Hg |
| 25 | T⁸⁸ | CH₂ | L⁵⁰ | Sdp 106-108°C/0,015 mm Hg |
| 26 | T³¹ | CH₂ | L⁵⁰ | Sdp 87-89°C/0,04 mm Hg |
| 27 | T⁷⁶ | CH₂ | L⁵⁰ | Sdp 121°C/0,05 mm Hg |
| 28 | T²³ | CH₂ | L⁵⁰ | Sdp 91-93°C/0,03 mm Hg |
| 29 | T³³ | CH₂ | L⁵⁰ | Sdp 96-98°C/0,02 mm Hg |
| 30 | T⁴⁴ | CH₂ | L⁵⁰ | Sdp 97-98°C/0,01 mm Hg |
| 31 | T³⁹ | CH₂ | L³⁷ | Sdp 90-96°C/0,1 mm Hg |
| 32 | T³⁹ | CH₂ | L⁵¹ | Blaßgelbes Öl |
| 33 | T³⁹ | CH₂ | L⁵² | Sdp 88-90°C/1,0 mm Hg |
| 34 | T³⁹ | CH₂ | L⁵³ | Sdp 110-115°C/0,07 mm Hg |
| 35 | T¹ | CH₂ | L⁵² | Sdp 98-99°C/0,085 mm Hg |
| 36 | T³¹¹ | CH₂ | L⁵⁰ | ¹H-NMR (CDCl₃): δ = 0,95 (d,3H), 1,31 (t,3H), 2,62 (s,3H), 3,61 (s,2H) |
| 37 | T³¹³ | CH₂ | L⁵⁰ | ¹H-NMR (CDCl₃): δ = 0,95 (d,3H), 1,30 (t,3H), 2,95 (q,2H), 3,68 (s,2H) 8,92 (s,1H) |
| 38 | T³¹⁰ | CH₂ | L⁵⁰ | ¹H-NMR (CDCl₃): δ = 0,95 (d,3H), 1,30 (t,3H) 2,70 (s,3H), 2,75 (q,2H), 3,56 (s,2H), 7,06 (s,1H) |
| 39 | T³⁹ | CH₂ | L¹⁴ | ¹H-NMR (CDCl₃): δ = 1,17 (d,3H), 3,57 (d,1H) 3,85 (d,1H), 7,77 (s,1H) 7,83 (s,2H) |
| 40 | T³⁹ | CH₂ | L³ | ¹H-NMR (CDCl₃): δ = 1,58-1,80 (m, 8H), 2,78 (m, 4H),3,61 (s, 2H), 7,77 (s, 1H), 7,83 (s, 2H) |
| 41 | T³⁹ | CH₂ | L¹⁸ | ¹H-NMR (CDCl₃): δ = 2,24 (m, 2H), 2,76 (t, 2H), 3,18 (m, 2H), 3,62 (S, 2H), 5,75 (m, 2H) |
| 42 | T³⁹ | CH₂ | L⁶ | ¹H-NMR (CDCl₃): δ = 3,56 (d, 1H), 3,91 (d, 1H) |
| 43 | T³⁹ | CH₂ | L²² | ¹H-NMR (CDCl₃): δ = 1,00 (d, 6H), 1,30 (t, 2H) 1,98 (m, 2H), 2,22 (m, 2H) 2,58 (m, 2H), 3,50 (s, 2H) |
| 44 | T³⁹ | CH₂ | L²³ | ¹H-NMR (CDCl₃): δ = 0,90 (d, 6H), 1,75 (m, 6H), 2,88 (m, 2H), 3,55 (s, 2H) |
| 45 | T³⁹ | CH₂ | L²⁴ | ¹H-NMR (CDCl₃): δ = 0,90 (t, 3H), 3,52 (s, 2H) |
| 46 | T³⁹ | CH₂ | L³⁰ | ¹H-NMR (CDCl₃): δ = 1,28 (t, 3H), 3,56 (s, 2H), 4,17 (q, 2H) |
| 47 | T³⁹ | CH₂ | L³¹ | ¹H-NMR (CDCl₃): δ = 1,43 (s, 9H), 2,58 (t, 4H), 3,50 (t, 4H), 3,58 (s, 2H) |
| 48 | T³⁹ | CH₂ | L³⁶ | ¹H-NMR (CDCl₃): δ = 1,82 (t, 4H), 2,73 (t, 4H), 3,57 (s, 2H), 3,98 (s, 4H) |
| 49 | T³⁹ | CH₂ | L⁴⁴ | ¹H-NMR (CDCl₃): δ = 0,95 (t, 3H), 3,56 (d, 1H), 3,87 (d, 1H) |
| 50 | T³⁹ | CH₂ | L⁴⁷ | ¹H-NMR (CDCl₃): δ = 2,96 (m, 4H), 3,77 (s, 2H), 3,83 (s, 2H), 7,0-7,2 (m, 4H) |
| 51 | T³⁹ | CH₂ | L¹⁷ | ¹H-NMR (CDCl₃): δ = 2,45 (s, 3H), 3,59 (s, 2H) |
| 52 | T³³ | CH₂ | L¹⁷ | ¹H-NMR (CDCl₃): δ = 2,36 (s, 3H), 3,57 (s, 2H), 7,55 (m, 4H) |
| 53 | T⁴⁴ | CH₂ | L¹⁷ | ¹H-NMR (CDCl₃): δ = 2,33 (s, 3H), 3,60 (s, 2H) |
| 54 | T³³ | CH₂ | L¹² | ¹H-NMR (CDCl₃): δ = 2,62 (t, 4H), 3,55 (s, 2H), 3,78 (t, 4H) |
| 55 | T⁴⁴ | CH₂ | L¹² | ¹H-NMR (CDCl₃): δ = 2,68 (t, 4H), 3,60 (s, 2H), 3,78 (t, 4H), 7,5 (m, 2H), 7,76 (d, 1H) |
| 56 | T²⁰⁰ | CH₂ | L¹² | ¹H-NMR (CDCl₃): δ = 2,70 (t, 4H), 3,65 (s, 2H), 3,78 (t, 4H), 7,97 (d, 1H), 8,72 (d, 1H) |
| 57 | T²⁰⁰ | CH₂ | L⁵⁰ | ¹H-NMR (CDCl₃): δ = 0,96 (d, 3H), 1,20-1,43 (m, 3H), 1,7 (m, 2H), 2,39 (m, 2H), 2,95 (m, 2H), 3,65 (s, 2H), 7,97 (d, 1H), 8,71 (d, 1H) |
| 58 | T²¹⁸ | CH₂ | L⁵⁰ | ¹H-NMR (CDCl₃): δ = 0,96 (d, 3H), 3,58 (s, 2H), 7,52 (d, 1H), 7,86 (dd, 1H), 8,81 (d, 1H) |
| 59 | T²¹⁴ | CH₂ | L⁵⁰ | ¹H-NMR (CDCl₃): δ = 0,94 (d, 3H), 3,55 (s, 2H), 7,30 (d, 1H), 7,78 (dd, 1H), 8,61 (d, 1H) |
| 60 | T²³³ | CH₂ | L⁵⁰ | ¹H-NMR (CDCl₃): δ = 0,96 (d, 3H), 3,58 (s, 2H), 7,78 (d, 1H), 7,80 (d, 1H) |
| 61 | T³⁹ | CH₂ | L³³ | ¹H-NMR (CDCl₃): δ = 0,95 (s, 3H), 1,06 (s, 3H), 1,24 (s, 3H), 3,57 (d, 1H), 3,65 (d, 1H) |
| 62 | T²⁰⁰ | CH₂ | L¹⁷ | ¹H-NMR (CDCl₃): δ = 2,48 (s, 3H), 3,68 (s, 2H), 7,96 (d, 1H), 8,72 (d, 1H) |
| 63 | T³⁹ | CH₂ | L³⁴ | ¹H-NMR (CDCl₃): δ = 1,6-2,1 (m, 8H), 2,75 (m, 3H), 2,97 (m, 2H), 3,46 (s, 2H) |
| 64 | T⁴⁴ | CH₂ | L³⁴ | ¹H-NMR (CDCl₃): δ = 2,4 (m, 2H), 2,60 (m, 4H), 2,95 (m, 2H), 3,60 (s, 2H), 7,45 (dd, 1H), 7,51 (d, 1H), 7,73 (d, 1H) |
| ₆₅ | T³³ | CH₂ | _{L}43 | ¹H-NMR (CDCl₃): δ = 2,57 (d, 2H), 3,48 (s, 2H), 7,13 (m, 3H), 7,27 (m, 2H), 7,54 (m, 4H) |
| 66 | T⁴⁴ | CH₂ | L⁴³ | ¹H-NMR (CDCl₃): δ = 2,57 (d, 2H), 3,58 (s, 2H), 7,13 (m, 3H), 7,27 (m, 2H), 7,48 (m, 2H), 7,70 (d, 1H) |
| 67 | T³⁹ | CH₂ | L⁴³ | ¹H-NMR (CDCl₃): δ = 2,58 (d, 2H), 3,50 (s, 2H), 7,13 (m, 3H), 7,27 (m, 2H), 7,78 (s, 1H), 7,82 (s, 2H) |
| 68 | T³³ | CH₂ | L³⁴ | ¹H-NMR (CDCl₃): δ = 1,82 (m, 4H), 2,32 (m, 2H), 2,62 (m, 4H), 2,97 (m, 2H), 3,53 (s, 2H), 7,55 (m, 4H) |
| 69 | T¹ | CH₂ | L¹⁴ | |
| 70 | T⁴⁶ | CH₂ | L⁵⁰ | ¹H-NMR (CDCl₃): δ = 0,97 (d, 3H), 1,36 (m, 3H), 2,21 (m, 2H), 2,94 (m, 2H), 3,52 (s, 2H), 7,20 (dd, 1H), 7,57 (d, 1H), 7,60 (d, 1H) |
| 71 | T⁴⁰⁰ | CH₂ | L⁵⁰ | ¹H-NMR (CDCl₃): δ = 0,97 (d, 3H), 3,50 (s, 2H), 6,97 (dd, 1H), 7,18 (dd, 1H), 7,22 (dd, 1H) |
| 72 | T⁴⁵⁰ | CH₂ | L⁵⁰ | ¹H-NMR (CDCl₃): δ = 0,97 (d, 3H), 3,47 (s, 2H), 7,10 (d, 1H), 7,24 (dd, 1H), 7,40 (d, 1H) |
| 73 | T³⁹ | CH(CH₃) | L⁵⁰ | |
| 74 | T³⁹ | C(CH₃)₂ | L⁵⁰ | |
| 75 | T³⁹ | CO | L⁵⁰ | |
| 76 | T³⁹ | CF₂ | L⁵⁰ | |
| 77 | T³⁹ | CH(CH₂Ph) | L⁵⁰ | Sdp 140°C/0,01 mm Hg |
| 78 | T⁴⁶ | CH(CH₃) | L⁵⁰ | |
| 79 | T⁴⁶ | C(CH₃)₂ | L⁵⁰ | |
| 80 | T⁴⁶ | CO | L⁵⁰ | |
| 81 | T⁴⁶ | CF₂ | L⁵⁰ | |
| 82 | T⁴⁶ | CH(CH₂Ph) | L⁵⁰ | |
| 83 | T¹ | CH₂ | L⁴⁵ | |
| 84 | T¹ | CH₂ | L¹⁹ | Öl |
| 85 | T³⁹ | CH₂ | L⁴⁵ | |
| 86 | T³⁹ | CH₂ | L¹⁹ | |
| 87 | T¹ | CH₂ | L¹² | |
| 88 | T³⁹ | CH₂ | L¹⁴ | |
| 89 | T²¹ | CH₂ | L⁵⁰ | Sdp 95°C/0,02 mm Hg |
| 90 | T⁶² | CH₂ | L⁵⁰ | Sdp 144°C/0,03 mm Hg |
| 91 | T⁴ | CH₂ | L⁵⁰ | Schmp 48-49°C |
| 92 | T¹⁰³ | CH₂ | L⁵⁰ | Schmp 89,5-90°C |
| 93 | T⁸⁶ | CH₂ | L⁵⁰ | Sdp 122-124°C/0,01 mm Hg |
| 94 | T³ | CH₂ | L⁵⁰ | Sdp 110-114°C/0,015 mm Hg |
| 95 | T¹⁰⁴ | CH₂ | L⁵⁰ | Sdp 113°C/0,04 mm Hg |
| 96 | T¹⁰⁵ | CH₂ | L⁵⁰ | Sdp 108°C/0,03 mm Hg |
| 97 | T⁵² | CH₂ | L⁵⁰ | Sdp 119-120°C/0,005 mm Hg |
| 98 | T⁸⁹ | CH₂ | L⁵⁰ | Sdp 144-146°C/0,015 mm Hg |
| 99 | T¹⁰⁶ | CH₂ | L⁵⁰ | Sdp 124°C/0,8 mm Hg |
| 100 | T⁵⁶ | CH₂ | L⁵⁰ | Sdp 126-128°C/0,05 mm Hg |
| 101 | T¹⁰⁷ | CH₂ | L⁵⁰ | Sdp 127°C/0,002 mm Hg |
| 102 | T¹⁰⁸ | CH₂ | L⁵⁰ | Sdp 110-112°C/0,05 mm Hg |
| 103 | T⁵³ | CH₂ | L⁵⁰ | Sdp 123-126°C/0,01 mm Hg |
| 104 | T¹¹¹ | CH₂ | L⁵⁰ | Sdp 135°C/0,05 mm Hg |
| 105 | T¹¹² | CH₂ | L⁵⁰ | Sdp 125°C/0,06 mm Hg |
| 106 | T⁵ | CH₂ | L⁵⁰ | Sdp 140-142°C/0,05 mm Hg |
| 107 | T⁷ | CH₂ | L⁵⁰ | Sdp 125°C/0,005 mm Hg |
| 108 | T¹¹⁶ | CH₂ | L⁵⁰ | Schmp 36-37°C |
| 109 | T¹¹⁷ | CH₂ | L⁵⁰ | Schmp 57-58°C |
| 110 | T¹¹⁸ | CH₂ | L⁵⁰ | Sdp 119°C/0,005 mm Hg |
| 111 | T³⁹ | CH₂ | L²² | Sdp 97-101°C/0,2 mm Hg |
| 112 | T³⁹ | CH₂ | L⁹⁰ | Sdp 120-125°C/0,07 mm Hg |
| 113 | T³⁹ | CH₂ | L²⁴ | Sdp 130°C/0,35 mm Hg |
| 114 | T⁸⁷ | CH₂ | L⁵⁰ | Sdp 126-129°C/0,11 mm Hg |
| 115 | T¹¹⁹ | CH₂ | L⁵⁰ | Schmp 66-68°C |
| 116 | T¹²⁰ | CH₂ | L⁵⁰ | Sdp 138-143°C/0,05 mm Hg |
| 117 | T⁷⁹ | CH₂ | L⁵⁰ | Schmp 62-63°C |
| 118 | T³⁹ | CH₂ | L⁷⁶ | |
| 119 | T⁹⁴ | CH₂ | L⁵⁰ | Schmp 47-48°C |
| 120 | T⁷⁸ | CH₂ | L⁵⁰ | Sdp 130°C/0,03 mm Hg |
| 121 | T²⁶ | CH₂ | L⁵⁰ | Sdp 110°C/0,04 mm Hg |
| 122 | T²⁵ | CH₂ | L⁵⁰ | Sdp 95°C/0,03 mm Hg |
| 123 | T¹²³ | CH₂ | L⁵⁰ | |
| 124 | T⁵⁹ | CH₂ | L⁵⁰ | |
| 125 | T³⁹ | CH₂ | L²⁸ | Schmp 47-48°C |
| 126 | T¹²⁴ | CH₂ | L⁵⁰ | Sdp 118°C/0,05 mm Hg |
| 127 | T¹²⁶ | CH₂ | L⁵⁰ | Sdp 131°C/0,1 mm Hg |
| 128 | T³⁹ | CH₂ | L⁷⁸ | Schmp 86-87°C |
| 129 | T³⁹ | CH₂ | L⁶⁴ | Schmp 70-72°C |
| 130 | T¹²⁸ | CH₂ | L⁵⁰ | Sdp 162°C/0,25 mm Hg |
| 131 | T¹²⁹ | CH₂ | L⁵⁰ | Schmp 58-59°C |
| 132 | T³⁹ | CH₂ | L⁸⁰ | Schmp 45°C |
| 133 | T¹³⁰ | CH₂ | L⁵⁰ | Sdp 132-134°C/0,2 mm Hg |
| 134 | T¹ | CH(Prⁱ) | L² | Sdp 130-130°C/0,2 mm Hg |
| 135 | T¹⁴¹ | CH₂ | L⁵⁰ | Sdp 126-132°C/0,1 mm Hg |
| 136 | T¹³¹ | CH₂ | L⁵⁰ | Sdp 112-116°C/0,1 mm Hg |
| 137 | T¹³² | CH₂ | L⁵⁰ | Sdp105-110°C/0,1 mmHg |
| 138 | T¹ | CH(Prⁱ) | L⁵⁰ | Sdp 156-158°C/1,75 mm Hg |
| 139 | T³⁹ | CH₂ | L⁸⁶ | Wachs |
| 140 | T¹ | CO | L⁵⁰ | Schmp 56-57°C |
| 141 | T¹³⁴ | CH₂ | L⁵⁰ | Sdp 160°C/0,2 mm Hg |
| 142 | T⁶⁴ | CH₂ | L⁵⁰ | Schmp 58-59°C |
| 143 | T¹³⁵ | CH₂ | L⁵⁰ | Sdp 145°C/0,1 mm Hg |
| 144 | T¹³³ | CH₂ | L⁵¹ | |
| 145 | T¹³⁶ | CH₂ | L⁵⁰ | Schmp 46-47°C |
| 146 | T¹³⁷ | CH₂ | L⁵⁰ | |
| 147 | T³⁹ | CO | L⁵⁰ | Schmp 110-111°C |
| 148 | T¹³⁸ | CH₂ | L⁵⁰ | Schmp 63-64°C |
| 149 | T³⁹ | CH₂ | L⁷⁹ | Sdp 185-187°C/0,7 mm Hg |
| 150 | T¹⁴⁰ | CH₂ | L⁵⁰ | Schmp 50-51 °C |
| 151 | T⁶ | CH₂ | L⁶³ | Öl |
| 152 | T³⁹ | CH₂ | L⁵⁰ | Sdp 92-95°C/0,04 mm Hg; Schmp 22°C |
| 153 | T⁴⁴ | CH₂ | L³³ | ¹H-NMR (CDCl₃): δ = 0,92 (s, 3H), 1,06 (s, 3H), 1,23 (s, 3H), 3,60 (d, 1H), 3,75 (d, 1H), 7,70 (d,1H) |
| 154 | T³³ | CH₂ | L³³ | ¹H-NMR (CDCl₃): δ = 0,86 (s, 3H), 1,06 (s, 3H), 1,25 (s, 3H), 3,53 (d, 1H), 3,65 (d, 1H), 7,50 (d,2H), 7,58 (d,2H) |
| 155 | T⁵¹¹ | CH₂ | L⁵⁰ | ¹H-NMR (CDCl₃): δ = 0,96 (d, 3H), 3,58 (s, 2H), 7,18 (dd, 1H), 7,68 (dd, 1H), 8,32 (dd,1H) |
| 156 | T⁵¹² | CH₂ | L⁵⁰ | ¹H-NMR (CDCl₃): δ = 0,95 (d, 3H), 3,48 (s, 2H), 7,78 (d, 1H), 8,36 (d, 1H) |
| 157 | T⁵⁰² | CH₂ | L⁵⁰ | ¹H-NMR (CDCl₃): δ = 0,97 (d, 3H), 3,70 (s, 2H), 7,75 (t, 1H), 7,83 (s, 1H), 8,19 (d, 1H), 8,55 (d, 1H) |
| 158 | T²¹⁹ | CH₂ | L⁵⁰ | Schmp 75-77°C |
| 159 | T³²⁵ | CH₂ | L²² | ¹H-NMR (CDCl₃): δ = 0,98 (d, 6H), 1,30 (t, 3H), 3,56 (d, 2H), 7,25 (s, 1H) |
| 160 | T³²⁵ | CH₂ | L⁵⁰ | ¹H-NMR (CDCl₃): δ = 0,97 (d, 3H), 2,60 (s, 3H), 3,57 (s, 2H), 7,27 (s, 1H) |
| 161 | T³²¹ | CH₂ | L⁵⁰ | ¹H-NMR (CDCl₃): δ = 0,97 (d, 3H), 2,80 (s, 3H), 3,59 (s, 2H), 7,49 (s, 1H) |
| 162 | T²³³ | CH₂ | L²² | ¹H-NMR (CDCl₃): δ = 0,98 (d, 6H), 1,30 (t, 3H), 3,55 (s, 2H), 7,77 (s, 1H), 7,79 (s, 1H) |
| 163 | T²³³ | CH₂ | L²³ | ¹H-NMR (CDCl₃): δ = 0,90 (d, 6H), 1,65-1,85 (m, 6H), 3,58 (s, 2H), 7,76 (s, 1H), 7,80 (s, 1H) |
| 164 | T³²⁵ | CH₂ | L³ | ¹H-NMR (CDCl₃): δ = 1,55-1,80 (m, 8H), 2,60 (s, 3H), 3,67 (s, 2H), 7,25 (s, 1H) |
| 165 | T²³¹ | CH₂ | L⁵⁰ | ¹H-NMR (CDCl₃): δ = 0,95 (d, 3H), 3,65 (s, 2H), 7,70 (d, 1H), 8,16 (d,1H) |
| 166 | T³²⁵ | CH₂ | L²³ | ¹H-NMR (CDCl₃): δ = 0,90 (d, 6H), 2,60 (t, 3H), 3,59 (s, 2H), 7,29 (s, 1H) |
| 167 | T³⁵⁰ | CH₂ | L⁵⁰ | Öl |
| 168 | T⁵²¹ | CH₂ | L⁵⁰ | Öl |
| 169 | T³⁹ | CH₂ | L⁶⁹ | Öl |
| 170 | T²³³ | CH₂ | L⁶⁹ | Öl |
| 171 | T¹⁴² | CH₂ | L⁵⁰ | Öl |
| 172 | T¹⁴³ | CH₂ | L⁵⁰ | Öl |
| 173 | T¹⁴⁴ | CH₂ | L⁵⁰ | Öl |
| 174 | T¹⁴⁵ | CH₂ | L⁵⁰ | Öl |
| 175 | T¹⁴⁶ | CH₂ | L⁵⁰ | Öl |
| 176 | T¹⁴⁸ | CH₂ | L⁵⁰ | Öl |
| 177 | T¹⁴⁹ | CH₂ | L⁵⁰ | Öl |
| 178 | T¹⁵⁰ | CH₂ | L⁵⁰ | Öl |
| 179 | T¹⁵¹ | CH₂ | L⁵⁰ | Öl |
| 180 | T¹⁵² | CH₂ | L⁵⁰ | Öl |
| 181 | T¹⁴⁷ | CH₂ | L⁵⁰ | Öl |
| 182 | T¹⁵³ | CH₂ | L⁵⁰ | Öl |
| 183 | T¹⁵⁴ | CH₂ | L⁵⁰ | Öl |
| 184 | T¹⁵⁵ | CH₂ | L⁵⁰ | Öl |
| 185 | T¹⁵⁶ | CH₂ | L⁵⁰ | Öl |
| 186 | T¹⁵⁷ | CH₂ | L⁵⁰ | Öl |
| 187 | T¹⁵⁸ | CH₂ | L⁵⁰ | Öl |
| 188 | T¹⁵⁹ | CH₂ | L⁵⁰ | Öl |
| 189 | T¹⁶⁰ | CH₂ | L⁵⁰ | Öl |
| 190 | T¹⁶¹ | CH₂ | L⁵⁰ | Öl |
| 191 | T¹⁶² | CH₂ | L⁵⁰ | Öl |
| 192 | T1⁶³ | CH₂ | L⁵⁰ | Öl |
| 193 | T¹⁶⁴ | CH₂ | L⁵⁰ | Öl |
| 194 | T¹⁶⁵ | CH₂ | L⁵⁰ | Öl |
| 195 | T¹⁶⁶ | CH₂ | L⁵⁰ | Öl |
| 196 | T¹⁶⁷ | CH₂ | L⁵⁰ | Öl |
| 197 | T²³⁶ | CH₂ | L⁵⁰ | ¹H-NMR(CDCl₃) : δ = 0,97 (d, 3H), 2,62 (s, 3H), 3,55 (s, 2H), 7,29 (s, 1H), 7,45 (s, 1H). |
| 198 | T²³³ | CH₂ | L⁷² | Öl |
| 199 | T³⁹ | CH₂ | L⁷² | Öl |
| 200 | T³²⁹ | CH₂ | L⁵⁰ | Öl |
| 201 | T²³⁷ | CH₂ | L⁵⁰ | Öl |
| 202 | T²³⁸ | CH₂ | L⁵⁰ | Öl |
| 203 | T²¹⁷ | CH₂ | L⁵⁰ | Öl |
| 204 | T²³³ | CH₂ | L⁹¹ | ¹H-NMR(CDCl₃) : δ = 3,62 (d, 2H), 7,80 (s, 2H). |
| 205 | T³⁹ | CH₂ | L⁹¹ | Öl |
| 206 | T⁵¹³ | CH₂ | L⁵⁰ | Öl |
| 207 | T¹⁶⁸ | CH₂ | L⁵⁰ | Öl |
| 208 | T⁴¹⁵ | CH₂ | L⁵⁰ | Schmp 54°C |
| 209 | T¹⁶⁹ | CH₂ | L⁵⁰ | Öl |
| 210 | T⁵¹⁴ | CH₂ | L⁵⁰ | Öl |
| 211 | T⁵¹⁵ | CH₂ | L⁵⁰ | Öl |
| 212 | T⁵¹⁶ | CH₂ | L⁵⁰ | Schmp 77-78°C |
| 213 | T⁴¹⁶ | CH₂ | L⁵⁰ | Schmp 96-97°C |
| 214 | T⁴⁰⁷ | CH₂ | L⁵⁰ | Schmp 73-74°C |
| 215 | T⁴¹⁷ | CH₂ | L⁵⁰ | Öl |
| 216 | T⁴⁰³ | CH₂ | L⁵⁰ | Öl |
| 217 | T¹⁷⁰ | CH₂ | L⁵⁰ | Öl |
| 218 | T¹⁷¹ | CH₂ | L⁵⁰ | Öl |
| 219 | T¹⁷² | CH₂ | L⁵⁰ | Öl |
| 220 | T¹⁷³ | CH₂ | L⁵⁰ | Öl |
| 221 | T¹⁷⁴ | CH₂ | L⁵⁰ | Öl |
| 222 | T¹⁷⁵ | CH₂ | L⁵⁰ | Öl |
| 223 | T¹⁷⁶ | CH₂ | L⁵⁰ | Öl |
| 224 | T¹⁷⁷ | CH₂ | L⁵⁰ | Öl |
| 225 | T¹⁷⁸ | CH₂ | L⁵⁰ | Öl |
| 226 | T¹⁷⁹ | CH₂ | L⁵⁰ | Öl |
| 227 | T¹⁸⁰ | CH₂ | L⁵⁰ | Öl |
| 228 | T¹⁸¹ | CH₂ | L⁵⁰ | Öl |
| 229 | T⁴¹⁸ | CH₂ | L⁵⁰ | Öl |
| 230 | T⁴¹⁹ | CH₂ | L⁵⁰ | Öl |
| 231 | T¹⁸⁹ | CH₂ | L⁵⁰ | Öl |
| 232 | T⁴²⁰ | CH₂ | L⁵⁰ | Öl |
| 233 | T⁵¹⁷ | CH₂ | L⁵⁰ | Öl |
| 234 | T²⁰³ | CH₂ | L⁵⁰ | 1 H-NMR(CDCl₃) : δ = 0,96 (d, 3H), 3,56 (s, 2H), 7,45 (s, 1H), 7,55 (s, 1H). |
| 235 | T⁵¹⁸ | CH₂ | L⁵⁰ | Öl |
| 236 | T⁴²¹ | CH₂ | L⁵⁰ | Öl |
| 237 | T⁴²² | CH₂ | L⁵⁰ | Öl |
| 238 | T⁴²³ | CH₂ | L⁵⁰ | Öl |
| 239 | T⁴²⁴ | CH₂ | L⁵⁰ | Öl |
| 240 | T⁴²⁵ | CH₂ | L⁵⁰ | Öl |
| 241 | T⁴²⁶ | CH₂ | L⁵⁰ | Öl |
| 242 | T⁴²⁷ | CH₂ | L⁵⁰ | Öl |
| 243 | T⁴²⁸ | CH₂ | L⁵⁰ | Öl |
| 244 | T⁴²⁹ | CH₂ | L⁵⁰ | Öl |
| 245 | T⁴³⁰ | CH₂ | L⁵⁰ | Öl |
| 246 | T⁴³² | CH₂ | L⁵⁰ | Öl |
| 247 | T⁴³¹ | CH₂ | L⁵⁰ | Öl |
| 248 | T¹⁹⁰ | CH₂ | L⁵⁰ | Öl |
| 249 | T¹⁹¹ | CH₂ | L⁵⁰ | Öl |
| 250 | T¹⁹² | CH₂ | L⁵⁰ | Öl |
| 251 | T¹⁹³ | CH₂ | L⁵⁰ | Öl |
| 252 | T¹⁹⁵ | CH₂ | L⁵⁰ | Öl |
| 253 | T¹⁹⁴ | CH₂ | L⁵⁰ | Öl |
| 254 | T¹⁸² | CH₂ | L⁵⁰ | Öl |
| 255 | T¹⁹⁶ | CH₂ | L⁵⁰ | Öl |
| 256 | T¹⁹⁷ | CH₂ | L⁵⁰ | Öl |
| 257 | T¹⁹⁸ | CH₂ | L⁵⁰ | Öl |
| 258 | T¹⁹⁹ | CH₂ | L⁵⁰ | Öl |
| 259 | T⁶⁰⁰ | CH₂ | L⁵⁰ | Öl |
| 260 | T⁶⁰¹ | CH₂ | L⁵⁰ | Öl |
| 261 | T⁶⁰² | CH₂ | L⁵⁰ | Öl |
| 262 | T⁶⁰³ | CH₂ | L⁵⁰ | Öl |
| 263 | T⁶⁰⁴ | CH₂ | L⁵⁰ | Öl |
| 264 | T¹⁸³ | CH₂ | L⁵⁰ | Öl |
| 265 | T⁶⁰⁵ | CH₂ | L⁵⁰ | Öl |
| 266 | T⁶⁰⁶ | CH₂ | L⁵⁰ | Öl |
| 267 | T³⁹ | CH₂ | L⁵³ | Öl |
| 268 | T⁶⁰⁷ | CH₂ | L⁵⁰ | Öl |
| 269 | T⁶⁰⁸ | CH₂ | L⁵⁰ | Öl |
| 270 | T⁶⁰⁹ | CH₂ | L⁵⁰ | Öl |
| 271 | T⁴³³ | CH₂ | L⁵⁰ | Öl |
| 272 | T⁴³⁴ | CH₂ | L⁵⁰ | Öl |
| 273 | T⁴³⁵ | CH₂ | L⁵⁰ | Öl |
| 274 | T⁴³⁶ | CH₂ | L⁵⁰ | Öl |
| 275 | T⁵²² | CH₂ | L⁵⁰ | Schmp 132°C |
| 276 | T⁵²³ | CH₂ | L⁵⁰ | Schmp 141°C |
| 277 | T⁵²⁴ | CH₂ | L⁵⁰ | Schmp 146°C |
| 278 | T5²5 | CH₂ | L⁵⁰ | Schmp 169°C |
| 279 | T⁵²⁶ | CH₂ | L⁵⁰ | Schmp 143°C |
| 280 | T⁵²⁷ | CH₂ | L⁵⁰ | Schmp 149°C |
| 281 | T³⁹ | CH₂ | L⁹³ | Öl |
| 282 | T²³³ | CH₂ | L⁹³ | Öl |
| 283 | T¹⁸⁵ | CH₂ | L⁵⁰ | Öl |
| 284 | T¹⁸⁶ | CH₂ | L⁵⁰ | Öl |
| 285 | T¹⁸⁷ | CH₂ | L⁵⁰ | Öl |
| 286 | T¹⁸⁸ | CH₂ | L⁵⁰ | Öl |
| 287 | T³⁹ | CH₂ | L⁹² | Öl |
| 288 | T²³³ | CH₂ | L⁹² | Öl |
| 289 | T⁶⁹ | CH₂ | L⁵⁰ | Schmp 61 °C |
| 290 | T⁷⁵ | CH₂ | L⁵⁰ | Schmp 73°C |
| 291 | T⁵¹⁹ | CH₂ | L⁵⁰ | 1H-NMR(CDCl₃) : δ = 0,96 (d, 3H), 3,5 (s, 2H), 7,23 (s, 2H). |
| 292 | T⁴³⁷ | CH₂ | L⁵⁰ | Öl |
| 293 | T⁴³⁸ | CH₂ | L⁵⁰ | Öl |
| 294 | T²³⁷ | CH₂ | L⁵⁰ | Öl |
| 295 | T¹⁴⁰ | CH₂ | L⁵⁰ | Öl |
| 296 | T⁴³⁹ | CH₂ | L⁵⁰ | Öl |
| 297 | T³² | CH₂ | L⁹³ | Öl |
| 298 | T⁴¹² | CH₂ | L⁵⁰ | Öl |
| 299 | T⁴¹³ | CH₂ | L⁵⁰ | Öl |
| 300 | T⁵²⁸ | CH₂ | L⁵⁰ | Öl |
| 301 | T⁵²⁹ | CH₂ | L⁵⁰ | Öl |
| 302 | T⁵³⁰ | CH₂ | L⁵⁰ | Öl |
| 303 | T⁵³¹ | CH₂ | L⁵⁰ | Öl |
| 304 | T⁵²⁰ | CH₂ | L⁵⁰ | 1 H-NMR(CDCl₃) : δ = 0,97 (d, 3H), 3,57 (s, 2H), 7,82 (s, 2H). |
| 305 | T⁴¹⁴ | CH₂ | L⁵⁰ | Öl |

Weitere Verbindungen, die in dieser Tabelle nicht gezeigt werden, können mit weiteren Kombinationen der definierten Gruppen T, A und L erhalten werden.

**Tabelle 2**

| | | | | | |
|---|---|---|---|---|---|
| 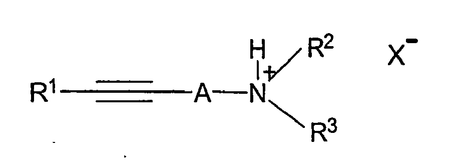 | | | | | |

| Verb Nr | R¹ | A | 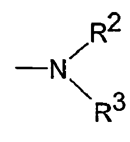 | X | Physiko-chemische Daten |
|---|---|---|---|---|---|
| 1000 | T⁶ | CH₂ | L⁵⁰ | Cl | Schmp 183-184°C |
| 1001 | T³⁹ | CH₂ | L²⁷ | Cl | Schmp 218°C |
| 1002 | T⁴⁶ | CH₂ | L⁵⁰ | ½ SO₄ | Schmp 139-141°C |
| 1003 | T³⁹ | CH₂ | L⁵⁴ | Cl | Schmp 205°C |
| 1004 | T⁴⁶ | CH₂ | L⁵⁰ | 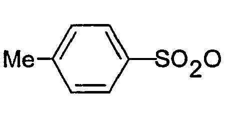 | Schmp 172-173°C |
| 1005 | T⁴⁶ | CH₂ | L⁵⁰ | CF₃COO | Schmp 42-44°C |
| 1006 | T⁴⁶ | CH₂ | L⁵⁰ | Cl | Schmp 178-183°C |
| 1007 | T⁴⁶ | CH₂ | L⁵⁰ | ½ OOC-COO | Schmp 165°C |
| 1008 | T⁴⁶ | CH₂ | L⁵⁰ | 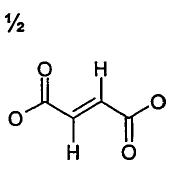 | Schmp 95°C |
| 1009 | T⁴⁶ | CH₂ | L⁵⁰ | 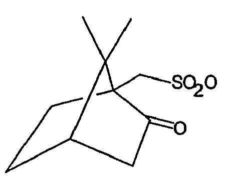 | Schmp 157°C |
| 1010 | T⁹ | CH₂ | L⁵⁰ | Cl | Schmp 185-186°C |
| 1011 | T¹⁰ | CH₂ | L⁵⁰ | Cl | Schmp 213-214°C |
| 1012 | T² | CH₂ | L⁵⁰ | Cl | Schmp 164-165°C |
| 1013 | T¹⁰⁹ | CH₂ | L⁵⁰ | Cl | Schmp 138-139°C |
| 1014 | T¹¹⁰ | CH₂ | L⁵⁰ | Cl | Schmp 200-202°C |
| 1015 | T¹¹³ | CH₂ | L⁵⁰ | Cl | Schmp 162°C |
| 1016 | T¹¹⁴ | CH₂ | L⁵⁰ | Cl | Schmp 185°C |
| 1017 | T¹¹⁵ | CH₂ | L⁵⁰ | Cl | Schmp 139-140°C |
| 1018 | T³⁹ | CH₂ | L³⁰ | Cl | Schmp 168-172°C |
| 1019 | T³⁹ | CH₂ | L⁷⁷ | Cl | Schmp 230-232°C |
| 1020 | T¹²⁵ | CH₂ | L⁵⁰ | Cl | Schmp 188°C |
| 1021 | T³⁹ | CH₂ | L⁷⁹ | Cl | Schmp 213-215°C |
| 1022 | T¹²⁷ | CH₂ | L⁵⁰ | Cl | Schmp 200-202°C |
| 1023 | T²⁰ | CH₂ | L⁵⁰ | Cl | Schmp 233-234°C |
| 1024 | T³⁹ | CH₂ | L⁴⁴ | Cl | Schmp 178-179°C |
| 1025 | T³⁹ | CH₂ | L⁸¹ | Cl | Schmp 195°C |
| 1026 | T³⁹ | CH₂ | L⁸² | Cl | Schmp 65-110°C |
| 1027 | T³⁹ | CH₂ | L⁸³ | Cl | Schmp 50-79°C |
| 1028 | T³⁹ | CH₂ | L⁸⁴ | Cl | Schmp 214-216°C |
| 1029 | T³⁹ | CH₂ | L⁸⁵ | Cl | Schmp 220-221°C |
| 1030 | T³⁹ | CH₂ | L⁸⁷ | Cl | Schmp 192-193°C |
| 1031 | T³⁹ | CH₂ | L¹³ | Cl | Schmp 210-212°C |
| 1032 | T¹³³ | CH₂ | L⁵⁰ | Cl₂ | Schmp 270°C |
| 1033 | T³⁹ | CH₂ | L⁸⁸ | Cl₂ | Schmp 282-283°C |
| 1034 | T³⁹ | CH₂ | L⁸⁹ | Cl | Schmp 202°C |
| 1035 | T¹⁷ | CH₂ | L⁵⁰ | Cl | Schmp 225°C |
| 1036 | T⁴⁶ | CH₂ | L⁵⁰ | Cl | Schmp 207-209°C |
| 1037 | T³⁹ | CHMe | L⁵⁰ | Cl | Schmp 205-210°C |
| 1038 | T⁷¹ | CH₂ | L⁵⁰ | Cl | Schmp 201-204°C |
| 1039 | T³⁹ | C(Me)₂ | L⁵⁰ | Cl | Schmp 208-210°C |
| 1040 | T⁴⁶ | CH₂ | L⁵⁰ | C₈H₁₇COO | ¹H-NMR (CDCl₃): δ = 0,85 (t,3H), 0,97 (d,3H), 3,58 (s,2H), 7,39 (d,1H), 7,56 (s,1H), 7,61 (d,1H) |
| 1041 | T³⁹ | CH₂ | L³ | Cl | Schmp 206-208°C |
| 1042 | T³³ | CH₂ | L⁵⁰ | Cl | Schmp 198-200°C |
| 1043 | T⁴⁴ | CH₂ | L⁵⁰ | Cl | Schmp 222-224°C |
| 1044 | T³⁹ | CH₂ | L²² | Cl | Schmp 187-189°C |
| 1045 | T²³³ | CH₂ | L⁵⁰ | C₈H₁₇COO | ¹H-NMR (CDCl₃): δ = 0,85 (t,3H), 0,97 (d,3H), 3,60 (s,2H), 7,78 (s,1H), 7,81 (s,1H) |
| 1046 | T²³³ | CH₂ | L⁵⁰ | CF₃COO | ¹H-NMR (CDCl₃): δ=1,05(d,3H), 4,20 (s,2H), 8,89 (s,1H), 8,91 (s,1H) |
| 1047 | T²³³ | CH₂ | L⁵⁰ | Cl | Schmp 203-206°C |
| 1048 | T²⁰² | CH₂ | L⁵⁰ | Cl | Schmp 172°C |
| 1049 | T²³⁶ | CH₂ | L⁵⁰ | Cl | Schmp 150°C |
| 1050 | T²³³ | CH₂ | L⁹¹ | Cl | Schmp 151°C |
| 1051 | T³⁹ | CH₂ | L⁵⁰ | CF₃COO | Schmp 188°C |
| 1052 | T³⁹ | CH₂ | L⁵⁰ | Cl | Schmp 212°C |
| 1053 | T³⁹ | CH₂ | L⁵⁰ | ½ SO₄ | Schmp 188°C |
| 1054 | T³⁹ | CH₂ | L⁵⁰ | 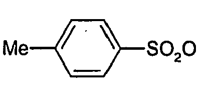 | Schmp 176°C |
| 1055 | T³⁹ | CH₂ | L⁵⁰ | ½ OOC-COO | Schmp 186°C |
| 1056 | T³⁹ | CH₂ | L⁵⁰ | 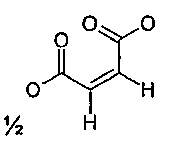 | Schmp 128°C |
| 1057 | T²⁰³ | CH₂ | L⁵⁰ | Cl | Schmp 183°C |
| 1058 | T⁵¹⁷ | CH₂ | L⁵⁰ | Cl | Schmp 192°C |
| 1059 | T⁴⁶ | CH₂ | L⁵⁰ | EtSO₂O | Schmp 132°C |
| 1060 | T²³³ | CH₂ | L⁵⁰ | EtSO₂O | Schmp 104°C |
| 1061 | T¹⁶⁹ | CH₂ | L⁵⁰ | Cl | Schmp 176°C |
| 1062 | T¹⁶⁹ | CH₂ | L⁵⁰ | 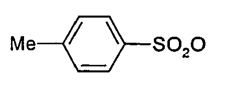 | Schmp 161°C |
| 1063 | T³⁹ | CH₂ | L⁵⁰ | EtSO₂O | Öl |
| 1064 | T²³⁶ | CH₂ | L⁵⁰ | EtSO₂O | Viskoses Öl |
| 1065 | T¹⁶⁹ | CH₂ | L⁵⁰ | CF₃COO | Schmp 78°C |
| 1066 | T¹⁶⁹ | CH₂ | L⁵⁰ | 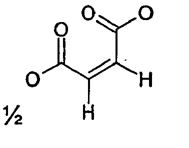 | Schmp 100°C |
| 1067 | T¹⁶⁹ | CH₂ | L⁵⁰ | EtSO₂O | Viskoses Öl |
| 1068 | T⁵¹⁸ | CH₂ | L⁵⁰ | Cl | Schaum |
| 1069 | T¹⁸⁴ | CH₂ | L⁵⁰ | Cl | Schmp 198°C |
| 1070 | T⁵²⁰ | CH₂ | L⁵⁰ | Cl | Schmp 168-170°C |

### B. Formulierungsbeispiele

### Beispiel A

Ein Stäubemittel wird erhalten, indem man 10 Gew.-Teile (Gewichtsteile) Wirkstoff und 90 Gew.-Teile Talkum als Inertstoff mischt und in einer Schlagmühle zerkleinert.

### Beispiel B

Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

### Beispiel C

Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.

### Beispiel D

Ein emulgierbares Konzentrat läßt sich herstellen aus 15 Gew.-Teilen Wirkstoff, 75 Gew.-Teilen Cyclohexan als Lösungsmittel und 10 Gew.-Teilen oxethyliertem Nonylphenol (10 EO) als Emulgator.

### Beispiel E

Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel B mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### C. Biologische Beispiele

In den folgenden Beispielen A bis M, P bis Z, AA bis Al wurden Verbindungen als aktiv angesehen, wenn sie bei einer Konzentration von 500 ppm oder weniger eine Wirkung auf die Schadorganismen von 50% oder mehr aufweisen.

### Beispiel A

Die Blätter von 12 Reispflanzen mit einer Halmlänge von 8 cm wurden für 5 Sekunden in eine wäßrige Lösung des zu prüfenden und formulierten Präparates getaucht. Nach dem Abtropfen wurden die so behandelten Reispflanzen in eine Petrischale gelegt und mit ca. 20 Larven (L3-Stadium) der Reiszikadenart Nilaparvata lugens besetzt. Nach dem Verschließen der Petrischale wurde diese in einer Klimakammer gelagert (16 Stunden Licht/Tag, 25 °C, 40-60 % RF). Nach 6 Tagen Lagerung wurde die Mortalität der Zikadenlarven bestimmt. Die Verbindungen gemäß folgender Beispiele waren aktiv: Nr. 29, 32, 42, 43, 51, 70, 84, 1002, 1004, 1005, 1040, 180, 184, 193, 197, 203, 209, 1059, 264, 265, 1066.

### Beispiel B

Eine Petrischale, deren Boden mit Filterpapier belegt war und ca. 5 ml Nährmedium enthielt, wurde vorbereitet. Filterpapierstücke mit ca. 30, 24 Stunden alten Eiern der amerikanischen Tabakknospeneule (Heliothis virescens) wurden für 5 Sekunden in einer wäßrigen Lösung des zu prüfenden und formulierten Präparates getaucht und anschließend in der Petrischale ausgelegt. Weitere 200 µl der wäßrigen Lösung wurden über das Nährmedium verteilt. Nach dem Verschließen der Petrischale wurde diese bei ca. 25°C in einer Klimakammer gelagert. Nach 6 Tagen Lagerung wurde die Wirkung des Präparates auf die Eier und die evtl. hieraus geschlüpften Larven festgestellt (Mortalität). Die Verbindungen gemäß folgender Beispiele waren aktiv: Nr. 14, 15, 17, 18, 29, 30, 32, 60, 70, 97, 100, 119, 124, 1000, 1002, 1004, 1005, 1011, 1038, 1040, 1042, 1044, 1045, 1046, 1047, 1036, 167, 171, 172, 173, 174, 175, 181, 176, 177, 178, 179, 180, 182, 183, 188, 189, 190, 191, 192, 193, 194, 195, 197, 201, 1048, 203, 204, 24, 207, 209, 210, 214, 215, 216, 217, 221, 222, 224, 225, 226, 229, 230, 152, 1051, 1052, 1054, 1055, 1056, 232, 234, 1057, 1059, 1060, 1061, 1062, 1063, 236, 239, 240, 241, 244, 245, 246, 247, 251, 252, 253, 254, 256, 263, 266, 1065, 1067, 270, 271, 272, 273, 283, 284, 285, 286, 289, 290, 291, 292, 295,296,300,301,302.

### Beispiel C

Eine Petrischale, deren Boden zur Hälfte mit Filterpapier belegt war und ein angekeimtes Maiskorn auf einem feuchten Wattetupfer enthielt, wurde vorbereitet. Auf das Filterpapier wurden ca. 50, 4-5 Tage alte Eier des Maiswurzelwurms (Diabrotica undecimpunctata) übertragen. Drei Tropfen von 200 µl einer wäßrigen Lösung des zu prüfenden und formulierten Präparates wurden auf die Eier und der Rest auf das Maiskorn pipettiert. Nach dem Verschließen der Petrischale wurde diese bei ca. 25 °C in einer Klimakammer gelagert. Nach 6 Tagen Lagerung wurde die Wirkung des Präparates auf die Eier und die evtl. hieraus geschlüpften Larven festgestellt (Mortalität). Die Verbindungen gemäß folgender Beispiele waren aktiv: Nr. 17, 18, 31, 32, 43, 58, 60, 70, 152, 1001, 1002, 1004, 1005, 1038, 1040, 1042, 1047, 1036, 185, 189, 190, 191, 193, 194, 196, 197, 201, 203, 1050, 204, 205, 298, 207, 209, 228, 152, 231, 1051, 1052, 1053, 1054, 1055, 1056, 234, 1057, 1058, 1059, 1060, 1061, 1062, 235, 1063, 1064, 1065, 1066, 1067, 1068, 1069, 267, 281, 282, 290, 291, 295, 297.

### Beispiel D

Äpfel wurden in einer wäßrigen Lösung des zu prüfenden und formulierten Präparates getaucht. Anschließend wurden die Äpfel mit 10 L1-Larven des Apfelwicklers (Carpocapsa pomonella) besetzt. Nach 14 Tagen Lagerung bei ca. 25 °C wurde die Wirkung des Präparates auf die Larven festgestellt (Mortalität). Die Verbindungen gemäß folgender Beispiele waren aktiv: Nr. 14, 15, 17, 18, 70, 1007, 1008, 1009, 1036, 1038, 197.

### Beispiel E

Blätter von Baumwollpflanzen wurden in eine Petrischale gelegt, mit 10 L2-Larven des ägyptischen Baumwollwurms (Spodoptera littoralis) besetzt und mit einer wäßrigen Lösung des zu prüfenden und formulierten Präparates besprüht. Nach 4 Tagen Lagerung bei ca. 23 °C wurde die Wirkung des Präparates auf die Larven festgestellt (Mortalität). Die Verbindungen gemäß folgender Beispiele waren aktiv: Nr. 18, 70, 1007, 1008, 1009, 1036 und 1038.

### Beispiel F

Angekeimte Ackerbohnen-Samen (Vicia faba) mit Keimwurzeln wurden in mit Leitungswasser gefüllte Braunglasfläschen übertragen und anschließend mit ca. 100 schwarzen Bohnenblattläusen (Aphis fabae) belegt. Pflanzen und Blattläuse wurden dann für 5 Sekunden in eine wäßrige Lösung des zu prüfenden und formulierten Präparates getaucht. Nach dem Abtropfen wurden Pflanze und Tiere in einer Klimakammer gelagert (16 Stunden Licht/Tag, 25 °C, 40-60 % RF). Nach 3 und 6 Tagen Lagerung wurde die Wirkung des Präparates auf die Blattläuse festgestellt (Mortalität). Die Verbindungen gemäß folgender Beispiele waren aktiv: 30, 43, 228, 262,291.

### Beispiel G

Eine Petrischale, deren Boden mit Filterpapier belegt war und ca. 5 ml Nährmedium enthielt, wurde vorbereitet. Fünf L2-Larven des ägyptischen Baumwollwurms (Spodoptera littoralis) wurden in einen kleinen Becher eingezählt. 200 µl einer wäßrigen Lösung des zu prüfenden und formulierten Präparates wurde in den Becher pipettiert. Danach wurden die behandelten Larven in die Petrischale ausgegossen und weitere 200 µl der wäßrigen Lösung wurden über das Nährmedium verteilt. Nach dem Verschließen der Petrischale wurde diese bei ca. 25 °C in einer Klimakammer gelagert. Nach 6 Tagen Lagerung wurde die Wirkung des Präparates auf die Larven festgestellt (Mortalität). Die Verbindungen gemäß folgender Beispiele waren aktiv: 29, 70, 60, 159, 1005, 1040, 1042, 1044, 1045, 1046, 1047.

### Beispiel H

In einem Glasgefäß wurde zu ca. 3000 frischgeschlüpften, aktiven (mobilen) Larven (2. Entwicklungsstadium) des Wurzelgallennematoden (Meloidogyne incognita) eine wäßrige Lösung des zu prüfenden und formulierten Präparates zugesetzt (Endvolumen 20 ml). Nach 6-tägiger Dauerexposition der Nematodenlarven wurde der prozentuale Anteil der durch die Einwirkung des Präparates bewegungslos gewordenen Individuen im Vergleich zu den unbehandelten Kontrollen bestimmt (Kontaktwirkung). Die Verbindungen gemäß folgender Beispiele waren aktiv: 39, 52, 159, 161, 162, 163, 164, 165, 1045, 1046, 1047, 179, 183, 187, 1048, 298, 206, 208, 227, 230, 1061, 237, 238, 245, 250, 255, 256, 260, 268, 269, 274, 275, 288, 290.

### Beispiel I

Zehn L1-Larven des Apfelwicklers (Carpocapsa pomonella) wurden in eine mit Nährmedium gefüllte Petrischale gesetzt. Nährmedium und eingesetzte Larven wurden dann mit einer wäßrigen Lösung des zu prüfenden und formulierten Präparates besprüht. Anschließend wurde die Petrischale mit einem Deckel verschlossen. Nach 8 Tagen Lagerung bei ca. 23 °C wurde die Wirkung des Präparates auf die Larven festgestellt (Mortalität). Die Verbindungen gemäß folgender Beispiele waren aktiv: 60, 70, 1002, 1005.

### Beispiel J

Ca. 20 Eier des Apfelwicklers (Carpocapsa pomonella) wurden in eine mit Nährmedium gefüllte Petrischale gelegt. Nährmedium und Eier wurden dann mit einer wäßrigen Lösung des zu prüfenden und formulierten Präparates besprüht. Anschließend wurde die Petrischale mit einem Deckel verschlossen. Nach 8 Tagen Lagerung bei ca. 23 °C wurde die Wirkung des Präparates auf die Eier und die evtl. hieraus geschlüpften Larven festgestellt (Mortalität). Die Verbindungen gemäß folgender Beispiele waren aktiv: 70 und 1036.

### Beispiel K

Ein Weißkohlblatt wurde mit einer wäßrigen Lösung des zu prüfenden und formulierten Präparates besprüht. Nach dem Abtrocknen wurde das behandelte Blatt mit Larven der Kohlschabe (Plutella maculipennis) besetzt. Nach 3 Tagen Lagerung bei ca. 23 °C wurde die Wirkung des Präparates auf die Larven festgestellt (Mortalität). Die Verbindungen gemäß folgender Beispiele waren aktiv: 60, 70, 1002, 1004, 1005, 1036, 197.

### Beispiel L

Kartoffelblätter wurden mit Larven des Kartoffelkäfers (Leptinotarsa decemlineata) besetzt. Blätter und Larven wurden dann mit einer wäßrigen Lösung des zu prüfenden und formulierten Präparates besprüht. Nach 4 Tagen Lagerung bei ca. 25 °C wurde die Wirkung des Präparates auf die Larven festgestellt (Mortalität). Die Verbindungen gemäß folgender Beispiele waren aktiv: 60, 70, 1002, 1004, 1005, 1036, 1047, 197.

### Beispiel M

Das formulierte Präparat wurde mit defibriniertem Rinderblut vermischt. 10 adulte Katzenflöhe (Ctenocephalides felis) wurden mit diesem Blut-Präparategemisch gefüttert. Nach 48 Stunden bei ca. 38 °C wurde die Wirkung des Präparates auf die Flöhe festgestellt (Mortalität). Die Verbindungen gemäß folgender Beispiele waren aktiv: 70, 1002, 1004, 1005, 1036, 60, 197, 1057.

### Beispiel N

### Test 1

Rübenblattscheiben (Durchmesser 49 mm) wurden auf 20%igen Agar in Plastik-Petri-Schalen (Durchmesser 9cm) gelegt. Jede Schale wurde mit 10 Adulten von Phaedon cochleariae, die an Rüben vermehrt wurden, besetzt. Die zu prüfende Verbindung wurde in einer 50 (Volumen-)%igen wäßrigen Aceton-Lösung angesetzt. Die Präparatelösung wurde dann mittels eines sogenannten 'Potter Towers' auf die infizierten Blattscheiben gespritzt, mit einer Aufwandmenge von 660 Litern pro Hektar. Pro Versuchsglied wurden 4 Wiederholungen durchgeführt. Bei den Kontrollen wurden die infizierten Blattscheiben entweder nicht oder allein mit der 50 %igen wässrigen Aceton-Lösung besprüht.

Nach 48 h wurde die Mortalität bestimmt und unter Verwendung von Abbotts Formel mit der Mortalität der Kontrollen verglichen. Anschließend wurde die LD₉₀₋Konzentration (Dosierung ab der 90% Mortalität auftritt, Angabe hier in Gew.-%) berechnet. Die Verbindungen gemäß Beispiel 14, 18, 29, 33, 152, 1001 und 1039 zeigten eine LD₉₀ bei einer Dosierung von 0,05% oder weniger.

### Test 2

Anstelle von Phaedon cochleariae wurden 5 Pieris brassicae-Larven des 2. Entwicklungsstadiums verwendet. Die Testdurchführung entsprach der in Test 1 beschriebenen Vorgehensweise. Die Verbindungen gemäß Beispiel 14, 29, 33, 100, 152, 1005 und 1039 zeigten eine LD₉₀ bei einer Dosierung von 0,006% oder weniger.

### Test 3

Anstelle von Pieris brassicae wurden 10 Plutella xylostella-Larven des 2. Entwicklungsstadiums verwendet. Die Testdurchführung entsprach der in Test 2 beschriebenen Vorgehensweise. Die Verbindungen gemäß Beispiel 4, 6, 7, 10, 12, 13, 14, 15, 16, 17, 18, 19, 20, 23, 24, 25, 26, 28, 29, 31, 32, 33, 34, 35, 152, 1000, 1001, 1005 und 1039 zeigten eine LD₉₀ bei einer Dosierung von 0,05% oder weniger.

### Test 4

Die zu prüfende Verbindung wurde in reinem Aceton angelöst und in Erde mit einer Feuchte von ca. 10% eingemischt. Nach dem Abdampfen des Lösungsmittels wurden jeweils 5 g behandelte Erde in eine Röhre gefüllt und mit 0,5 ml Wasser versetzt. 10 Gryllus bimaculatus -Nymphen (1. Entwicklungsstadium) und eine Grünkohlscheibe, als Nahrungsquelle, wurden in jede Röhre eingesetzt. Pro Versuchsglied wurden 4 Wiederholungen durchgeführt. Die Kontrollen bestanden aus unbehandelter und mit Aceton allein behandelter Erde. Die belüfteten Röhren wurden bei 25 °C für 48 h kultiviert bevor die Bestimmung der Mortalität erfolgte.

Nach Berücksichtigung der Kontrollmortalität unter Verwendung der Abbott-Formel, wurde die LC₉₀ als ppm-Konzentration in der Erde bestimmt. Die Verbindungen gemäß Beispiel 152 und 1039 zeigten eine LC₉₀ bei einer Konzentration von 50 ppm oder weniger.

### Test 5

Die zu prüfende Verbindung wurde in reinem Aceton angelöst und eine kleine Menge dieser Lösung wurde auf die Oberfläche einer Milch-Hefe-Agar-Diät (5 ml) in einem Probegläschen (7 cm x 2,5 cm) gegeben. Nach 24 h wurden ca. 10 Lucilia sericata-Larven des 1. Entwicklungsstadiums auf die behandelte Diät gesetzt. Pro Versuchsglied wurden 3 Wiederholungen durchgeführt. Die Diät in den Röhren der Kontrolle wurde mit einer äquivalenten Menge Aceton allein behandelt. Die 'Minimale Effektive Dosis' (MED) wurde definiert als die niedrigste Dosierung bei der die Weiterentwicklung der Larven verhindert wird und es so zu keinem anschließenden Schlupf adulter Fliegen kommt.

Die Verbindungen gemäß Beispiel 4 und 1039 zeigten eine MED bei 100 µg/Gläschen oder weniger.

### Test 6

Die zu prüfende Verbindung wurde in einer 50 (Volumen-)%igen wäßrigen Aceton-Lösung angesetzt. Zwei Wochen alte Boophilus microplus-Larven, "sandwich"-artig umgeben von 2 Scheiben Filterpapier, wurden für 4 Minuten in die Präparatelösung getaucht. Pro Versuchsglied wurden 2 Wiederholungen durchgeführt. Die entsprechenden Kontrollen wurden mit der 50 %igen wässrigen Aceton-Lösung allein behandelt. Anschließend wurden die Larven auf neue Filterpapierscheiben umgesetzt und bei ca. 25 °C und 95% rel. Feuchte gelagert. Die'Minimale Effektive Konzentration' (MEK) wurde definiert als die niedrigste Konzentration, ausgedrückt als Gew.-% der zu prüfenden Verbindung in der Lösung, bei der 100% Mortalität innerhalb von 48 h auftrat. Die Verbindungen gemäß Beispiel 2, 4, 6, 9, 20, 22, 25, 27, 28 und 32 hatten eine MEK von 0,04% oder weniger.

### Test 7

Die zu prüfende Verbindung wurde in einer 50 (Volumen-)%igen wäßrigen Aceton-Lösung angesetzt. Vollständig vollgesaugte Weibchen der Rinderzecke (Boophilus microplus), die auf natürliche Weise von künstlich infizierten Kälbern abgefallen waren, wurden gewaschen und getrocknet. Anschließend wurden sie für 1 Minute unter ständigem Rühren in die Präparatelösung getaucht. Nach dem Abtropfen der Lösung wurden die Weibchen in Probegläschen (7 cm x 2,5 cm) überführt und bei 30 °C und 94% rel. Feuchte in einem Inkubator gelagert. Jedes Versuchsglied bestand aus 2 x 10 Zecken-Weibchen. Die Kontrollgruppe wurde mit der 50 %igen wäßrigen Aceton-Lösung allein behandelt. Sechs Tage nach Behandlung wurden Mortalität und Eiablage bewertet. Die'Minimale Effektive Konzentration' (MEK) wurde definiert als die niedrigste Konzentration, ausgedrückt als Gew.-% der zu prüfenden Verbindung in der Lösung, bei der keine lebenden Eier mehr produziert werden konnten. Die Verbindungen gemäß Beispiel 20, 27 und 32 hatten eine MEK von weniger als 0,2%.

### Beispiel O

### Test 1

Die zu prüfende Verbindung wurde in reinem Aceton angelöst und mit einer Standard-Schabenköder-Mischung, bestehend überwiegend aus Wasser, Maissirup und Glycerin, sowie in geringeren Anteilen aus Geflügelleber und Stärke mit geringen Zusätzen von Hydroxymethylcellulose, Propyl- und Methylparahydroxybenzoesäure sowie eines Duftstoffes, versetzt. Nach dem Abdampfen des Acetons wurden 0,1 g des Köders in eine Kunststoffbox gegeben und anschließend 20 adulte Blattella germanica (jeweils 10 Männchen und 10 Weibchen) eingesetzt (sog. 'no choice'-Test). Nach 7 Tagen wurde die Wirkung bestimmt. Die Verbindungen gemäß Beispiel 70 und 1036 bewirkten mit 0,5 Gew.-% im Köder eine Mortalität von 45% bzw. 100%. Bei 1,0 Gew.-% im Köder bewirkten die Verbindungen gemäß Beispiel 70 und 1036 zusätzlich einen Repellent-Effekt.

### Test 2

Die zu prüfende Verbindung wurde in reinem Aceton angelöst und mit einer Standard-Schabenköder-Mischung, bestehend überwiegend aus Wasser, Maissirup und Glycerin, sowie in geringeren Anteilen aus Geflügelleber und Stärke mit geringen Zusätzen von Hydroxymethylcellulose, Propyl- und Methylparahydroxybenzoesäure sowie eines Duftstoffes, versetzt. Nach dem Abdampfen des Acetons wurden 0,1 g des so vorbereiteten Köders und 0,1 g eines identischen Köders ohne Prüfverbindung in eine Kunststoffbox gegeben. Anschließend wurden 20 adulte Blattella germanica (jeweils 10 Männchen und 10 Weibchen) eingesetzt (sog. 'choice'-Test). Nach 7 Tagen wurde die Wirkung bestimmt. Die Verbindungen gemäß Beispiel 70 und 1036 bewirkten mit 0,5 Gew.-% im Köder eine Mortalität von 30% bzw. 60%. Bei 1,0 Gew.-% im Köder bewirkten die Verbindungen gemäß Beispiel 70 und 1036 einen Repellent-Effekt ohne Mortalität, da die Köder mit den Prüfverbindungen nicht gefressen wurden.

### Test 3

Die zu prüfende Verbindung wurde in reinem Aceton angelöst und in geringer Menge in Gefäße mit jeweils 100 ml Wasser einpipettiert. Moskito-Larven des 2. Entwicklungsstadiums von Aedes aegypti, Anopheles arabiensis und Culex quinquefasciatus wurden jeweils in die Gefäße hineinpipettiert. Nach 24 h wurde die Wirkung bestimmt. Die Verbindungen gemäß Beispiel 70 und 1036 bewirkten bei 100 ppm eine Mortalität von 100% bei den Larven von Aedes aegypti, Anopheles arabiensis und Culex quinquefasciatus.

### Test 4

Jeweils 50 mg der zu prüfenden Verbindung wurden in reinem Aceton angelöst und auf einen wirkstoff-freien Verdampfungs-Filz (syn. 'Emanator Mat'; ® Bengal Mosquito Mats, Zobele Industrie Chimiche S.p.A., Via Fersina, Trento, Italy) pipettiert. Nach dem Abdampfen des Acetons wurde der so vorbereitete Verdampfungs-Filz in ein elektrisches Heizgerät (® Bengal Heater, Zobele Industrie Chimiche S.p.A., Via Fersina, Trento, Italy) eingesetzt. Nach Erhitzung des Filzes auf 150 °C verflüchtigte sich die zu prüfende Verbindung in einer Testkammer mit einem Volumen von 2 m³.

Die Wirkung der Verbindung wurde jeweils an 100 adulten Moskito-Weibchen von Aedes aegypti und von Culex quinquefasciatus mit 3 Wiederholungen geprüft. Die Auswertung nach Immobilität (syn.'knockdown') erfolgte in Minutenabständen und wurde nach insgesamt 20 Minuten beendet. Die Verbindungen gemäß Beispiel 70 und 1036 bewirkten bei 50% der Weibchen von Aedes aegypti einen Knockdown (syn. KT₅₀; 'knockdown time of 50% of individuals', d.h. Zeitspanne nach der 50 % der Individuen immobilisiert wurden) nach 12 bzw. 11,5 Minuten. Bei Culex quinquefasciatus betrug die KT₅₀ 8 bzw. 9,25 Minuten.

### Test 5

Jeweils 50 mg der zu prüfenden Verbindung wurden in reinem Aceton angelöst und auf einen wirkstoff-freien Verdampfungs-Filz (syn. 'Emanator Mat; ® Bengal Mosquito Mats, Zobele Industrie Chimiche S.p.A., Via Fersina, Trento, Italy) pipettiert. Nach dem Abdampfen des Acetons wurde der so vorbereitete Verdampfungs-Filz einmal für 20 Minuten verwendet und anschließend für 2 Wochen gelagert. Im Abschluß daran wurde der vorbereitete Verdampfungs-Filz nochmals überprüft. Die Testdurchführung entsprach der in Test 4 beschriebenen Vorgehensweise. Die Verbindungen gemäß Beispiel 70 und 1036 bewirkten bei Aedes aegypti eine KT₅₀; von 11 bzw. 10,5 Minuten. Bei Culex quinquefasciatus betrug die KT₅₀ 7,5 bzw 9 Minuten.

### Beispiel P

Ein Weißkohlblatt wurde für ca. 5 Sekunden in eine wäßrige Lösung des zu prüfenden und formulierten Präparates getaucht. Nach dem Trocknen wurde das so behandelte Kohlblatt in einen Behälter gegeben und mit 10 Larven der Kohlschabe (Plutella maculipennis) besetzt. Anschließend wurde der Behälter mit einem Deckel verschlossen. Nach 3 Tagen Lagerung bei ca. 23 °C wurde die Wirkung des Präparates auf die Larven festgestellt. Die folgenden Beispiele waren aktiv: 1036, 70, 60, 209.

### Beispiel Q

Abgeschnittene Stengel mit einem Blatt von Bohnenpflanzen (Phaseolus vulgaris) wurden in mit Leitungswasser gefüllte Braunglasfläschen übertragen und anschließend mit ca. 100 Spinnmilben (Tetranychus urticae) belegt. Pflanzenblatt und Spinnmilben wurden dann für 5 Sekunden in eine wäßrige Lösung des zu prüfenden und formulierten Präparates getaucht. Nach dem Abtropfen wurden Pflanze und Tiere in einer Klimakammer gelagert (16 Stunden Licht/Tag, 25 °C, 40-60 % RF). Nach 6 Tagen Lagerung wurd die Mortalität des Präparates auf alle Stadien der Spinnmilben festgestellt. Die folgenden Beispiele waren aktiv: 299, 305, 206,268,269,287.

### Beispiel R

Angekeimte Ackerbohnen-Samen (Vicia faba) mit Keimwurzeln wurden in mit Leitungswasser gefüllte Braunglasfläschen übertragen. Vier Milliliter einer wäßrigen Lösung des zu prüfenden und formulierten Präparates wurden in das Braunglasfläschen hineinpipettiert. Anschließend wurde die Ackerbohne mit ca. 100 schwarzen Bohnenblattläusen (Aphis fabae) stark belegt. Pflanze und Tiere wurden dann in einer Klimakammer gelagert (16 Stunden Licht/Tag, 25 °C, 40-60 % RF). Nach 3 und 6 Tagen Lagerung wurde die wurzelsystemische Wirkung des Präparates auf die Blattläuse festgestellt (Mortalität). Die folgenden Beispiele waren aktiv: 184, 186, 305, 220, 265.

### Beispiel S

Eine Petrischale, deren Boden mit Filterpapier belegt war und ca. 5 ml Nährmedium enthält, wurde vorbereitet. Fünf L2-Larven der Zuckerrübeneule (Spodoptera exigua) wurden in einen kleinen Becher eingezählt. 200 µl einer wäßrigen Lösung des zu prüfenden und formulierten Präparates wurde in den Becher pipettiert. Danach wurden die gehandelten Larven in die Petrischale ausgegossen und weitere 200 µl der wäßrigen Lösung wurden über das Nährmedium verteilt. Nach dem Verschließen der Petrischale wurde diese bei ca. 25 °C in einer Klimakammer gelagert. Nach 6 Tagen Lagerung wurde die Mortalität der Larven festgestellt. Die folgenden Beispiele waren aktiv: 1036, 175, 184, 1051, 1054, 1055, 234, 1057, 1059, 1060, 1063, 243, 245, 251, 257, 259, 266, 1065, 1067, 290, 291.

### Beispiel T

10 Larven der Wintersaateule (Agrotis segetum) wurden in eine mit Nährmedium gefüllte Petrischale gesetzt. Nährmedium und eingesetzte Larven wurden dann mit einer wäßrigen Lösung des zu prüfenden und formulierten Präparates besprüht. Anschließend wurde die Petrischale mit einem Deckel verschlossen. Nach 7 Tagen Lagerung bei ca. 23 °C wurde die Mortalität der Larven festgestellt. Das folgende Beispiel war aktiv: 70.

### Beispiel U

Reissaatgut wurde auf feuchter Watte in Zuchtgläsern angekeimt. Nach dem Heranwachsen auf ca. 8 cm Halmlänge wurden die Blätter mit einer wäßrigen Lösung des zu prüfenden und formulierten Präparates bis zum beginnenden Abtropfen besprüht. Nach dem Abtropfen wurden die so behandelten Reispflanzen in Zuchtbehälter gegeben und mit je 10 Larven (L3-Stadium) der Reiszikadenart Nilaparvata lugens besetzt. Pflanzen und Tiere wurden in einer Klimakammer gelagert (16 Stunden Licht/Tag, 25 °C, 40-60 % RF). Nach 4 Tagen Lagerung wurde die Mortalität der Larven bestimmt. Das folgende Beispiel war aktiv: 70.

### Beispiel V

Nährmedium (gefriergetrockneter Würfel) wurde in eine wäßrige Lösung des zu prüfenden und formulierten Präparates getaucht und anschließend in eine Petrischale gelegt. Danach wurden 10 L2-Larven der amerikanischen Tabakknospeneule (Heliothis virescens) eingesetzt. Anschließend wurde die Petrischale mit einem Deckel verschlossen. Nach 4 Tagen Lagerung bei ca. 23 °C wurd die Mortalität der Larven festgestellt. Die folgenden Beispiele waren aktiv: 1036, 60.

### Beispiel W

2,4 ml einer wäßrigen Lösung des zu prüfenden und formulierten Präparates wurden in ein mit 21,6 ml Wasser gefülltes Fläschen pipettiert. Eine 14 Tage alte Kohlpflanze wurde in das Fläschen übertragen. Nach einer Woche wurde die Pflanze mit 5 Larven der Kohlschabe (Plutella maculipennis) besetzt. Pflanze und Tiere wurden dann in einer Klimakammer gelagert (23 °C, 40-60 % RF). Nach 3 Tagen Lagerung wurde die wurzelsystemische Wirkung des Präparates auf die Larven festgestellt (Mortalität). Die folgenden Beispiele waren aktiv: 1036, 70, 60.

### Beispiel X

Nährmedium (gefriergetrockneter Würfel) wurde in eine wäßrige Lösung des zu prüfenden und formulierten Präparates getaucht und anschließend in eine Petrischale gelegt. Danach wurden 10 L2-Larven der Zuckerrübeneule (Spodoptera exigua) eingesetzt. Anschließend wurde die Petrischale mit einem Deckel verschlossen. Nach 4 Tagen Lagerung bei ca. 23 °C wurde die Mortalität der Larven festgestellt. Die folgenden Beispiele waren aktiv: 1036, 60.

### Beispiel Y

Baumwollpflanzen wurden mit einer wäßrigen Lösung des zu prüfenden und formulierten Präparates besprüht. Nach dem Abtrocknen wurden Blätter abgeschnitten, in eine Petrischale gelegt und mit 5 L2-Larven der Zuckerrübeneule (Spodoptera exigua) besetzt. Nach 4 Tagen Lagerung bei ca. 23 °C wurde die Mortalität der Larven festgestellt. Die folgenden Beispiele waren aktiv: 1036, 70, 60, 1047, 197.

### Beispiel Z

Baumwollblätter wurden mit 5 L2-Larven der Zuckerrübeneule (Spodoptera exigua) besetzt und anschließend mit einer wäßrigen Lösung des zu prüfenden und formulierten Präparates besprüht. Nach 4 Tagen Lagerung bei ca. 23 °C wurde die Mortalität der Larven festgestellt. Die folgenden Beispiele waren aktiv: 1036, 70, 60, 1047, 197.

### Beispiel AA

Nährmedium wurde mit einer wäßrigen Lösung des zu prüfenden und formulierten Präparates vermischt und mit 10 L1-Larven des Apfelwicklers (Carpocapsa pomonella) besetzt. Nach 14 Tagen Lagerung bei ca. 23 °C wurde die Mortalität der Larven festgestellt. Die folgenden Beispiele waren aktiv: 70, 197.

### Beispiel AB

Baumwollblätter wurden in eine Petrischale gelegt, mit 5 L2-Larven der amerikanischen Tabakknospeneule (Heliothis virescens) besetzt und mit einer wäßrigen Lösung des zu prüfenden und formulierten Präparates besprüht. Nach 4 Tagen Lagerung bei ca. 25 °C wurde die Mortalität der Larven festgestellt. Die folgenden Beispiele waren aktiv: 70, 1047, 197.

### Beispiel AC

Wäßrige Lösung des zu prüfenden und formulierten Präparates wurde mit Erde vermischt. Nach 0d, 21 d und 42d wurden Eier des Maiswurzelwurms (Diabrotica undecimpunctata) zusammen mit 2 angequollenen Maiskörnern in eine Schale gelegt, mit der behandelten Erde bedeckt und mit 10 ml Wasser angegossen. Erde und Eier wurden im Gewächshaus aufgestellt (23 °C, 60 % RF). Nach 2 Wochen Lagerung wurde die Wirkung des Präparates auf die Eier und die evtl. hieraus geschlüpften Larven festgestellt (Mortalität). Die folgenden Beispiele waren aktiv: 70, 60, 197, 204.

### Beispiel AD

Buschbohnen (Phaseolus vulgaris) wurden zur Eiablage für 48 Stunden mit Adulten der weißen Fliege (Trialeurodes vaporariorum) besetzt. Nach dem Schlüpfen der Larven wurden die Pflanzen mit einer wäßrigen Lösung des zu prüfenden und formulierten Präparates bis zum beginnenden Abtropfen besprüht. Nach 11 Tagen wurde die larvizide Wirkung ermittelt. Die folgenden Beispiele waren aktiv: 1036, 70, 60, 1040, 197.

### Beispiel AE

Wäßrige Lösung des zu prüfenden und formulierten Präparates wurd mit Erde vermischt. 2/3 der Erde wurden in einen Topf gefüllt, ein angekeimtes Maiskorn und ca. 30 Larven der Wintersaateule (Agrotis segetum) wurden aufgesetzt und mit der restlichen Erde abgedeckt. Erde und Larven wurden im Gewächshaus aufgestellt (23 °C, 60 % RF). Nach 7 Tagen Lagerung wurde die Mortalität der Larven festgestellt. Das folgende Beispiel war aktiv: 70

### Beispiel AF

Das formulierte Präparat wurde auf Filterpapier pipettiert. Nach dem Verdampfen des Lösungsmittels wurde das Filterpapier mit 20-30 Zeckenlarven (Rhipicephalus sanguineus) besetzt. Nach 24 Stunden bei ca. 25°C wurde die Wirkung des Präparates auf die Zecken festgestellt (Mortalität). Die folgenden Beispiele waren aktiv: 197, 1057.

### Beispiel AG

In jedes Loch einer Mikrotiterplatte wurden 190 µl Nährlösung und ca. 20 Eier der Gelbfiebermücke (Aedes aegypti) gegeben. Nach dem Schlüpfen der Larven wurden 10 µl wäßrige Lösung des zu prüfenden und formulierten Präparates hinzupipettiert. Nach 3 Tagen Lagerung bei 25 °C und 60 % RF wurde die larvizide Wirkung des Präparates festgestellt. Die folgenden Beispiele waren aktiv: 209, 211, 213, 215, 216, 218, 219, 234, 1057, 1059, 1060, 1061, 1062, 235, 1064, 242, 244, 245, 248, 249, 250, 251, 252, 254, 256, 258, 259, 261, 264, 265, 266, 1065, 1066, 1067, 1068, 1069, 267, 289, 290, 291, 292, 293, 294, 301, 303.

### Beispiel AH

Baumwollblätter wurden in eine Petrischale gelegt, mit einer wäßrigen Lösung des zu prüfenden und formulierten Präparates besprüht und nach dem Abtrocknen mit 5 L2-Larven der amerikanischen Tabakknospeneule (Heliothis virescens) besetzt. Nach 2, 3 und 4 Tagen Lagerung bei ca. 25 °C wurde die Antifeeding-Wirkung des Präparates auf die Larven festgestellt. Die folgenden Beispiele waren aktiv: 1036, 70,60.

### Beispiel Al

Eine Kartoffelpflanze wurde mit einer wäßrigen Lösung des zu prüfenden und formulierten Präparates besprüht und zusammen mit einer unbehandelten Pflanze in einen Käfig gestellt. Der Käfig wurde mit 100 Larven des Kartoffelkäfers (Leptinotarsa decemlineata) besetzt. Nach 1 und 3 Tagen Lagerung bei ca. 25 °C wurde die Repellent-Wirkung des Präparates auf die Larven festgestellt. Die folgenden Beispiele waren aktiv: 1036, 70, 60.

## Patentansprüche

1. Ein Verfahren zur Bekämpfung von schädlichen Arthropoden und/oder Helminthen, wobei auf diese oder die von ihnen befallenen Pflanzen oder Tieren, Flächen oder Substrate eine wirksame Menge einer Verbindung gemäß Formel (I), 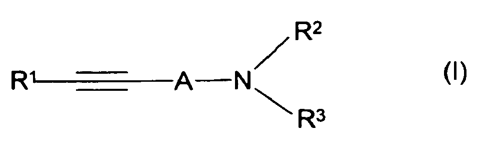 gegebenenfalls auch als N-Oxid und/oder Salz, worin
a) R¹ ein gegebenenfalls ein- oder mehrfach durch gleiche oder verschiedene Reste substituiertes Heteroaryl, Phenyl, Biphenyl oder Phenanthryl bedeutet,
und wenn b1) A eine Gruppe CR⁴R⁵ bedeutet, wobei
R⁴ Wasserstoff, Alkyl bedeutet;
R⁵ Wasserstoff oder einen gegebenenfalls substituierten Kohlenwasserstoffrest, welcher geradkettige, verzweigte oder cyclische, gesättigte, teilgesättigte oder ungesättigte organische Reste umfasst, bedeutet;
dann c1) R² und R³ gemeinsam mit dem Stickstoffatom, an das sie gebunden sind, ein gegebenenfalls ein- oder mehrfach durch gleiche oder verschiedene Reste substituiertes N-Heteroaryl, Aziridinyl, Pyrrolidinyl, Isoxazolidinyl, Isothioazolidinyl, Oxazolidinyl, Thiazolidinyl, Imidazolidinyl, 1,2,4-Oxadiazolidinyl, 1,2,4-Thiadiazolidinyl, 1,2,4-Triazolidin-3-yl, 1,3,4-Thiadiazolidin-2-yl, 1,3,4-Triazolidin-1-yl, 1,3,4-Triazolidin-2-yl, 2,3-Dihydropyrrolyl, 2,5-Dihydropyrrolyl, 2,3-Dihydroisoxazolyl, 4,5-Dihydroisoxazolyl, 2,5-Dihydroisothiazolyl, 2,3-Dihydropyrazolyl, 4,5-Dihydropyrazolyl, 2,5-Dihydropyrazolyl, 2,3-Dihydrooxazolyl, 4,5-Dihydrooxazolyl, 2,5-Dihydrooxazolyl, 2,3-Dihydrothiazolyl, 4,5-Dihydrothiazolyl, 2,5-Dihydrothiazolyl, 2,3-Dihydroimidazolyl, 4,5-Dihydroimidazolyl, 2,5-Dihydroimidazolyl, Morpholinyl, Piperidinyl, Piperazinyl, Tetrahydropyridazinyl, Tetrahydropyrazinyl, 1,3,5-Tetrahydrotriazinyl, 1.2,4-Tetrahydrotriazin-1-yl, 1,2,4-Tetrahydrotriazin-3-yl, 1,3-Dihydrooxazinyl, 3,4,5,6-Tetrahydropyridin-2-yl, 1,2,5,6-Tetrahydropyridin-1-yl, 1,2,3,4-Tetrahydropyridin-1-yl, 1,2-Dihydropyridin-1-yl, 1,4-Dihydropyridin-1-yl, 4H-1,3-Thiazinyl, 4H-3,1-Benzothiazin-2-yl, 2H-1,4-Benzothiazinyl, 1,3-Dihydrooxazin-2-yl. Hexahydroazepin-1-yl, Homopiperazin-1-yl, 1,2,3,4- Tetrahydrochinolin-1-yl, Decahydrochinolin-1-yl, 1,2,3,4-Tetrahydroisochinolin-1-yl, Docahydroisochinulin-1-yl, 1,3,3-Trimethyl-B-azabicyclo[3.2.1]octan-6-yl, 2,5-Diazabicyclo[2.2.1]heptan-2-yl, 2-Aza-5-oxabicyclo[2.2.1]heptan-2-yl, 2-Aza-5-thiabicyclo[2.2.1]heptan-2-yl, 2-Methyl-2,5-diazabicyclo[2.2.1]heptan-5-yl, 2-Benzyl-2,5-Diazabicyclo[2.2.1]heptan-5-yl oder 4-Azatricyclo[4.3.1.1(3,8)]undecan-5-on-4-yl bildet;
oder wenn b2) A eine Gruppe C=O bedeutet,
dann c2) R² und R³ gemeinsam mit dem Stickstoffatom, an das sie gebunden sind, ein gegebenenfalls ein- oder mehrfach durch gleiche oder verschiedene Reste substituiertes Piperidin bildet,
appliziert wird.

2. Verbindungen gemäß Formel (I), 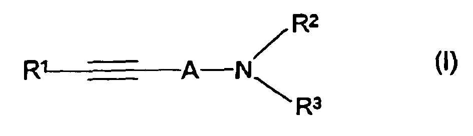 sowie deren N-Oxide und Salze, worin
a) R¹ ein gegebenenfalls ein- oder mehrfach durch gleiche oder verschiedene Reste substituiertes Heteroaryl oder ein- oder mehrfach durch gleiche oder verschiedene Reste substituiertes Phenyl, Biphenyl oder Phenanthryl bedeutet,
b) A eine Gruppe CR⁴R⁵, oder C=O bedeutet, wobei
R⁴ Wasserstoff. Alkyl bedeutet;
R⁵ Wasserstoff oder einen gegebenenfalls substituierten Kohlenwasserstoffrest, welcher geradkettige, verzweigte oder cyclische, gesättigte, teilgesättigte oder ungesättigte organische Reste umfasst, bedeutet;
c) R² und R³ gemeinsam mit dem Stickstoffatom, an das sie gebunden sind, ein gegebenenfalls ein- oder mehrfach durch gleiche oder verschiedene Reste substituiertes Piperidin bildet,
mit der Maßgabe, daß, wenn R² und R³ gemeinsam mit dem Stickstoffatom an das sie gebunden sind einen unsubstituierten oder in der 4-Position durch gegebenenfalls substituiertes Alkyl substituierten Piperidinrest bilden, R¹ nicht 3,5-Bistrifluormethylphenyl ist;
und mit der Maßgabe, daß, die Verbindungen gemäß Formel (I) nicht
1-[3-(3-Chlor-2,4,6-trimethoxy-phenyl)-prop-2-inyl]-piperidin,
3,5-Bis-(piperidin-1-yl-prop-2-inyl)-1-chlor-2,4,6-trimethylbenzol.
2,4-Bis-(piperidin-1-yl-prop-2-inyl)-1,5-dimethoxybenzol,
2,4-Bis-(piperidin-1-yl-prop-2-inyl)-1,5-di(*n*-propoxy)benzol,
1-(3-*m*-Tolyl-prop-2-inyl)-piperidin,
1-(3-*p*-Tolyl-prop-2-inyl)-piperidin.
1-(3-*o*-Tolyl-prop-2-inyl)-piperidin,
1-[3-(4-Hydroxy-phenyl)-prop-2-inyl]-2,5-dimethyl-piperidin-4-ol,
1-Phenyl-3-(1-piperidyl)-prop-1yn,
1-[3-(4-*n*-Propoxyphenyl)-prop-2-inyl]-2,5-dimethyl-piperidin-4-ol,
1-[3-Phenyl-prop-2-inyl]-2,5-dimethyl-piperidin-4-ol
sind.

3. Verwendung einer wirksamen Menge einer Verbindung gemäß Formel (I) in Anspruch 1, gegebenenfalls in Form eines N-Oxids und/oder Salzes, zur Bekämpfung von schädlichen Arthropoden und/oder Helminthen.

4. Verfahren zur Herstellung einer Verbindung der Formel (I) gemäß Anspruch 2, wobei man eine Verbindung der Formel (II)
R¹―X (II)
wobei
X -O-SO₂-CF₃ oder Halogen bedeutet und
R¹ die in Formel (I) gemäß Anspruch 2 angegebenen Bedeutungen hat,
in Gegenwart eines Palladiumkatalysators, einer Base und eines Kupfer(I)salzes mit einer Verbindung der Formel (III), 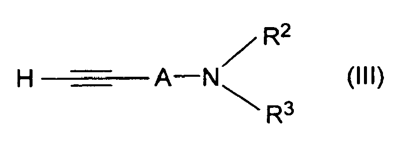 worin A, R² und R³ die in Formel (I) gemäß Anspruch 2 angegebenen
Bedeutungen haben,
umsetzt.

5. Schädlingsbekämpfungsmittel, enthaltend mindestens eine Verbindung gemäß Formel (I) in Anspruch 2 und mindestens ein Formulierungsmittel.

6. Insektizides, akarizides oder nematizides Mittel gemäß Anspruch 5, enthaltend eine wirksame Menge mindestens einer Verbindung gemäß Formel (I) in Anspruch 2 zusammen mit den für diese Anwendung üblichen Zusatz- oder Hilfsstoffen.

7. Schädlingsbekämpfungsmittel, enthaltend eine insektizid, akarizid oder nematizid wirksame Menge mindestens einer Verbindung gemäß Formel (I) in Anspruch 2 und mindestens einem weiteren Wirkstoff zusammen mit den für diese Anwendung üblichen Hilfs- und Zusatzstoffen.

8. Mittel zur Anwendung im Holzschutz oder als Konservierungsmittel in Dichtmassen, in Anstrichfarben, in Kühlschmiermitteln für die Metallbearbeitung oder in Bohr- und Schneidölen, enthaltend eine wirksame Menge mindestens einer Verbindung gemäß Formel (I) in Anspruch 2 zusammen mit den für diese Anwendungen üblichen Hilfs- und Zusatzstoffen.

9. Verbindung gemäß Formel (I) in Anspruch 2 oder Mittel gemäß Anspruch 5, 6 oder 7 zur Herstellung eines Tierarzneimittels.

10. Saatgut, behandelt oder beschichtet mit einer wirksamen Menge einer Verbindung gemäß Formel (I) in Anspruch 2 oder eines Mittels gemäß einem der Ansprüche 5, 6 oder 7.

## Claims

1. Method for controlling harmful arthropods and/or helminths, which comprises applying to these or to the plants or animals, areas or substrates infected by them an effective amount of a compound of the formula (I) 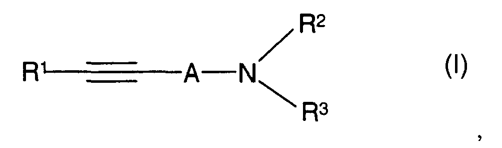 if appropriate also as N-oxide and/or salt, where
a) R¹ is heteroaryl, phenyl, biphenyl or phenanthryl, unsubstituted or mono- or polysubstituted by identical or different radicals,
and if b1) A is a group CR⁴R⁵, where
R⁴ is hydrogen or alkyl;
R⁵ is hydrogen or an unsubstituted or substituted hydrocarbon radical which comprises straight-chain, branched or cyclic saturated, partially saturated or unsaturated organic radicals;
then c1) R² and R³ together with the nitrogen atom to which they are attached form N-heteroaryl, aziridinyl, pyrrolidinyl, isoxazolidinyl, isothioazolidinyl, oxazolidinyl, thiazolidinyl, imidazolidinyl, 1,2,4-oxadiazolidinyl, 1,2,4-thiadiazolidinyl, 1,2,4-triazolidin-3-yl, 1,3,4-thiadiazolidin-2-yl, 1,3,4-triazolidin-1-yl, 1,3,4-triazolidin-2-yl, 2,3-dihydropyrrolyl, 2,5-dihydropyrrolyl, 2,3-dihydroisoxazolyl, 4,5-dihydroisoxazolyl, 2,5-dihydroisothiazolyl, 2,3-dihydropyrazolyl, 4,5-dihydropyrazolyl, 2,5-dihydropyrazolyl, 2,3-dihydrooxazolyl, 4,5-dihydrooxazolyl, 2,5-dihydrooxazolyl, 2,3-dihydrothiazolyl, 4,5-dihydrothiazolyl, 2,5-dihydrothiazolyl, 2,3-dihydroimidazolyl, 4,5-dihydroimidazolyl, 2,5-dihydroimidazolyl, morpholinyl, piperidinyl, piperazinyl, tetrahydropyridazinyl, tetrahydropyrazinyl, 1,3,5-tetrahydrotriazinyl, 1,2,4-tetrahydrotriazin-1-yl, 1,2,4-tetrahydrotriazin-3-yl, 1,3-dihydrooxazinyl, 3,4,5,6-tetrahydropyridin-2-yl, 1,2,5,6-tetrahydropyridin-1-yl, 1,2,3,4-tetrahydropyridin-1-yl, 1,2-dihydropyridin-1-yl, 1,4-dihydropyridin-1-yl, 4h-1,3-thiazinyl, 4h-3,1-benzothiazin-2-yl, 2h-1,4-benzothiazinyl, 1,3-dihydrooxazin-2-yl, hexahydroazepin-1-yl, homopiperazin-1-yl, 1,2,3,4-tetrahydroquinolin-1-yl, decahydroquinolin-1-yl, 1,2,3,4-tetrahydroisoquinolin-1-yl, decahydroisoquinolin-1-yl, 1,3,3-trimethyl-6-azabicyclo[3.2.1]octan-6-yl, 2,5-diazabicyclo[2.2.1]heptan-2-yl, 2-aza-5-oxabicyclo[2.2.1]heptan-2-yl, 2-aza-5-thiabicyclo[2.2.1]heptan-2-yl, 2-methyl-2,5-diazabicyclo[2.2.1]heptan-5-yl, 2-benzyl-2,5-diazabicyclo[2.2.1]heptan-5-yl or 4-azatricyclo[4.3.1.1(3,8)]undecan-5-on-4-yl, unsubstituted or mono- or polysubstituted by identical or different radicals;
or if b2) A is a group C=O,
then c2) R² and R³ together with the nitrogen atom to which they are attached form a piperidine which is unsubstituted or mono- or polysubstituted by identical or different radicals.

2. Compound of the formula (I) 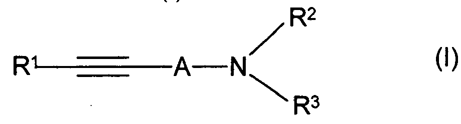 or its N-oxide or salt, where
a) R¹ is heteroaryl which is unsubstituted or mono- or polysubstituted by identical or different radicals or is phenyl, biphenyl or phenanthryl, mono- or polysubstituted by identical or different radicals,
b) A is a group CR⁴R⁵ or C=O, where
R⁴ is hydrogen or alkyl;
R⁵ is hydrogen or an unsubsituted or substituted hydrocarbon radical which comprises straight-chain, branched or cyclic saturated, partially saturated or unsaturated organic radicals;
c) R² and R³ together with the nitrogen atom to which they are attached form a piperidine which is unsubstituted or mono- or polysubstituted by identical or different radicals,
with the proviso that, if R² and R³ together with the nitrogen atom to which they are attached form a piperidine radical which is unsubstituted or substituted in the 4-position by unsubstituted or substituted alkyl, R¹ is not 3,5-bistrifluoromethylphenyl;
and with the proviso that compounds of the formula (I) are not
1-[3-(3-chloro-2,4,6-trimethoxyphenyl)prop-2-ynyl]piperidine,
3,5-bis(piperidin-1-ylprop-2-ynyl)-1-chloro-2,4,6-trimethylbenzene,
2,4-bis(piperidin-1-ylprop-2-ynyl)-1,5-dimethoxybenzene,
2,4-bis(piperidin-1-ylprop-2-ynyl)-1,5-di-(n-propoxy)benzene,
1-(3-m-tolylprop-2-ynyl)piperidine,
1-(3-p-tolylprop-2-ynyl)piperidine,
1-(3-o-tolylprop-2-ynyl)piperidine,
1-[3-(4-hydroxyphenyl)prop-2-ynyl]-2,5-dimethylpiperidin-4-ol,
1-phenyl-3-(1-piperidyl)prop-1-yne,
1-[3-(4-n-propoxyphenyl)prop-2-ynyl]-2,5-dimethylpiperidin-4-ol,
1-[3-phenylprop-2-ynyl]-2,5-dimethylpiperidin-4-ol.

3. Use of an effective amount of a compound of the formula (I) as set forth in Claim 1, if appropriate in the form of an N-oxide and/or salt, for controlling harmful arthropods and/or helminths.

4. Process for preparing a compound of the formula (I) according to Claim 2, which comprises reacting a compound of the formula (II)
R¹―X (II)
where
X is -O-SO₂-CF₃ or halogen and
R¹ is as defined in formula (I) according to Claim 2
in the presence of a palladium catalyst, a base and a copper(I) salt with a compound of the formula (III) 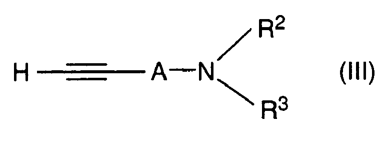 where A, R² and R³ are as defined in formula (I) according to Claim 2.

5. Pesticide, comprising at least one compound of the formula (I) as set forth in Claim 2 and at least one formulation auxiliary.

6. Insecticidal, acaricidal or nematicidal composition according to Claim 5, comprising an effective amount of at least one compound of the formula (I) as set forth in Claim 2 together with additives or auxiliaries customary for this application.

7. Pesticide, comprising an insecticidally, acaricidally or nematicidally effective amount of at least one compound of the formula (I) as set forth in Claim 2 and at least one further active compound together with auxiliaries and additives customary for this application.

8. Composition for use in the protection of wood or as a preservative in sealants, in paints, in cooling lubricants for metal working or in drilling and cutting oils, comprising an effective amount of at least one compound of the formula (I) as set forth in Claim 2 together with auxiliaries and additives customary for these applications.

9. Compound of the formula (I) according to Claim 2 or composition according to Claims 5, 6 and 7 for preparing a veterinary medicament.

10. Seed, treated or coated with an effective amount of a compound of the formula (I) as set forth in Claim 2 or a composition according to any of Claims 5, 6 and 7.

## Revendications

1. Procédé pour lutter contre les arthropodes et/ou les helminthes nuisibles, où l'on applique sur ceux-ci ou sur des plantes ou animaux, des surfaces ou substrats, infestés par ceux-ci, une quantité active d'un composé selon la formule (I) : 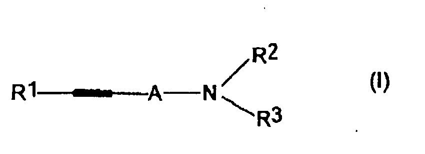 le cas échéant également, comme N-oxyde et/ou sel, où
a) R¹ représente un radical hétéroaryle, phényle, biphényle ou phénanthryle, le cas échéant substitué une ou plusieurs fois par des restes identiques ou différents,
et lorsque
b1) A représente un radical CR¹R⁵, ou
R⁴ représente l'atome d'hydrogène, un radical alcoyle,
R⁵ représente l'atome d'hydrogène ou un reste hydrocarbure le cas échéant substitué, qui comprend un reste organique, linéaire, ramifié ou cyclique, saturé, partiellement saturé ou insaturé,
alors
c1) R² et R³ avec l'atome d'azote sur lequel ils sont liés, forment un N-hétéroaryle le cas échéant une ou plusieurs fois substitué par des restes identiques ou différents, aziridinyle, pyrrolidinyle, isoxazolidinyle, isothiazolidinyle, oxazolidinyle, thiazolidinyle, imidazolidinyle, 1,2,4-oxadiazolidinyle, 1,2,4-thiadiazolidinyle, 1,2,4-triazolidin-3-yle, 1,3,4-thiadiazolidin-2-yle, 1,3,4-triazolidin-1-yle, 1,3,4-triazolidin-2-yle, 2,3-dihydropyrrolyle, 2,5-dihydropyrrolyle, 2,3-dihydroisoxazolyle, 4,5-dihydroisoxazolyle, 2,5-dihydroisothiazolyle, 2,3-dihydropyrazolyle, 4,5-dihydropyrazolyle, 2,5-dihydropyrazolyle, 2,3-dihydrooxazolyle, 4,5-dihydrooxazolyle, 2,5-dihydrooxazolyle, 2,3-dihydrothiazolyle, 4,5-dihydrothiazolyle, 2,5-dihydrothiazolyle, 2,3-dihydroimidazolyle, 4,5-dihydroimidazolyle, 2,5-dihydroimidazolyle, morpholinyle, pipéridinyle, pipérazinyle, tétrahydropyridazinyle, tétrahydropyrazinyel, 1,3,5-tétrahydrotriazinyle, 1,2,4-tétrahydrotriazin-1-yle, 1,2,4-tétrahydrotriazin-3-yle, 1,3-dihydrooxazinyle, 3,4,5,6-tétrahydropyridin-2-yle, 1,2,5,6-tétrahydropyridin-1-yle, 1,2,3,4-tétrahydropyridin-1-yle, 1,2-dihydropyridin-1-yle, 1,4-dihydropyridin-1-yle, 4H-1,3-thiazinyle, 4H-3,1-benzothiazin-2-yle, 2H-1,4-benzothiazinyle, 1,3-dihydrooxazin-2-yle, hexahydroazépin-1-yle, homopipérazin-1-yle, 1,2,3,4-tétrahydroquinoléin-1-yle, décahydroquinoléin-1-yle, 1,2,3,4-tétrahydroisoquinoléin-1-yle, décahydroisoquinoléin-1-yle, 1,3,3-triméthyl-8-azabicyclo[3.2.1]octan-6-yle, 2,5-diazabicyclo[2.2.1]heptan-2-yle, 2-aza-5-oxabicyclo[2.2.1]-heptan-2-yle, 2-aza-5-thiabicyclo[2.2.1]heptan-2-yle, 2-méthyl-2,5-diazabicyclo[2.2.1]heptan-5-yle, 2-benzyl-2,5-diazabicyclo[2.2.1]heptan-5-yle, ou 4-azatricyclo-[4.3.1.1(3.8)]undécan-5-on-4-yle ;
ou lorsque
b2) A représente un radical C=O,
alors
c2) R² et R³ avec l'atome d'azote sur lequel ils sont liés, forment une pipéridine le cas échéant une ou plusieurs fois substituée par des restes identiques ou différents.

2. Composés selon la formule (I) : 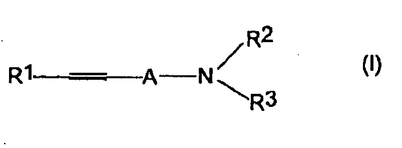 ainsi que leurs N-oxyde et/ou sels, où
a) R¹ représente un radical hétéroaryle, le cas échéant substitué une ou plusieurs fois par des restes identiques ou différents, ou un radical phényle, biphényle ou phénanthryle, le cas échéant substitué une ou plusieurs fois par des restes identiques ou différents,
b) A représente un radical CR⁴R⁵ ou C=O, où
R⁴ représente l'atome d'hydrogène, un radical alcoyle,
R⁵ représente l'atome d'hydrogène ou un reste hydrocarbure le cas échéant substitué, qui comprend un reste organique, linéaire, ramifié ou cyclique, saturé, partiellement saturé ou insaturé,
c) R² et R³ avec l'atome d'azote sur lequel ils sont liés, forment une pipéridine le cas échéant une ou plusieurs fois substituée par des restes identiques ou différents,
avec la condition que lorsque R² et R³ avec l'atome d'azote sur lequel ils sont liés, sont un reste pipéridine non substitué ou substitué en position 4 par un radical alcoyle le cas échéant substitué,
R¹ n'est pas 3,5-bistrifluorométhylphényle ;
et avec la condition que les composés selon la formule (I) ne sont pas
la 1-[3-(3-chloro-2,4,6-triméthoxyphényl)prop-2-inyl]pipéridine,
le 3,5-bis(pipéridin-1-ylprop-2-inyl)-1-chloro-2,4,6-triméthylbenzène,
le 2,4-bis(pipéridin-1-ylprop-2-inyl)-1,5-diméthoxybenzène,
le 2,4-bis(pipéridin-1-ylprop-2-inyl)-1,5-di(n-propoxy)benzène,
la 1-(3-m-tolylprop-2-inyl)pipéridine,
la 1-(3-p-tolylprop-2-inyl)pipéridine,
la 1-(3-o-tolylprop-2-inyl)pipéridine,
le 1-[3-(4-hydroxyphényl)prop-2-inyl]-2,5-diméthylpipéridin-4-ol,
le 1-phényl-3-(1-pipéridyl)prop-1-yne,
le 1-[3-(4-n-propoxyphényl)prop-2-inyl]-2,5-diméthylpipéridin-4-ol,
le 1-[3-phénylprop-2-inyl]-2,5-diméthylpipéridin-4-ol.

3. Utilisation d'une quantité active d'un composé selon la formule (I) de la revendication 1, le cas échéant sous la forme d'un N-oxyde et/ou d'un sel, pour lutter contre les arthropodes et/ou helminthes nuisibles.

4. Procédé de préparation d'un composé de la formule (I) selon la revendication 2, où l'on fait réagir un composé de la formule (II) :
R¹ - X (II)
où X représente le radical -O-SO₂-CF₃ ou un atome d'halogène, et
R¹ a la signification donnée à la formule (I) selon la revendication 2,
en présence d'un catalyseur au palladium, d'une base et d'un sel de cuivre (I) avec un composé de la formule (III) : 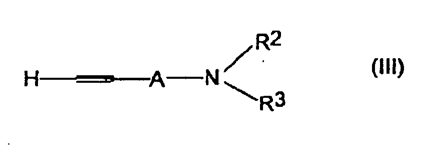
où A, R² et R³ ont les significations données à la formule (I) selon la revendication 2.

5. Agent de lutte contre les parasites, contenant au moins un composé selon la formule (I) à la revendication 2 et du moins un agent de formulation.

6. Agent insecticide, acaricide ou nématicide selon la revendication 5, contenant une quantité active d'au moins un composé selon la formule (I) de la revendication 2, avec les additifs ou auxiliaires usuels pour cette utilisation.

7. Agent de lutte contre les parasites, contenant une quantité active de manière insecticide, acaricide ou nématicide d'au moins un composé selon la formule (I) de la revendication 2, et au moins un autre agent actif avec les additifs ou auxiliaires usuels pour cette utilisation.

8. Agent à utiliser dans la protection du bois ou comme agent de conservation dans les masses d'étanchéité, dans les peintures, dans les réfrigérants lubrifiants du traitement des métaux ou dans les huiles de forage et de coupe, contenant une quantité active d'au moins un composé selon la formule (I) de la revendication 2, avec les additifs ou auxiliaires usuels pour cette utilisation.

9. Composé selon la formule (I) de la revendication 2 ou agent selon la revendication 5, 6 ou 7, pour la préparation d'agents pharmaceutiques vétérinaires.

10. Semence, traitée ou revêtue d'une quantité active d'un composé selon la formule (I) de la revendication 2 ou d'un agent selon l'une des revendications 5, 6 ou 7.
